# EUROPEAN PATENT SPECIFICATION

(11) **EP 1 807 520 B1**
(45) Date of publication and mention of the grant of the patent: **25.07.2012**
(21) Application number: 05802972.9
(22) Date of filing: 25.10.2005
(51) Int. Cl.: C12N 15/82

(54) **RNA CONSTRUCTS**
RNA-KONSTRUKTE
CONSTRUCTIONS D'ARN

(30) Priority: 25.10.2004 GB 0423659; 25.10.2004 US 621800 P; 20.05.2005 US 683551 P
(43) Date of publication of application: 18.07.2007
(60) Divisional application: 10183880.3
(73) Proprietor: Devgen NV, 9052 Zwijnaarde (BE)
(72) Inventor: VAN DE CRAEN, Marc, B-9880 Aalter (BE); PLAETINCK, Geert, B-9820 Merelbeke-Bottelare (BE); VERCAUTEREN, Isabelle, B-9550 Woubechtegem (BE); LOGGHE, Marc, Georges, B-9051 St Denijs Westrem (BE); BOGAERT, Thierry, Andre, Olivier, Eddy, B-8500 Kortrijk (BE); ZWAAL, Richard, B-3001 Leuven (BE)
(74) Representative: White, Nina Louise
(86) International application number: PCT/IB2005/003557
(87) International publication number: WO 2006/046148

(56) References cited:
- WO-A-99/49029
- US-A1- 2003 154 034
- LLAVE CESAR ET AL: "Endogenous and silencing-associated small RNAs in plants" PLANT CELL, vol. 14, no. 7, July 2002 (2002-07), pages 1605-1619, XP002390784 ISSN: 1040-4651
- MYERS J W ET AL: "Recombinant Dicer efficiently converts large dsRNAs into siRNAs suitable for gene silencing" NATURE BIOTECHNOLOGY, NATURE PUBLISHING GROUP, NEW YORK, NY, US, vol. 21, no. 3, March 2003 (2003-03), pages 324-328, XP002302300 ISSN: 1087-0156
- ELBASHIR SAYDA M ET AL: "Duplexes of 21-nucleotide RNAs mediate RNA interference in cultured mammalian cells" NATURE, NATURE PUBLISHING GROUP, LONDON, GB, vol. 411, no. 6836, 24 May 2001 (2001-05-24), pages 494-498, XP002206451 ISSN: 0028-0836
- SUSI P ET AL: "Characteristics of RNA silencing in plants: similarities and differences across kingdoms" PLANT MOLECULAR BIOLOGY, vol. 54, no. 2, January 2004 (2004-01), pages 157-174, XP002390785 ISSN: 0167-4412 cited in the application
- BAULCOMBE D: "RNA SILENCING IN PLANTS" NATURE, NATURE PUBLISHING GROUP, LONDON, GB, vol. 431, 16 September 2004 (2004-09-16), pages 356-363, XP000962079 ISSN: 0028-0836 cited in the application
- MCMANUS M T ET AL: "GENE SILENCING USING MICRO-RNA DESIGNED HAIRPINS" RNA, COLD SPRING HARBOR LABORATORY PRESS, WOODBURY, NY, US, vol. 8, no. 6, June 2002 (2002-06), pages 842-850, XP008021481 ISSN: 1355-8382
- LIANG L ET AL: "Optimizing the delivery systems of chimeric RNA.DNA oligonucleotides. Beyong general oligonucleotide transfer" EUROPEAN JOURNAL OF BIOCHEMISTRY, BERLIN, DE, vol. 269, 2002, pages 5753-5758, XP002399593 ISSN: 0014-2956

## Description

### FIELD OF THE INVENTION

The present invention relates to the field of double-stranded RNA (dsRNA) mediated gene silencing. More particularly, the present Invention relates to genetic constructs designed to be more effective in dsRNA silencing by targeting multiple target sequences .

These constructs are particularly useful in dsRNA mediated plant pest control.

### BACKGROUND TO THE INVENTION

Many dsRNA constructs have been described In the art. A classic dsRNA is produced from a DNA construct comprising two convergent promoters flanking the sequence complementary to the target sequence which needs to be downregulated (see for example WO00/01846). As the technology of dsRNA mediated gene silencing advanced, new constructs were designed to improve the dsRNA for various purposes.

In order to produce the dsRNA more efficiently, a stem-loop-stem structure or 'hairpin' was developed. As described in, for example, document WO99/53050, this hairpin allows the formation of dsRNA from one single RNA transcript. The RNA transcript comprises the sense and anti-sense version of the complementary sequence, separated by a non-complementary loop structure allowing the-RNA transcript to fold back and the base pair into a dsRNA stem portion.

In order to produce dsRNA that is more effective in gene silencing, multiple copies of the sequence complementary to the target sequence were incorporated in one construct and converted into one dsRNA. Document WO99/49029 describes in more detail a synthetic gene comprising multiple structural gene sequences, wherein each structural gene sequence is substantially identical to the target gene.

Document WO2004/001013 describes constructs especially designed to be used in clinical applications for the prevention or treatment of diseases or infection without the generation of adverse side-effects due to dsRNA-induced toxicity. It has been described that some dsRNA may induce an interferon response that can lead to cell death (Jaramillo et al., Cancer Invest. 13: 327-338, 1995). These constructs are characterized by moieties that are sensitive to RNA processing in order to improve the formation of Short interfering RNAs (siRNAs) that mediate gene silencing whilst avoiding dsRNA toxicity caused by long (more than 30 base pairs) dsRNA. Short interfering RNAs (siRNAs) mediate cleavage of specific single-stranded target RNAs. These siRNAs are commonly around 21 nt in length, suggesting that siRNA expression in the host causes efficient and specific down-regulation of gene expression, resulting in functional inactivation of the targeted genes.

DsRNA gene silencing finds application In many different areas, such as for example dsRNA mediated gene silencing in plants. DsRNA gene silencing also finds application in the.field of plant pest control (WO 00/01864). Generally, the pest organism is eradicated via the uptake of dsRNA, capable of silencing the expression of a target gene, which expression is necessary for the viability, growth and/or development of the pest species. Contacting the pest organisms with the dsRNA may occur in various ways, one example of which is the production of the dsRNA within the plant cell affected by the pest organism.

One problem when expressing dsRNA in plants is that it may be processed by the RNA processing machinery of the plant cell(Susi et al, 2004. PMB 54: 157-174, Baulcombe, 2004. Nature 431: 356-363).

Llave et al., 2002 (The Plant Cell 14: 1605-1619), describes the identification of endogenous small RNAs of predominantly 21 to 24 nucleotides in plants, particularly *Arabidopsis*.

Myers et al., 2003 (Nature Biotechnology 21: 324-328), describes the production of 20- to 21-base pair siRNAs *in vitro* using purified recombinant forms of the enzyme Dicer. The Dicer-generated siRNAs were shown to be effective in silencing transiently transfected reporter genes and endogenous genes.

### SUMMARY OF THE INVENTION

While the formation of short interfering RNAs (siRNAs) of about 21 nt is desired for gene Silencing, it is now been found by the present inventors that the minimum length of dsRNA needs to be at least 80-100 nt in order to be efficiently taken up by the pest organism. There are indications that in invertebrates such as the free living nematode *C. elegans* or the plant parasitic nematode *Meloldogyne incognita* these longer fragments are more effective in gene silencing, possibly due to a more efficient uptake of these long dsRNA by the invertebrate.

The present invention addresses this problem by providing dsRNA constructs that are efficient in dsRNA mediated gene silencing, whilst retaining sufficient length.

In addition the present Invention provides concatemer dsRNA design, allowing to combine several short fragments In one longer dsRNA construct and allowing to increase the efficacy of the control of the pests' viability, growth and/or development.

The present invention provides an isolated double-stranded ribonucleic acid (dsRNA) with a length of at least 80 base pairs effective in RNAi gene silencing, wherein the dsRNA comprises at least two dsRNA fragments of at least 17 base pairs, each fragment comprising annealed complementary strands, wherein each dsRNA fragment comprises a strand that is complementary to at least part of the nucleotide sequence of a target sequence wherein:
(i) each dsRNA fragment comprises a strand that is complementary to at least part of the nucleotide sequence of a different target sequence, wherein said different target sequences originate:
   (a) from a single target gene, wherein the dsRNA fragments are scrambled and combined deliberately in any rank order in the dsRNA and wherein the dsRNA fragments are not combined in the original order in which the fragments appear in the target gene, or
   (b) from different target genes in a single target species or in different target species, or
   (c) from different target species, or
(ii) said dsRNA comprises (a) at least two copies of one dsRNA fragment or (b) at least two copies of a series of dsRNA fragments.

The constructs herein described and suitable for efficient dsRNA mediated pest control, are designed to meet at least some of the following requirements (1) the dsRNA construct has good stability in the host cell producing the dsRNA (2) the dsRNA is taken up by the pest organisms (3) the dsRNA has good stability in the pest organisms and/or (4) the dsRNA is effective in the pest organism to control its viability, growth and/or development.

These dsRNA construct designs have one or more of the following advantages:
(1) The concatemer constructs of the present invention allow the incorporation of multiple dsRNA fragments to target multiple target sequences or target genes simultaneously. These multiple target sequences or target genes may originate from the same or from different pest species. These multiple target sequences or target genes may be orthologs or homologs or may be unrelated. Alternatively, the concatemer constructs allow the incorporation of multiple dsRNA fragments directed against one or more parts of one target gene;
(2) the constructs of the present invention allow development of dsRNA of which the length and/or size and/or shape is compatible with sufficient uptake by a pest organism;
(3) contrarily to prior art dsRNA constructs that have been designed to be processed quickly into smaller fragments, it is now one of the purposes of the present invention to design dsRNA that is more stable in the host cell or organism (for example in the plant and/or in the plant pest).
(4) the constructs of the present invention have the advantage of being stable in the host organism in which the dsRNA construct is produced. For example, when expressed in a plant cell, the dsRNA construct as provided by the present invention is protected against RNA processing in the plant. In this way, the dsRNA is less diced by the host machinery and can be taken up in a more intact (e.g. larger) form by the plant pest organism when it feeds on or from the plant.

The present invention further relates to DNA constructs encoding the dsRNA constructs according to the present invention, to expression vectors comprising such DNA constructs and to host cells comprising such dsRNA. DNA or expression vectors.

The present invention also encompasses methods for producing such dsRNA constructs, methods for producing DNA expression constructs, methods for producing host cells, methods for using these constructs in gene silencing, methods for producing transgenic organisms and methods for controlling pests.

### DETAILED DESCRIPTION OF THE INVENTION

### Concatemer constructs

According to a first embodiment, the present invention relates to an isolated (e.g. substantially pure) double-stranded ribonucleic acid (dsRNA) effective in RNAi gene silencing, wherein the dsRNA (portion or fragment) comprises multiple dsRNA fragments, each fragment comprising annealed complementary strands, one of which is complementary to at least part of the nucleotide sequence of a target sequence to be silenced or a target gene of interest, said dsRNA being capable of forming a double-stranded RNA portion or fragment.

A concatemer construct according to the present invention comprises multiple dsRNA fragments within one dsRNA stem. Such a concatemer construct can be used "per se", hereinafter named "a concatemer construct per se" or can be used as a dsRNA stem in the stabilized RNA constructs described herein . Accordingly, the RNA constructs of the present invention comprising multiple dsRNA fragments in one dsRNA stem are also generally referred to as "concatemers". As a non-limiting list of examples of "concatemers", the present invention provides a concatemer cloverleaf, a concatemer dumbbell, a concatemer hairpin, a concatemer stem dsRNA. All these concatemers may optionally be stabilized with a lock as described herein and may optionally be provided with a linker as described herein.

The present invention thus relates to concatemer constructs comprising double-stranded RNA (also named a dsRNA molecule) comprising annealed complementary strands, one of which has a nucleotide sequence which is complementary to at least part of a target nucleotide sequence of a target gene of a pest species. In one embodiment, the multiple RNA fragments are present that are complementary to different (e.g. distinct) sequences in one target gene. In another embodiment, the present invention also relates to concatemer constructs as described above, comprising multiple RNA fragments that are complementary to sequences of different (e.g. distinct) target genes. In one embodiment, the dsRNA fragments are separated by a linker sequence or by a lock. Preferably the linker sequence is double stranded and the strands are complementary, thus also forming a double stranded region. The linker sequence may comprise a short random nucleotide sequence that is not complementary to target sequences.

The term "multiple" In the context of the present invention means at least two, at least three, at least four, at least five, at least six, etc... and up to at least 10, 15, 20 or at least 30.

The present invention thus relates to an isolated dsRNA or ds RNA construct as described herein, wherein said dsRNA comprises at least one repeat of one dsRNA fragment. As used herein, one repeat means two copies of the same dsRNA fragment

In another embodiment, the present invention relates to an isolated dsRNA or ds RNA construct as described herein, wherein said dsRNA comprises at least one repeat of a series of dsRNA fragments. Thus as described herein, one repeat means two copies of a series of dsRNA fragments.

The present invention also relates to an isolated dsRNA as described above wherein said dsRNA comprises at least two or three copies, preferably at least four, five or six copies, more preferably at least seven, eight, nine, ten or more copies of one dsRNA fragment or of a series of dsRNA fragments. In other words, said multiple dsRNA fragments are repeats of a single dsRNA fragment or of a series of dsRNA fragments.

It should be clear that the expression "multiple dsRNA" also encompasses dsRNAs comprising copies of one or more dsRNA fragments and further comprising other dsRNA fragments, that are different from the repeated or copied or multimerized dsRNA fragments. Therefore the invention also relates to an isolated dsRNA comprising one or more repeats of dsRNA fragments and further comprising at least one dsRNA fragment which is distinct from the repeated fragment(s).

The term "complementary" as used herein relates to DNA-DNA and RNA-RNA - complementarity as well as to DNA-RNA complementarity. In analogy herewith, the term "RNA equivalent" means that in the DNA sequence(s), the base "T" may be replaced by the corresponding base "U" normally present in ribonucleic acids.

A "complementary region" as used herein means a region that is complementary to at least part of a nucleotide sequence of a target gene. "Complementary" when used in the context of the present invention for a dsRNA, means having substantial sequence identity to one of the strands of the target sequence. In performance of the present invention, the complementary region will generally comprise a nucleotide sequence having more than about 75% sequence identity to the corresponding sequence of the target gene; however, a higher homology might produce a more efficient modulation of expression of the target gene. Preferably the sequence identity is about 80%, 85%, 90%, 95%, and even more preferably more than about 99%. In the context of the present invention, the expression "more than about" has the same meaning as "at least".

Preferably, the complementary region is a fragment that is not harmful for organisms other than the target organism(s). Preferably, the fragment does not have more than 20 contiguous nucleotides in common with a sequence of an organism other than the target organism. For example, when the target organism is a plant pathogen, such as a plant parasitic nematode or an insect, the fragment does not have 20 contiguous nucleotides in common with a nucleotide sequence form a plant or a mammal (a human in particular).

The terms "double-stranded RNA (dsRNA)" and "RNA capable of forming a dsRNA" are used herein interchangeably. The term "dsRNA construct" as used herein encompasses all constructs capable of forming double stranded RNA, such as any of the concatemer or stabilized constructs described herein. As described further, the dsRNA or dsRNA construct may comprise other sequences that are not complementary to a target gene or sequence but that have other functions.

The terms "double stranded RNA, fragment" or "double-stranded RNA region" refer to a small entity of the double-stranded RNA corresponding with (part of) the target gene. As used herein, the expression "corresponding to" means "complementary to".

In one embodiment, in the dsRNA of the invention, said multiple dsRNA fragments are not separated by a non-complementary region. This means that no non-hybridizing RNA regions are present between the separate dsRNA fragments.

According to other embodiments in the dsRNA of the invention, the dsRNA fragments are not separated by a spacer or a lock sequence as described further.

In the concatemer constructs, the length of each of the dsRNA fragments may be at least 17 bp, 18 bp, 19 bp, 20 bp, 21 bp, 22 bp, 23 bp, 24 bp, 25 bp or more, for example about 30 bp, about 40bp, about 50 bp, about 60 bp, about 70 bp, about 80 bp, about 90 bp, about 100 bp, about 110 bp or about 120 bp. Preferred dsRNA fragments in a concatemer construct have a length between 17 and 2000 bp, preferably between 21 and 1000 or 500 or 250 bp, preferably between 40 and 150 bp, more preferably between 50 and 120 bp or any number in between.

A "target gene" as used herein means a gene that needs to be silenced in the target species. A target gene encompasses a promoter region, a 5' untranslated region, a coding sequence wherein introns may be present, and a 3' untranslated region. The target gene may be selected from the genome of any target species as described herein. According to one embodiment, the target sequence is chosen from the genome of an organism, which organism is different from the organism in which the dsRNA is expressed. This means that the dsRNA is expressed in one cell or organism and is subsequently transferred or taken up by another cell or organism comprising the target gene. According to one specific embodiment of the present invention, the dsRNA is expressed in a plant or a plant cell and the target gene is chosen from the genome of a bacterium, a virus, a virion, an invertebrate, more particularly from a plant pest species, such as a virion, a virus, a nematode, a fungus or an insect.

"Transfer" of the dsRNA from the plant to the pest species means that the dsRNA is produced in the plant cell and is being taken up, relocated or brought into contact with the pest organism. A plant parasitic nematode or an insect for example, may take up the dsRNA produced in the plant by feeding from the plant cell cytoplasm. A fungal cell which is contacted with the dsRNA may be a plant pathogenic fungal cell in a life stage outside a plant cell, for example in the form of a hypha, germ tube, appressorium, conidium (asexual spore), ascocarp, cleistothecium, or ascospore (sexual spore outside the plant). Alternatively, the fungal cell which is contacted with the dsRNA is a plant pathogenic fungal cell in a life stage inside a plant cell, for example a pathogenic form such as a penetration peg, a hypha, a spore or a haustorium.

According to other embodiments of the invention, it may suffice to contact the pest cell or pest species with the dsRNA, in which case transfer of dsRNA means contacting with a composition comprising the dsRNA or dsRNA construct.

According to another embodiment, the dsRNA is expressed in a bacterial or fungal cell and the bacterial or fungal cell is taken up or eaten by the pest species. According to still another embodiment, the dsRNA is isolated from, or purified from, the bacterial or fungal cell expressing the dsRNA, and the dsRNA is provided as a pesticide or in a pesticidal formulation to the pest species.

Particular suitable target genes are genes that are involved in an essential biological pathway of the target species, meaning that the target gene is an essential gene to the target species and that gene silencing of the target gene has an adverse effect on the viability the growth and/or development of the target species. Suitable target genes include genes associated with infection, propagation or pathogenesis of the pest species in the host

### Choice of target gene(s) to be targeted by a concatemer construct

The choice of target gene(s) to be targeted by one single concatemer construct, depends on the choice of target gene which is to be silenced in the target organism or organisms in order to achieve the desired effect of pest control. For the concatemers designed herein below the target gene(s) was (were) chosen from one or more of the following categories of genes:
1. "essential" genes encompass genes that are vital for one or more target organisms and result in a lethal or severe (e.g. movement feeding, paralysis, drinking, fertility) phenotype when silenced . The choice of a strong lethal target gene results in a potent RNAi effect. In the concatemer constructs of the invention, multiple dsRNA fragments targeting the same or different very effective lethal genes were combined to further increase the potency, efficacy or speed of the dsRNA in pest control.
2. "pathogenicity genes" are genes that are involved in the pathogenicity or infectivity of the pest. Targeting said genes may reduce pathogenicity or infectivity of the pest thereby protecting the infested organism against pest infestation.
3. "weak" genes encompass target genes with a particularly interesting function, but which result in a weak phenotypic effect when silenced independently. Targeting a particular but weak target gene results in a specific RNAi effect, meaning that the mode of action is very focussed and controlled. For example, interesting but weak genes could be genes that are very species specific, or even species restricted but that do not result in an effective RNAi effect when targeted separately. In the concatemer constructs of the invention, multiple dsRNA fragments targeting a single or different weak gene(s) were combined to obtain a stronger RNA effect.
4. "pest specific" genes encompass genes that have no substantial homologous counterpart in non-pest organisms as can be determined by bioinformatics homology searches, for example by BLAST searches. The choice of a pest specific target gene results in a species specific RNAi effect, with no effect or no substantial (adverse) effect in non-target organisms.
5. "conserved genes" encompass genes that are conserved (at the amino acid level) between the target organism and non-target organism(s). Some target genes may be very RNAi effective, but may be very conserved between organisms. To reduce possible effects on non-target species, such effective but conserved genes were analysed and target sequences from the variable regions of these conserved genes were chosen to be targeted by the dsRNA fragments in the concatemer constructs of the invention herein exemplified. Here, conservation is assessed at the level of the nucleic acid sequence. Such variable regions thus encompass the least conserved sections of the conserved target gene(s).
6. "conserved pathway" genes encompass genes that are involved in the same biological pathway or cellular process, or encompass genes that have the same functionality in different species.
   a. Preferred examples of such "conserved pathway" target genes are genes involved in vital cellular pathways or functions, which pathways or functions are RNAi sensitive, such as, but not limited to: endocytosis, the cytoskeleton, intracellular and intercellular transport, calcium binding, nucleus import and export, nucleic acid binding, signal peptidase-protein binding, the proteasome, protein translation, vesicle transport, neuro-transmission, waterbalance, ionbalance, gene transcription, splicing, mitosis, meiosis, chromosome organisation, stability or integrity, micro RNAs, siRNAs, posttranslational protein modifications, electron transport, metabolism (anabolism or catabolism), apoptosis, membrane integrity, and cell adhesion.
   b. In one embodiment, the concatemer constructs according to the present invention target multiple genes from the same biological pathway, resulting in a specific and potent RNAi effect and more efficient pest control.
   c. Alternatively, the concatemer constructs according to the present invention target multiple genes from different biological pathways, resulting in a broad cellular RNAi effect and more efficient pest control.
   d. Alternatively, a combination of b) and c).

### Choice of target sequence(s) targeted by the dsRNA fragments in the concatemer construct

Once a target gene is selected (or multiple target genes are selected), one or more particular target sequences to be targeted by the dsRNA fragment of the concatemer construct is selected from that (those) target gene(s). In the concatemer constructs of the , invention, the selection of such target sequences was made based on one or more of the following selection criteria:
1. The target sequence targeted by the dsRNA fragment in the concatemer construct does not have substantial nucleotide sequence homology with non-target organisms. A preferred criterion is that the target sequence does not have substantial homology to human sequences and/or does not have substantial homology with host plant sequences and organisms living in symbiosis with the plant (e.g. plant symbiotic bacteria). A non-limiting list of host plants according to the invention comprises for example corn, cotton, tomato, potato, banana, canola, sunflower, alfalfa, wheat, rice, sorghum, millet and soybean.
2. The target sequence targeted by the dsRNA fragment in the concatemer construct is selected from a region of the target gene containing the best predicted siRNA, which prediction can for instance be made according to "Tuschl rules" (Yuan et al. "siRNA Selection Server: an automated siRNA oligonucleotide prediction server", W130-W134, Nucleic acid research, 2004, vol. 32, Web Server issue). Basically this criterium involves the determination of the %GC content versus % AT content of the DNA. Preferably, the target sequences targeted by the dsRNA fragments of the concatemer constructs of the present invention have a GC content ranging from about 40% to about 60%, more preferably they have a GC content of about 50%. Alternative predictions for choosing siRNA sequences can be found in: Saetrom and Snove 2004 ("A comparison of siRNA efficacy predictors", Biochem. Biophys. Res. Commun. Vol 321(1): 247-253);.Chalk et al. 2004 ("Improved and automated prediction of effective siRNA.", Biochem. Biophys. Res. Commun. 319(1):264-74); Levenkova et al. 2004 ("Gene specific siRNA selector.", Bioinformatics. 20(3):430-2); Reynolds et al. 2004 ("Rational siRNA design for RNA interference.", Nat Biotechnol. 22(3):326-30); Henschel et al. 2004 ("DEQOR: a web-based tool for the design and quality control of siRNAs.", Nucleic Acids Res. (Web Server issue):W113-20).
3. The target sequence targeted by the dsRNA fragment in the concatemer construct is in a conserved region (at the nucleotide acid level) of the target gene. Such conserved regions are determined by comparing the sequences of homologous genes from the same and/or different species. As such, multiple gene family members may be down regulated in one or in multiple species.
4. Alternatively, the target sequence targeted by the dsRNA fragment in the concatemer construct is in a non-conserved region of the target gene (for the reasons explained earlier therein).

### Ways of combining multiple dsRNA fragments into one concatemer construct:

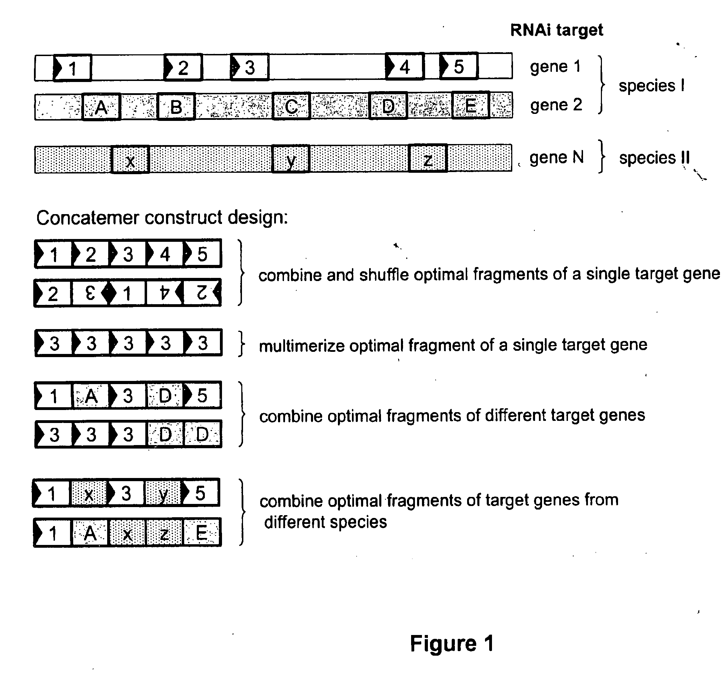

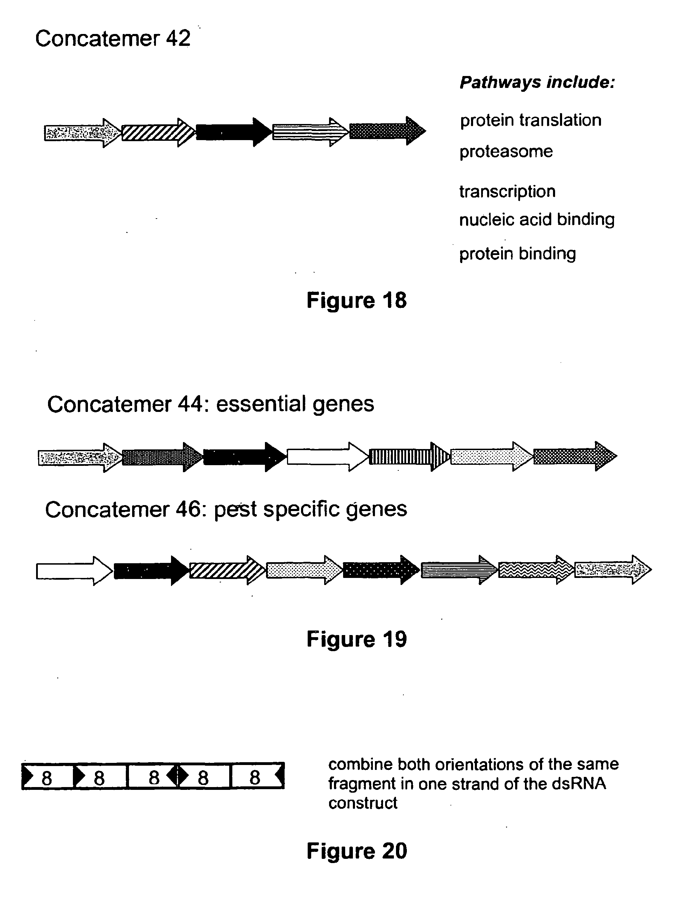

All the above given alternatives for target gene selection and target sequence selection may be easily combined with each other. The corresponding dsRNA fragments (or regions) targeting such target genes and target sequences may be combined in a variety of ways into the concatemer construct. In the concatemer constructs of the invention, one or more of the following ways of combining dsRNA fragments were used (see also Figure 1 and 20):
1. when multiple dsRNA fragments targeting a single target gene are combined, they may be combined in the original order (i.e., the order in which the fragments appear in the target gene) in the concatemer construct,
2. alternatively, the original order of the fragments may be ignored so that they are scrambled and combined randomly or deliberately in any rank order into the concatemer construct,
3. alternatively, one single fragment may be repeated several times, for example from 1 to 10 times, e.g. 1, 2, 3, 4, 5, 6, 7, 8, 9 or 10 times in the concatemer construct, or 4. the dsRNA fragments (targeting a single or different target genes) may be combined in the sense or antisense orientation.

The possibility to combine dsRNA fragments in the concatemer construct is especially advantageous to avoid coincidental overlap with non-target sequence at the conjunction of the multiple dsRNA fragments in the concatemer construct. For example, when two dsRNA fragments with no homology to non-target organism over 20 consecutive nucleotides are combined, there might arise at the conjunction a new sequence which might have homology to non-target organism over a range of 20 consecutive nucleotides. In such case, the concatemer design as described herein allows to convert one of the dsRNA fragments into another orientation (e.g. convert from sense to antisense) and/or allows to change the order of the fragments (e.g. convert from A-B to B-A in the concatemer construct) to overcome this problem.

In addition, it is advantageous that in the nucleotide sequence of the final concatemer construct, no plant splice acceptor and splice donor sites are created. It is also recommended that the nucleotide sequence of the final concatemer construct does not contain a large ORF.

This possibility of combining dsRNA fragments in the concatemer construct is also advantageous for cloning purposes, because the separate fragments may be randomly ligated to each other.

The dsRNA constructs of the invention may be formed from a single RNA polynucleotide molecule which includes regions of self-complementarity, such that when folded it is capable of forming a structure including one or more double-stranded portions effective in gene silencing by RNAi. The constructs may also be formed from two or more separate polynucleotide strands which together form a double stranded, folded or assembled structure which includes at least one double-stranded portion effective In gene silencing by RNAi. The RNA constructs may, when folded or assembled, include both double-stranded and single-stranded regions, as illustrated in the accompanying Figures. The RNA constructs may include non-natural bases and/or non-natural backbones linkages.

The dsRNA or dsRNA constructs comprising multiple dsRNA fragments may herein be generally referred to as concatemers. The actual fragment that is double stranded is also referred to as "portion". Said portion contains one or multiple dsRNA fragments.

The concatemer constructs and methods of the present invention are particularly useful to combine multiple target sequences simultaneously. These multiple sequences may originate from one target gene. Alternatively, the multiple target sequences may originate from multiple target genes. These multiple target genes may originate from one and the same pest species. Alternatively, these multiple target genes may originate from different pest species from the same or different order. These multiple target genes may be related, for example may be homologs or orthologs, or may be unrelated. Therefore, one concatemer dsRNA construct of the present invention, for example in the form of a concatemer stem, a concatemer hairpin or a concatemer cloverleaf, may simultaneously target multiple sequences originating from the same pest species, or may simultaneously target multiple target genes from the same pest species, or may simultaneously target multiple target genes of multiple pest species of the same or different order.

The present invention thus encompasses an isolated dsRNA or dsRNA construct comprising at least two dsRNA fragments, wherein each dsRNA fragment comprises a strand that is complementary to at least part of the nucleotide sequence of a different (e.g. distinct) target sequence. In one embodiment, said different target sequences originate from a single (or the same) target gene. In another embodiment, said different target sequences originate from different (e.g. distinct) target genes.

According to one particular embodiment of the present invention, the concatemer targets multiple target genes originating from multiple species. For example, one concatemer may target multiple genes from multiple plant pest organisms, and by expressing the concatemer in the plant, the plant acquires resistance against multiple plant pests simultaneously. Similarly, a plant or a surface or substance susceptible to pest infestation may be sprayed with a composition (or the like) comprising the dsRNA concatemers, thereby protecting the plant or the surface or substance against infestation from multiple pests. For example, the plant acquires resistance against nematodes and insects, or against nematodes, insects and/or fungi. Also the concatemers construct allows the plant to acquire resistance against multiple nematodes of a different genus, family, order or class, and/or against insects of a different genus, family or order, and/or against fungi of a different genus, family or order.

In another particular embodiment of the present invention, the concatemer targets multiple target genes originating from different species from the same order. For example, one concatemer which targets genes of different bacterial, viral, fungal, insect or nematode species, may be used as an effective and broad spectrum bacteria, virus, fungus, insect killer or broad spectrum nematode killer. Combination of dsRNA fragments targeting multiple target sequences from different pest species into one concatemer construct according to the present invention is favorable to enlarge the pest species spectrum of the RNAi effect of the dsRNA molecules.

In another particular embodiment of the present invention, the concatemer targets multiple target genes originating from the same organism, for example from the same pest species. Such a construct offers the advantage that several weak target genes from the same organism can be silenced together to efficiently control the pest organism, while silencing one or more of the weak target genes separately is not effective to control the pest. Also, several strong target genes from the same organism can be silenced simultaneously, in order to further improve the efficacy of the pest control, or to avoid the occurrence of resistance of the pest organisms by mutation.

The present invention thus encompasses an isolated dsRNA or dsRNA construct as described above, wherein said different target genes originate from a single target (or pest) species, or wherein said different target genes originate from distinct target (or pest) species; for instance pest species belonging to the same (in one embodiment) or to different (in other embodiments) genera, families, orders or even phyla.

The dsRNA constructs described herein and targeting multiple target genes, are characterized by accumulating multiple RNAi capacity, resulting in synergistic effects, and capable of triggering multiple RNAi effects in the target cell or target organism.

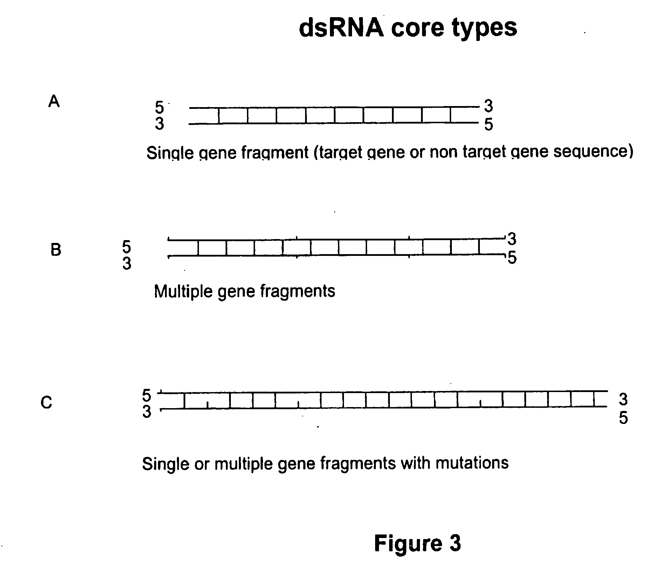

Figure 3 shows the different dsRNA core types of the present invention, which form part of the concatemer and/or stabilized dsRNA constructs as described herein. In dsRNA core type A, the repeated single gene fragment may be complementary to a target gene sequence or to a non-target gene sequence. In dsRNA core type B, the multiple gene fragments may be present in sense or anti-sense orientation and may originate from a single target gene or from different target genes, for example from the same species or from different species. dsRNA core type B thus represents a basic concatemer in stem format.

In dsRNA core type C, the sense or antisense strand comprises for example 5 to 7 mutations in each -21 bp fragment. These mutations may be for example C to T mutations. The anti-sense or sense strand comprises no mutations and is 100 % complementary to the target gene mRNA. This type of construct will provide protection against transcriptional gene silencing of the transgene. In this type of construct single or multiple gene fragments can be included.

### Stabilized constructs

Also described herein is a substantially pure ribonucleic acid (RNA) construct capable of forming a double-stranded RNA (dsRNA) portion effective in RNAi gene silencing, which RNA construct comprises at least one sequence capable of protecting the dsRNA (portion) against RNA processing. More specifically, the isolated RNA construct comprises at least one dsRNA fragment, wherein the dsRNA comprises annealed complementary strands, one of which is complementary to at least part of the nucleotide sequence of a target sequence, which RNA construct further comprises at least one sequence that protects the dsRNA against RNA processing. Also encompassed are isolated RNA constructs comprising any df the (concatemer) dsRNA molecules described above, which RNA construct further comprises at least one sequence that protects the dsRNA (or dsRNA portion) against RNA processing.

"Protecting against RNA processing" is impeding or hampering or inhibiting the RNA processing. The constructs described herein are protected in the host cell, particularly in a plant cell and/or in a plant pest species.

Whenever a stabilized or protected construct is described, the term "core" refers to the dsRNA portion, which core may comprise at least one dsRNA fragment or which may comprise multiple dsRNA fragments, e.g. a concatemer, as described In detail above.

Also described herein are isolated RNA constructs wherein said at least one sequence (capable of) protecting the dsRNA against RNA processing is chosen from a GC-rich clamp, a short non-complementary loop of between 4 and 100 nucleotides (for instance 4, 5, 6, 7, 8, 9, 10, 15, 20, 30, 40, 50, 60, 70, 80, 90 nucleotides), a mismatch lock and a protein binding RNA structure.

A sequence capable of protecting the dsRNA portion against RNA processing may also be referred to as a "lock".

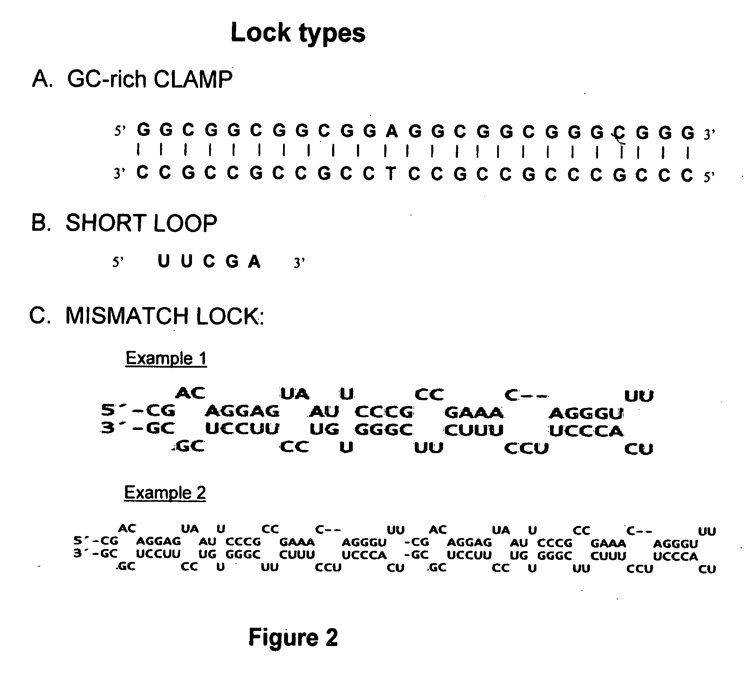

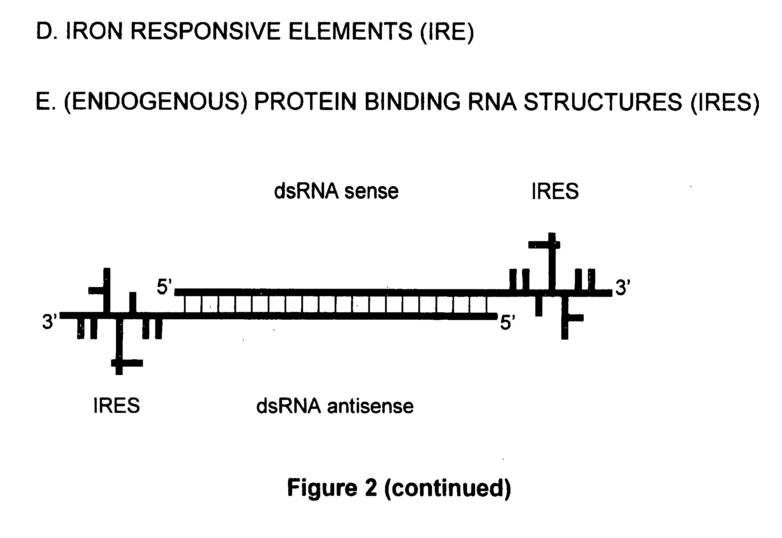

Examples of locks are given below:
1. A "GC-rich" clamp (se Figure 2A) is a stretch of nucleotides with multiple (contiguous) G residues which base pair with a complementary strand comprising multiple (contiguous) C residues, The base pair composition of the GC-rich clamp may vary and the length of the GC-rich clamp may vary from about 5bp to about 1000bp.
2. A "non-complementary loop" (see Figure 2B) capable of protecting the RNA from RNA processing is for example between about 3 nt and about 100 nt in length, preferably is smaller than 9 nt, more preferably is about 4 nt or about 5 nt. The sequence may be chosen randomly or may be homologous to specific sequences such as (conserved) miRNAs.
3. A "mismatch lock" (see Figure 2C) is a dsRNA wherein some nucleotides are not base paired. In a mismatch lock there are just enough matches included in the dsRNA to allow proper dsRNA pairing (preferably about 67% to 74% of the bases are paired). The mismatches consist mainly of insertions and deletions on one strand relative to the other. Viroids (e.g. from the Pospiviroidae, Avsunviroldae, Hepadnavirus family, human hepatitis delta virus, potato spindle tuber viroid, avocado sunblotch viroid or Citrus exocortis viroid) serve as excellent examples In nature to design mismatch locks that slow down the processing of dsRNA in the host species.
One example of a mismatch lock is a lock comprising a sequence as described in Chang et al. (J Virol. 2003 Nov;77(22):11910-7).

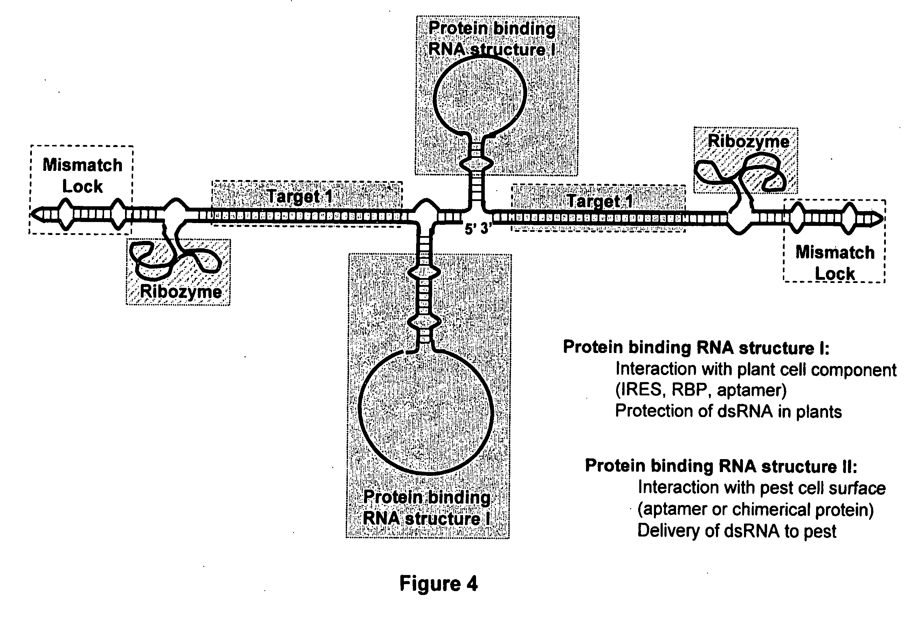

These sequences are derived from potato spindle tuber viroid (PSTVd), avocado sunblotch viroid (ASBVd) or human hepatitis delta virus (HDV) RNAs, have a predicted Intramolecular base-pairing of 70%, 67% and 74% respectively, and are resistant to dicer activity. These sequences are depicted In Figure 4 of Chang et al. and can be used as locks in the constructs described wherein each separately, or combined with each other. Therefore, also described are dsRNA constructs suitable for RNA silencing, which constructs comprise as a sequence capable of protecting the dsRNA against RNA processing, the above mentioned HDV sequence, PSTVd sequence, ASBVd sequence or the combinations HDV- PSTVd- ASBVd or HDV- ASBVd- PSTVd. Examples of such a single mismatch lock are given In Figure 2C, as well as an example of a composed mismatch lock. Another example of a mismatch lock is dsRNA complementary to a target sequence of a target species, which comprises about 70% intramolecular base pairing. For example, the anti-sense strand comprises no mutations and is 100% complementary to the target sequence while the sense strand comprises about 30% mutations causing mismatches In the dsRNA. 4. Another type of locks are protein binding RNA structures. These are RNA sequences that are recognized and bound by proteins, preferably by proteins endogenous to the host cell in which the dsRNA construct is expressed. When these locks are occupied by the binding protein, they protect the dsRNA portion against RNA processing. Examples of such "protein binding RNA locks" are IRES; 5' regions of virus genomes; IRE; plant dsRNA binding domain (e.g. Hyl-1-like domain); endogenous ssRNA binding proteins (or domains) (e.g. transcription factors, translation factors, ribosome components, SRP, PTB domains etc) provided that they are transgenically expressed in a way that does not Interfere with the wild type protein function; and others.

An "IRES" is an internal ribosome entry site. A general representation of IRES comprising dsRNA constructs is given in Figure 2E. Sequences represented by SEQ ID Nos: 1 to 7 represent IRES sequences of CrPV-like viruses. Cricket paralysis Virus like (CrPV-like) IRES sequences HTH are one suitable example of an IRES. The enclosed nucleotides are derived from the following viral genbank nucleotide sequences: PSIV: AB006531, nt 6005-6204; HIPV: AB017037, nt 6286-6484; DCV: AF014388, nt 6078-6278; RhPV: AF022937, nt 6935-7121; TrV: AF178440, nt 5925-6123; CrPV: AF218039, nt 6029-6228; BQCV:AF183905, nt 5647-5848 (Kanamori and Nakashima, RNA. 2001 7(2):266-74). The identifying header is compiled as follows: <Genbank accession number>_<start position>_<stop position> <spedes name>.

Other suitable IRES sequences may be found by a person skilled in the art. Preferred IRES sequences are recognizable by ribosomes of different organisms, preferably recognizable by ribosomes from a plant or from a plant pest species. Examples of plant IRES sequences are IRES sequences of *Arabidopsis thaliana, Cuscuta japonica, Funaria hygrometrica*, *Nicotiana tabacum, Oryza sativa, Triticum aestivum* or *Zea mays* as described in document WO03/020928.

IRES sequences are incorporated in the constructs described herein for Instance in constructs as represented by SEQ ID Nos: 18 to 21.

One example of a 5' region of a virus, or a fragment thereof, useful as a lock in the constructs is described and illustrated in Miller et al. (1998. J. Mol. Biol. 284(3): 591-608). Other examples of IRES sequences that are encompassed are described and Illustrated, for instance, in Spahn et al. (2004. Cell 20 118(4): 465-475). Further, 3' regions of viruses, or fragments thereof, may also be used as a lock. An "IRE" is an Iron Regulatory Element. One IRE suitable as a lock in the constructs described is the IRE element derived from the soy bean NRAMP homologue GmDMT1 as described in Kaiser et at. (Plant J. 2003, 35(3), 295-304).

The sequence of the IRE is represented by SEQ ID NO: 8.

Other examples of protein binding RNA locks are RNA sequences recognized by RNA binding proteins as described for example in Lorkovic and Barta (Nucleic Acids Res. 2002 Feb 1;30(3):623-35). RNA-binding proteins from the flowering plant Arabidopsis thaliana, which have an RNA recognition motif (RRM) or a K homology (KH) domain are described. The corresponding RNA sequences recognized by these proteins may be cloned by techniques well known by a person skilled in the art, for example via the One-Hybrid technique. Figure 4 shows a preferred construct.

The isolated RNA constructs as described above may comprise at least one protecting sequence chosen from the internal ribosome entry sites (IRESes) from the encephalomyocarditis virus (EMCV) and the upstream of N-*ras* (UNR). A sequence comprising at least part of the EMCV-IRES sequence is presented in SEQ ID NO: 13. Constructs comprising at least part of the EMCV-IRES sequence are represented by SEQ ID Nos: 18 and 19.

A sequence comprising at least part of the UNR-IRES sequence is presented in SEQ ID NO: 14. Constructs comprising at least part of the UNR-IRES sequence are represented by SEQ ID Nos: 20 and 21.

The IRES sequence of the EMCV viral genome is represented In the Genbank accession number NC_001479; the IRES sequence of the human UNR genome is represented in the Genbank accession number NM_001007553. The Invention thus relates to the use of the complete IRES sequence or a functional fragment thereof in RNA constructs comprising dsRNA fragments as described above.

Any of the above mentioned locks may be combined with each other to form a composite lock. Specific examples of such compositions are the closed GC clamp or a dosed mismatch lock as represented In the figures.

The length of a lock may vary from about 3 base pairs to about 10,000 base pairs, In the case of double-stranded locks, or from 3 nt to about 10,000 nt In the case of single-stranded locks. The locks may have the extra advantage of causing steric hindrance to the RNA processing machinery of the host cell.

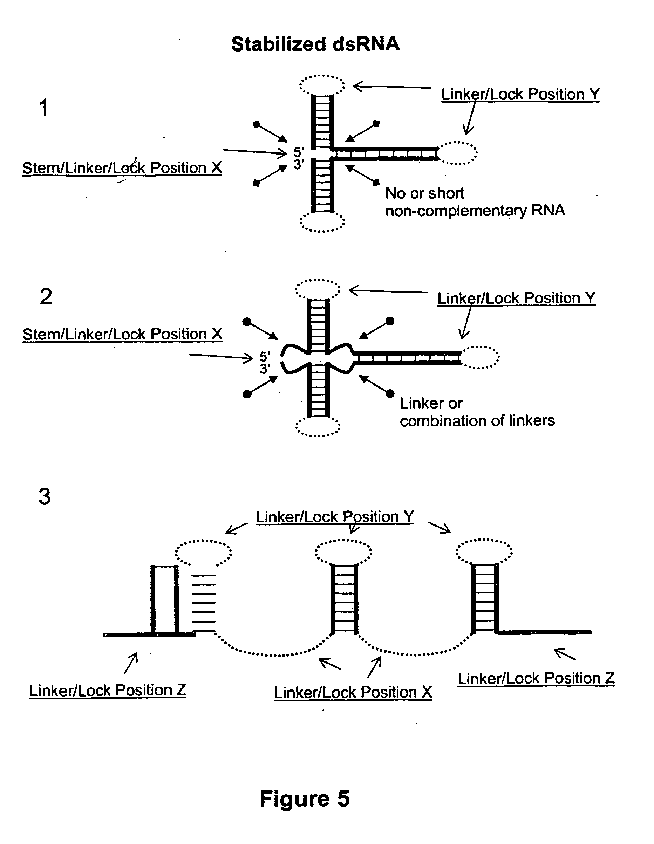

The location of the locks In the constructs described herein may be a terminal position at the extremity of the dsRNA or it might be somewhere embedded (within) In the dsRNA. Accordingly, the position and the number of the locks may vary. Preferably, 2 or 4 locks are present at the extremity (the edge) of the dsRNA portion, in case of a stem (or concatemer) RNA core. Preferably, one lock or a combination of locks Is present as a fourth stem in case of a multi-stem "cloverleaf" dsRNA core type (see for Instance Figure 5 constructs 1 and 2).

Another mechanism of protecting the dsRNA against RNA processing, is to embed the dsRNA fragment effective in gene silencing Into a larger RNA structure which occurs naturally and which is not normally processed or which exhibits reduced processing In its natural environment. Examples of such natural, unprocessed RNAs are siRNA, TRNA, ribosomal RNA, components of the spliceosome or other non-coding RNAs transcribed from RNA polymerase I. II or III promoters. Therefore, also described herein

are natural, unprocessed RNAs comprising a dsRNA fragment complementary to a target sequence, for example a plant pest target sequence, and which is capable of silencing the expression of a target gene. Advantageously, these constructs may provide a camouflage for the dsRNA fragment capable of gene silencing and will contribute to the stability of this dsRNA fragment In the host cell. This approach may be combined with any dsRNA core type exemplified herein and/or with any other sequence capable of protecting dsRNA against RNA processing as exemplified herein and/or with any linker as exemplified herein..

Still another mechanism to protect the dsRNA against RNA processing is the so-called "Triple RNA" construct. The triple RNA comprises 3 parallel RNA strands, which are encoded by two separate RNA strands wherein:
the first RNA strand comprises from 5' to 3'
   (a) a sense RNA core strand corresponding to a target sequence (core), followed by
   (b) a second sense RNA region (B), followed by
   (c) a long non-complementary loop, which loop is
      a. longer that the length of the core RNA, the (B) RNA region and the (A) RNA region together, and
      b. which loop optionally comprises a lock as described hereinabove, such as an IRES,
      c. followed by
   (b) a third sense RNA region (A), and wherein
the second RNA strand comprises from 5' to 3'
   (a) an antisense RNA region (A) complementary to sense RNA region (A),
   (b) an antisense RNA core strand corresponding to the target sequence and complementary to the sense core RNA,
   (c) an antisense RNA region (B) complementary to sense RNA region (B)

Yet another mechanism to protect dsRNA from RNA processing is to embed the dsRNA core in a viroid-like dsRNA structure is described and illustrated for instance in Navarro and Flores (2000 EMBO Journal 19(11) p 2662. The dsRNA may be incorporated within the viroid as such, or in the viroid mutated to avoid internal cleavage (for example by ribozymes) or to avoid translation. Mutations can be based on information from Dais et al. (1991, NAR 19(8), p 1893). These type of constructs may be transported to the chloroplasts, where it can receive extra protection against dsRNA processing.

Another mechanism to protect dsRNA from processing is to signal the dsRNA towards an intracellular compartment of the host cell. For example the dsRNA can be compartmentalized in an intermediate host cell, before it is transferred to the target host ceil. In particular, the dsRNA construct may be compartmentalized in a plant cell, for example, it may be located in the chloroplast, mitochondrion or plastid, before it is transferred to the plant pest species, for example the plant pest nematode or insect. Compartmentalization may occur in a variety of ways, such as for example via the use of viroid structures, or via the use of signal sequences, for example chloroplast, mitochondrial or plastid signal sequences. These organelles are from prokaryotic origin and may offer a protective environment away from the plant RNA processing machinery.

Yet another mechanism to protect the dsRNA from RNA processing is to express sense and antisense separately and to target them to different locations within the host that expresses the sense and the antisense strands. In this embodiment, sense and antisense mRNA fragments corresponding to a selected gene of a particular pest species are cloned behind different promoters driving expression (i) separate plant tissues or (ii) within the same cell but in separate cellular compartments. These promoters are tissue or organel specific and allow strong simultaneous expression in different cellular compartments or in adjacent tissues.

For example, the sense and antisense strands may be targeted to different plant tissues, to different cell types, or to different subcellular organelles or different subcellular locations. For example, in a leaf the sense strand might be expressed in the nerve cells while the antisense is expressed in the palisade tissue.. The advantage of this technique is that the sense and antisense strands never come together in the plant cell, and therefore no degradation or autosilencing or RNA interference can occur within the plant by Dicer. When the pest organisms feeds on the plant, the strands are set free and mixed allowing annealing of dsRNA in the gut lumen, and base pairing between the sense and antisense strands may occur to form long dsRNA. Subsequently this dsRNA may be taken up efficiently and leads to the desired RNAl response, leaing to degradation of the target mRNA in the pest and death of the pest.

This approach can be accomplished by feeding the pest species with two bacterial strains, for instance present in a composition, one strain producing the sense, the other producing the antisense strand.

Any of the dsRNA molecules or RNA constructs herein described, capable of forming a dsRNA portion effective in gene silencing, may further comprise at least one linker, for instance said linker is chosen from a conditionally self-cleaving RNA sequence, such as a pH sensitive linker or a hydrophobic sensitive linker, and an intron.

In the presence of a lock as described herein, the function of the linker may be to set the lock free prior to gene silencing, leading to RNA processing of the dsRNA construct by the intermediate host cell or by the target host cell. In the absence of a lock, for example within the concatemer construct Itself, the function of the linker may be to uncouple the multiple dsRNA fragments and to divide the long dsRNA into pieces effective in gene silencing. Advantageously, in this situation the linker sequence may promote division of the long dsRNA into pieces under particular circumstances, resulting in the release of separate dsRNA fragments under these circumstances and leading to more efficient gene silencing by these smaller dsRNA fragments.

Different linker types for dsRNA constructs are provided by the present invention.

"Conditionally self-cleaving linkers" are RNA sequences capable of being processed under certain conditions.
1. One example of suitable conditionally self-cleavlng linkers is an RNA sequence that is self-cleaving at low pH conditions. Suitable examples of such RNA sequences are described by Jayasena and Gold (Proc Natl Acad Sci USA. 1997 Sep 30; 94(20):10612-7). These are synthetic sequences obtained via cloning of randomized sequences and retrieved via a SELEX protocol (systematic evolution of ligands by exponential enrichment; Gold et al., 1995. Ann. Rev. Biochem. 64: 763-797).
2. Other examples of suitable conditionally self-cleaving linkers are RNA sequences that are self-cleaving at high pH conditions. Suitable examples of such RNA sequences are described by Borda et al. (Nucleic Acids Res. 2003 May 15;31(10):2595-600). One suitable linker sequence originates from the catalytic core of the hammerhead ribozyme HH16. According to one particular embodiment of the present invention, the above-mentioned pH dependent self-cleaving linkers are used in constructs designed to be produced in plants for the control of pest organisms. Here the linkers may be used to disconnect the locks of a stabilized construct or to disconnect the multiple dsRNA fragments of a concatemer construct in the pest organism. According to a particular embodiment the past species has a gut system, such as for example nematodes and insects, and the linker is seff-cleaving in the gut of such pest species, for example a plant pest species. The pH in the gut is variable ranging from extremely acid to extremely basic. Particular insect pest species of interest for application of this technique are stem borers or for instance the tobacco bud worm.
3. Alternatively, the linkers are self-cleaving in the endosomes. This may be advantageous when the constructs of the present Invention are taken up by the pest organisms via endocytosis or transcytosis, and are therefore compartmentalized in the endosomes of the pest species. The endosomes may have a low pH environment, leading to cleavage of the linker.
4. Yet other examples of suitable conditionally self-cleaving linkers are RNA sequences that are self-cleaving in hydrophobic conditions. Suitable examples of such RNA sequences are described by Riepe et al. (FEBS Lett. 1999 Aug 27;457(2):193-9), A highly specific self-cleavage reaction occurs in the hydrophobic interior of a micelle. These RNA sequences are derived from hammerhead and hairpin dbozymes.

The above mentioned linkers that are self cleaving in hydrophobic conditions are particularly useful in dsRNA constructs of the present invention when used to be transferred from one cell to another via the transit in a cell wall, for example when crossing the cell wall of a plant pest organism. Particular plant pest organisms of interest for application of this technique are plant parasitic fungi or plant parasitic viruses or bacteria.

An intron may also be used as a linker. An "intron" as used herein may be any non-coding RNA sequence of a messenger RNA. Particular suitable intron sequences for the constructs of the present invention (1) are U-rich (35-45%); (2) have an average length of 100 bp (varying between about 50 and about 500 bp) which base pairs may be randomly chosen or may be based on known Intron sequences; (3) start at the 5' end with -AG:GT-or -CG:GT- and/or (4) have at their 3' end -AG:GC- or -AG:AA.

According to the invention, a linker sequence may be present between the dsRNA fragments or not. For instance, when present, the linker may comprise a short random nucleotide sequence that is not complementary to target sequences but that is the result of the cloning. In other embodiments, for Instance when the dsRNA comprising the dsRNA fragments is chemically synthesized, the dsRNA fragments may be directly adjacent to each other, without the presence of non-target sequences.

A by itself non-complementary RNA sequence, ranging from about 1 base pair to about 10000 base pairs, for instance of at least 10, 20, 30, 50, 60, 70, 80, 90, 100. 200, 500, 1000,1500, 2000, 3000, 10000 base pairs, or any number In-between, may also be used as a linker.

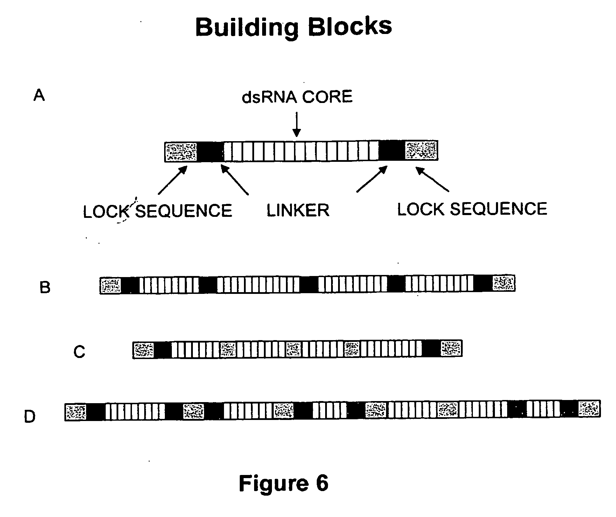

The linker may be located at the edge of the dsRNA construct. Alternatively, the linker may be located between the different dsRNA fragments embedded in the dsRNA. furthermore, as is exemplified in Figure 6, multiple linkers and multiple locks may be located at the edge or within the dsRNA construct.

The linker may be located adjacent to or In the proximity of a lock sequence, more preferably a linker is located adjacent to or in the proximity of each lock sequence.

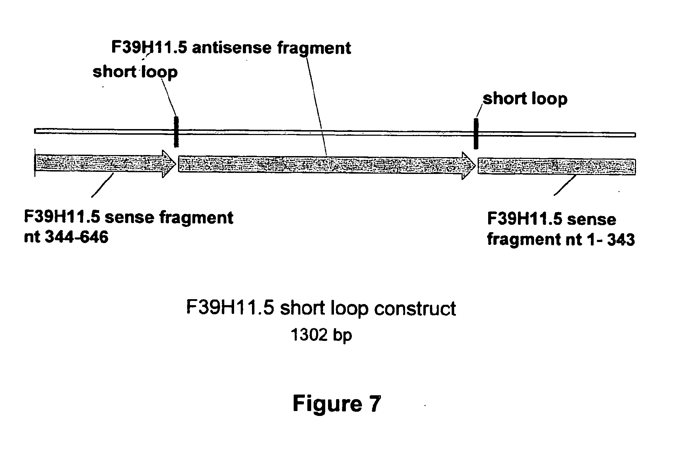

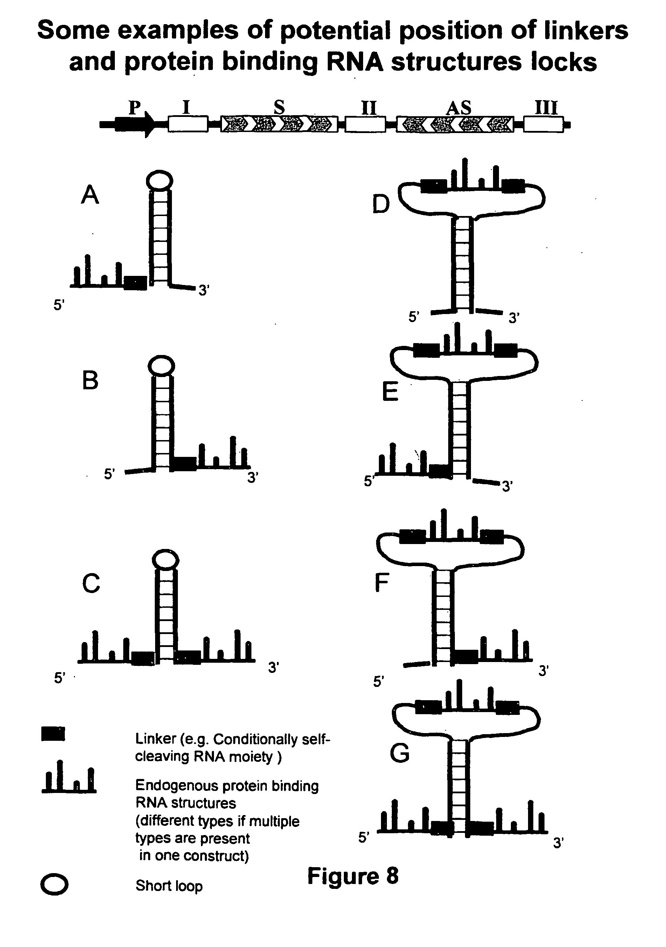

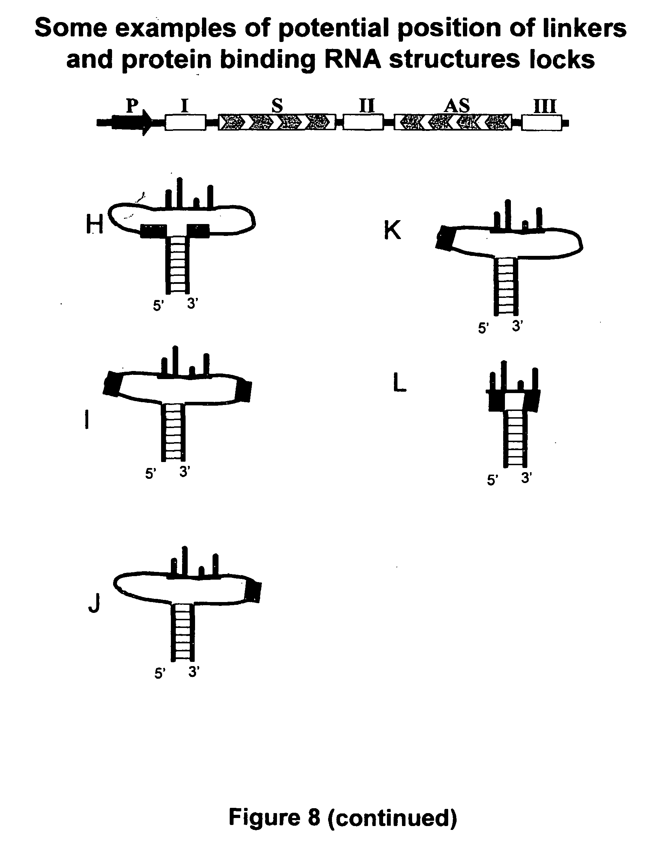

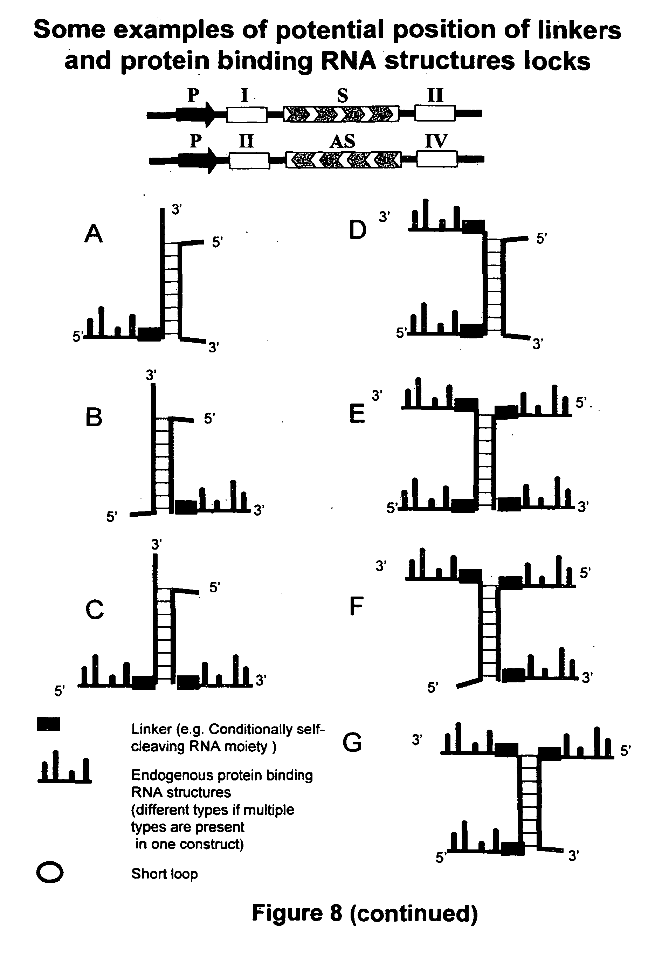

One feature of the concatemer and/or stabilized constructs described herein is that within one concatemer and/or stabilized construct multiple dsRNA core types may be combined and/or multiple lock types may be combined and/or multiple linker types may be combined. For example in a clover-leaf structure any one or more of the 4 dsRNA stems may comprise a GC clamp or a mismatch lock and additionally any one or more of the four dsRNA may comprise a non-complementary loop capable of protecting the RNA construct against RNA processing. This also applies to the dumbbell structure
wherein at least one edge of the dsRNA stem comprises a non-complementary loop which Is capable of protecting the RNA construct against RNA processing (see Figure 7). SEQ ID Nos: 9 to 12 represent different DNA sequences used in the examples described herein. These sequences represent a dumbbell construct with the sense and antisense fragments against beta-tubulin target genes originating from M. incognita, C. *elegans,* hopper and *Magnaporthe grises*. These Constructs further comprise a pH sensitive linker (underlined) and a short loop (boxed). The dumbbell RNA construct may also comprise, on at least one of the edges of the dsRNA stem, a GC clamp or a mismatch lock. Further examples of dsRNA constructs comprising linkers and protein binding RNA sequences are demonstrated in Figure 8.

An interstem base pairing module may be included within the construct described, herein, These interstem base pairing modules contribute to the stability of the dsRNA In the host cell and allow complex dsRNA constructs to fold compactly.

Within the constructs described herein, there may be included a moiety capable of delivering the dsRNA to the pest species. Such constructs are described in patent application of applicant,

The dsRNA construct described herein further comprises at least one aptamer.

The term "aptamer' or "aptamer sequence", or "aptamer domain" are used herein as synonym and are well known to a person of skill In the art. These terms refer to synthetic nucleic acid ligands capable of specifically binding a wide variety of target molecules, such as proteins or metabolites. As used herein aptamers are oligonucleotide sequences with the capacity to recognize virtually any class of target molecules with high affinity and specificity. In a preferred embodiment, the aptamer specifically binds to a structure in the plant tissue or to a structure in the pest species.

Also described herein are dsRNA constructs comprising aptamers that target the dsRNA to a high affinity binding site in the pest species. These can be localized on gut epithelial cells of feeding pests, on other cells in the body of the feeding pest or even on interacting cell surfaces of for instance fungi that feed on plant tissue.

The ds RNA construct thus may comprise an aptamer which allows endocytosis into the gut cell of a pest organism, e.g. an enterocyte. In another example, the aptamer allows (or promotes or enables) transcytosis from the lumen of the gut to the coelumic fluid or haemolymph of the pest organism.

Alternatively, the ds RNA construct may comprise an aptamer which allows endocytosis into a tissue cell of the pest organism, such as for instance, but not limited to, a muscle cell, a gonade cell, a nerve cell. In another example, an aptamer allows (or promotes or enables) transcytosis from an endothelial cell lining an organ to the lumen of said organ of the pest organism.

The dsRNA construct may also comprise at least two aptamers, for instance one aptamer which allows (or promotes or enables) transcytosis from the gut cell of a pest organism to the coelumic fluid or haemolymph of the pest organism, and another aptamer which allows (or promotes or enables) endocytosis into a tissue cell of the pest organism.

Alternatively, the dsRNA can be co-expressed with an RNA delivery molecule consisting of different modules. Such a delivery molecule may consist for example of a polypeptide sequence comprising (i) at least one RNA-binding domain, (ii) at least one targeting polypeptide able to bind to a cellular endocytosis and/or transcytosis receptor molecule and (iii) optionally at least one peptide linker and/or at least one purification tag.

Such a delivery-promoting molecule is used to facilitate the uptake and the correct delivery of double stranded RNA to a suitable target site in a plant-feeding pest organism for the purpose of RNA interference. The terms "RNA delivery module", "RNA delivery molecule" and "RNA delivery vehicle" are used herein as synonym and refer to the multidomain or multimodular protein which binds to the dsRNA mediated silencing molecule.

In one embodiment of the present invention, the RNA delivery molecule consisting of different modules, comprises at least one RNA binding module, at least one targeting module able to be endocytosed and/or transcytosed or able to bind to a cellular endocytosis and/or transcytosis receptor molecule, optionally at least one linker for linking the dsRNA binding module to the targeting module, and optionally a module comprising a purification tag.

One module of the RNA delivery molecule is an RNA binding domain.

An "RNA binding domain" as used herein may bind double-stranded RNA generically or specifically, single-stranded RNA generically or specifically. The RNA binding molecule may bind dsRNA and/or ssRNA structure-specifically.

Preferred examples of RNA binding proteins include but are not limited to coliphage HK022 NUN protein, *Bacillus subtilis* LicT protein, or bacteriophage MS2 coat protein or essential parts, or homologues thereof.

A second module of the RNA delivery molecule comprises a targeting module. The terms "targeting module" and "targeting protein" are used herein as synonyms and both refer to a protein, or an essential part, or a homologue thereof capable of targeting the RNA delivery molecule to a targeting site in a living pest organism.

The targeting module preferably comprises a protein which is capable of being endocytosed and/or transcytosed in a cell of the pest organism, or a protein able to bind an endocytosis and/or transcytosis receptor molecule present on a cell or a tissue of the pest organism, or any combinations thereof.

### Stem-loop-stem structures

One example of a dsRNA or an RNA capable of forming dsRNA is a hairpin construct. A hairpin or "stem-loop-stem" structure is a nucleic acid molecule, preferably an RNA nucleic acid, comprising in 5' to 3' order, a first strand, a loop, and a second strand, wherein said first and second strands hybridize to each other under physiological conditions and said loop connects said first strand to said second strand to form at least one double-stranded RNA region.

When different stem-loop-stem structures are present in one dsRNA molecules, the connection between the stem-loop-stem structures may be in various ways.

For example, they may be chemically cross-linked to form an RNA complex. Alternatively, the multiple stem-loop-stem structures are genetically linked to each other with a linker as mentioned herein above.

In a preferred embodiment, 2 to 20 stem-loop-stem structures may be linked to each other into a "sphere" structure. In a more preferred embodiment, 4 stem-loop-stem structures are linked to each other into a clover-leaf structure, wherein the 5' and 3' edge of the RNA construct forms the fourth dsRNA stem portion. In another embodiment, the clover-leaf structures of the present Invention may comprise at least one GC clamp or mismatch lock or another type of lock as described herein.

The concatemer constructs according to the present invention are particularly useful for the control of plant pest organisms, more particularly in plant pest organisms which are selective In taking up dsRNA. For example, nematodes are selective for the length of the dsRNA to be taken up. It has been demonstrated that fragments of 100 base pairs are not taken up as efficiently as fragments of 200 to 500 base pairs. Also fungi and insects may be selective in the uptake of dsRNA. In view of the selective uptake of dsRNA by some pest organisms, the entire length of the dsRNA constructs described herein, when folded or assembled, is generally between 17 and 20000 base pairs, preferably between 21 and 1000 base pairs. More preferably the length is at least 17 bp, 18 bp, 19 bp, 20 bp, 21 bp, 50 bp, 80 bp, 100 bp, 150 bp, 200 bp, 250 bp, 300 bp, 350 bp, 400 bp, 450 bp, 500 bp, 550 bp, 600 bp, 650 bp or 700 bp, 900 bp, 1000 bp, 1100 bp, 1200 bp, 1300 bp, 1400 bp or 1500 bop. More preferably the length is about 50 bp, 80 bp, 100 bp, 150 bp, 200 bp, 250 bp, 300 bp, 350 bp, 400 bp, 450 bp, or 500 bp. Even more preferably, the total length of any of the dsRNA concatemer and/or stabilized constructs described herein is 150bp, 250bp or 350 bp.

The present invention thus relates to any of the isolated dsRNA or RNA constructs herein described wherein the dsRNA portion has a length between about 17 to 2000 base pairs, preferably between about 50 and 1000 base pairs, more preferably between about 80 and 500 base pairs..

### Target species and pest

The "target species" as used in the present invention, may be any species. Suitable target species are chosen from the group comprising virions, viruses, bacteria, yeast, fungi, insects, protozoa, metazoa (comprising nematodes), algae, plants, animal (including mammals, including humans). Most suitable for the methods of the present invention are target species which are pest organisms, more particularly plant pest organisms, such as nematodes, insects, fungi, bacteria and viruses.

According to a specific embodiment, the invention relates to any of the isolated dsRNA or RNA constructs described, wherein the target sequence or target gene is of a plant pest organism (ie the target species).

"Nematodes" as used herein comprises species of the order *Nematoda.* Many species of nematodes are parasitic and cause health problems to humans and animals (for example species of the orders Ascaradida, Oxyurida, Strongylida, Stronglyloides and Trichocephalida), as well as to plants and fungi (for example species of the orders Aphelenchida, Tylenchida and others). Preferably, "nematodes" as used herein, refers to plant parasitic nematodes and nematodes living in the soil. Plant parasitic nematodes include, but are not limited to, ectoparasites such as *Xiphinema spp*., *Longidorus spp*., and *Trichodorus spp*.; semiparasites such as *Tylenchulus spp*.; migratory endoparasites such as *Pratylenchus spp*., *Radopholus spp*., and *Scutellonerna*. *spp*.; sedentary parasites such as *Heterodera spp*., *Globodera spp*., and *Meloidogyne spp*., and stem and leaf endoparasites such as *Ditylenchus spp*., *Aphelenchoides spp*., and *Hirshmaniella spp.* Most preferably, "nematodes" as used herein, refers to root parasitic soil nematodes such as the cyst-forming nematodes of the genera Heterodera and Globodera and the root knot nematodes of the genus Meloidogyne. The RNA constructs of the present invention are particularly suitable to control harmful species such as *Meloidogyne incognita, Heterodera glycines* (soybean cyst nematode) and *Globodera rostochiensis* (potato cyst nematode). However, the use of the dsRNA constructs according to the invention is in no way restricted to these genera and species, but also extends in the same manner to other nematodes.

"Fungi" as used herein comprises all species of the order Fungi. According to a preferred embodiment of the invention, the target gene originates from a plant parasitic fungus such as *Magnaporthe oryzae* (rice blast, formerly *Magnaporthe grisae*; anamorph *Pyricularia oryzae Cav.* and *Pyricularia grisae*); *Rhizoctonia* spp., particularly *Rhizoctonia solani* and *Rhizoctonia oryzae*; *Gibberella fujikuroi*; *Sclerotinium* spp.; *Helminthosporium sigmoideum; Pythium* spp.; *Alternaria* spp., particularly *Altemaria solani*; *Fusarium* spp., particularly *Fusarium solani and Fusarium germinearum*; *Acremoniella* spp.; *Leptosphaeria salvinii*; *Puccinia* spp., particularly *Puccinia recondita* and *Puccinia striiformis*; *Septoria nodorum*; *Pyrenophora teres*; *Rhincosporium secalis*; *Erysiphe* spp., particularly *Erysiphe graminis*; *Cladosporium* spp.; *Pyrenophora* spp.; *Tilletia* spp.; *Phytophthora spp*., particularly *Phytophthora infestans*; *Plasmopara viticola*; *Uncinula necator*, *Botrytis* cinerea; *Guiguardia bidwellii*; *C*. *viticola; Venturia inaequalis; Erwinia armylovora*; *Podosphaera leucotricha; Venturia pirina; Phakospora sp (soybean rust), Ustilago maydis (corn smut*).

"Insects" as used herein comprises all insect species. According to a preferred embodiment of the invention, the insects are insects that damage plants. Important plant pest insects to be controlled by the methods of the present invention comprise amongst others insects of the order coleoptera, chosen for example from the non-limiting list of *Lissorhopterus oryzophilus, Echinocnemus squamos, Oulema oryzae, Diabrotica spp. (Diabrotica virgifera virgifera, Daibrotica undecimpunctata howardi, Diabrotica barberi), Chaetocnema pulicaria, Sitophilus zeamais, Anthonomus grandis, Epilachna varivestis, Cerotoma trifurcata, Leptinotarsa decemlineata.* Alternatively, the plant pest insects to be controlled by the methods of the present invention belongs to the order of Homoptera. More particularly, the homoptera insect is chosen from the non-limiting list of *Nilaparvata lugens, Laodelphax striatellus, Sogatella furcifera, Nephotettix virescens, Rhopalosiphum maidis, Aphis spp. (Aphis gossypii, Aphis glycines), Empoasca spp. (Empoasca fabae,* Empoasca *solana), Bemisia tabaci, Myzus persicae, Macrosiphum euphorbiae.* The plant pest insects to be controlled by the methods of the present invention may also belong to the order of Leptidoptera, chosen for example from the non-limiting list of *Heliothis spp*., *Helicoverpa spp., Spodoptera spp., Ostrinia spp., Pectinophora spp, Agrotis spp., Scirphophaga spp., Cnaphalocrocis spp.,* Sesamia *spp*, *Chilo spp*., *Anticarsia spp*., *Pseudoplusia spp*., *Epinotia spp*., and *Rachiplusia spp*., preferably *Heliothis virescens*, *Helicoverpa zea, Helicoverpa armigera, Helicoverpa punctera, Ostrinia nubilafis, Spodoptera frugiperda, Agrotis ipsilon, Pectinophora gossypiella, Scirphophaga incertulas*, *Cnaphalocrocis medinalis*, *Sesamia inferens, Chilo partellus*, *Anticarsia gemmatalis*, *Pseudoplusia includens*, *Epinotia aporema* and *Rachiplusia nu*. The RNA constructs of the present invention are particularly suitable to control harmful species such as the rice brown planthopper (*Nilaparvata lugens*), rice striped stem borer (*Chilo suppressalis*) and Colorado potato beetle (*Leptinotarsa delineata*).

"Bacteria" that damage plants and that can be controlled with the constructs and methods of the present invention are for example *Agrobacterium* ssp.; *Arachnia* ssp.; *Clavibacter* ssp.; *Corynebacterium* ssp.; *Erwinia* ssp.; *Fusobacterium* ssp.; *Hafnia* ssp.; *Pseudomonas* ssp.; *Spiroplasma* ssp.; *Streptomyces* ssp.; *Xanthomonas* ssp.; *Xylella* ssp. and *Xylophilus* ssp.

"Viruses" that damage plants and that can be controlled with the constructs and methods of the present invention are for example African cassava mosaic virus; Alfalfa mosaic virus; American plum line pattern virus; Andean potato latent virus; Andean potato mottle virus; Apple chlorotic leaf spot virus; Apple mosaic virus; Apple stem grooving virus; Arabis mosaic virus; Arracacha virus B, oca strain; Asparagus virus 2; Australian grapevine viroid; Avocado sunblotch viroid; Barley mild mosaic virus; Barley stripe mosaic virus; Barley yellow dwarf virus; Barley yellow mosaic virus; Bean common mosaic virus; Bean golden mosaic virus; Bean leaf roll virus; Bean pod mottle; Bean yellow mosaic virus; Bearded iris mosaic virus; Beet curly top virus; Beet leaf curl virus; Beet mosaic virus; Beet necrotic yellow vein virus; Beet pseudo yellows virus; Beet western yellows virus; Beet yellow stunt virus; Belladona mottle virus; Black rasberry latent virus; Blight (et analogues/en analoge); Blueberry leaf mottle virus; Broad bean wilt virus; Bromoviruses; Cacao swollen shoot virus; Cacao yellow mosaic virus; Cactus virus X; Cadan-cadang viroid; Camation cryptic virus; Camation etched ring virus; Camation latent virus; Camation mottle virus; Camation necrotic fleck virus; Camation ringspot virus; Camation vein mottle virus; Cassava common mosaic virus; Cauliflower mosaic virus; Cherry leafroll virus; Cherry rasp leaf virus; Cherry rasp leaf virus (American); Cherry rugose virus; Chrysanthemum B virus; Chrysanthenum stunt viroid; Citrus exocortis viroid; Citrus leaf rugose virus; Citrus mosoie virus; Citrus tristeza virus (European isolates); Citrus tristeza virus (non-European isolates); Citrus variegation virus; Citrus veinenation woody gall; Citrus viroids; Clover Yellow vein virus; Cocksfoot mild mosaic virus group; Cocksfoot streak virus; Cowpea mild mottle virus; Cucumber mosaic virus; Cucumber yellows virus; Cucumovirus satellites; Cymbidium mosaic virus; Dahlia mosaic virus; Dasheen mosaic virus; Dianthoviruses; Echtes Ackerbohnenmosaic virus; Elderberry carlavirus; Euphorbia mosaic virus; Florida tomato virus; Grapevine algerian latent virus; Grapevine bulgarian latent virus; Grapevine fanleaf virus; Grapevine flavescence dorée mycoplasm; Grapevine leafroll associated virus (I to V); Grapevine tunusian ringspot virus; Grapevine virus A; Grapevine yellow speckle viroids (I & II); Grapewine chrome mosaic virus; Heracleum latent virus; Hippeastrum mosaic virus; Honeysuckle latent virus; Hop (American) latent virus; Hop latent virus; Hop mosaic virus; Hop stunt viroids; Hop virus A; Hop virus C; Hydrangea ringspot virus; lliaviruses; Iris mild mosaic virus; Leek yellow stripe virus; Leprosis; Lettuce infectious yellows virus; Lettuce mosaic virus; Lilac chlorotic leafspot virus; Lilac ring mottle virus; Lilly symptomless virus; Luteovirus satellites; Maize dwarf mosaic virus; Maize streak virus; Marafiviruses; Melon necrotic spot virus; Myrobolan latent ringspot virus; Narcissus latent virus; Narcissus mosaic virus; Narcissus tip necrosis virus; Narcissus yellow stripe virus; Oat golden stripe virus; Oat mosaic virus; Odontoglossum ringspot virus; Olive latent ringspot virus; Onion yellow dwarf virus; Papaya mosaic virus; Papaya ringspot virus; Parsnip yellow fleck virus; Pea early browning virus; Pea enation mosaic virus; Pea seed borne mosaic virus; Peach mosaic virus (American); Pear decline mycoplasm; Pelargonium leaf curl virus; Pepper mild tigré virus; Plant reoviruses; Plum line pattern virus (American); Plum pox virus; Poinsettia mosaic virus; Poplar mosaic virus; Potato aucuba mosaic virus; Potato black ringspot virus; Potato leafroll virus; Potato leafroll virus (non European isolates); Potato mop-top virus; Potato spindle tuber viroid; Potato virus A; Potato virus A (non European isolates); Potato virus M; Potato virus M (non european isolates); Potato virus S; Potato virus S (non European isolates); Potato virus T; Potato virus X; Potato virus X (non European isolates); Potato virus Y; Potato virus Y (non European isolates); Potato yellow dwarf virus; Potato yellow mosaic virus; Prune dwarf virus; Prunus necrotic ringspot virus; Raspberry bushy dwarf virus; Raspberry leaf curl virus (American); Raspberry ringspot virus; Raspberry vein chlorosis virus; Red clover mottle virus; Red clover vein mosaic virus; Ribgrass mosaic virus; Rice stripe virus group; Rubus yellow net virus; Saguro cacao virus; Satellites (andere dan geciteerde); Satsuma dwarf virus; Shallot latent virus; Sharka virus; Sobemoviruses; Sowbane mosaic virus; Sowthistle yellow vein virus; Spinach latent virus; Squash leaf curl virus; Stolbur mycoplasm; Strawberry crinkle virus; Strawberry latent C virus; Strawberry latent ringspot virus; Strawberry mild yellow edge virus; Strawberry vein banding virus; Sugar beet yellows virus; Tater leaf virus; Tobacco etch virus; Tobacco mosaic virus; Tobacco necrosis virus; Tobacco rattle virus; Tobacco ringspot virus; Tobacco streak virus; Tobacco stunt virus; Tomato apical stunt viroid; Tomato aspermy virus; Tomato black ring virus; Tomato bunchy top viroid; Tomato bushy stunt virus; Tomato mosaic virus; Tomato planta macho viroid; Tomato ringspot virus; Tomato spotted wilt virus; Tomato yellow leaf curf virus; Tulare apple mosaic virus; Tulip breaking virus; Turnip crinkle virus satellites; Turnip crinkle virus; Turnip mosaic virus; Turnip yellow mosaic virus; Tymoviruses; Velvet tobacco mottle virus; other Viroids; Watermelon mosaic virus 2; Wheat dwarf virus; Wheat soil-bome mosaic virus; Wheat spindle steak mosaic virus; Wheat yellow mosaic virus; White clover mosaic virus; Yam mosaic virus; Zucchini yellow fleck virus; and Zucchini yellow mosaic virus.

### Recombinant DNA constructs

According to a further aspect of the present invention, there is provided an isolated nucleic acid ((deoxyribonucleic acid (DNA)) encoding any of the dsRNA or dsRNA constructs described herein. In addition, the present invention also provides recombinant DNA constructs, for instance expression constructs, comprising said nucleic acid(s).

The expression constructs, also encompassed by the expression "recombinant DNA construct", facilitate introduction into a plant cell and/or facilitate expression and/or facilitate maintenance of a nucleotide sequence encoding a dsRNA construct according to the invention. Accordingly, there is provided a recombinant DNA construct (e.g. an expression construct) comprising a nucleic acid encoding a dsRNA or RNA construct as described herein, operably linked to one or more control sequences capable of driving expression of the above nucleic acid, and optionally a transcription termination sequence. Preferably, the control sequence is selected from the group comprising constitutive promoters or tissue-specific promoters as described herein.

Therefore, the present invention also relates to a transgene encoding any of the double-stranded RNA or RNA constructs described herein, placed under the control of a strong constitutive promoter such as any selected from the group comprising the CaMV35S promoter, doubled CaMV35S promoter, ubiquitin promoter, actin promoter, rubisco promoter, GOS2 promoter, Figwort mosaic viruse (FMV) 34S promoter.

The expression constructs may be inserted into a plasmid or a vector, which may be commercially available. According to one embodiment of the present invention, the expression construct is a plant expression vector, suitable for transformation into plants and suitable for maintenance and expression of an RNA construct according to the present invention in a transformed plant cell.

The term "control sequence" as used herein is to be taken in a broad context and refers to regulatory nucleic acid sequences capable of driving and/or regulating expression of the sequences to which they are ligated and/or operably linked. Encompassed by the aforementioned term are promoters and nucleic acids or synthetic fusion molecules or derivatives thereof which activate or enhance expression of a nucleic acid, so called activators or enhancers. The term "operably linked" as used herein refers to a functional linkage between the promoters sequence and the gene of interest, such that the promoter sequence and the gene of interest, such that the promoter sequence is able to initiate transcription of the dsRNA construct. According to one embodiment of the present invention, the control sequence is operable in a plant; preferably the control sequence is derived from a plant sequence. The term "control sequence" encompasses a promoter or a sequence capable of activating or enhancing expression of a nucleic acid molecule in a cell, tissue or organ.

By way of example, the transgene nucleotide sequence encoding the double-stranded RNA or RNA construct may be placed under the control of an inducible or growth or developmental stage-specific promoter which permits transcription of the dsRNA to be turned on, by the addition of the inducer for an inducible promoter or when the particular stage of growth or development is reached.

Furthermore, when using the methods of the present invention for developing transgenic plants resistant against pests, it might be beneficial to place the nucleic acid encoding the double-stranded RNA according to the present invention under the control of a tissue-specific promoter. In order to improve the transfer of the dsRNA from the plant cell to the pest, the plants could preferably express the dsRNA in a plant part that is first accessed or damaged by the plant pest. In case of a plant pathogenic pest, preferred tissues to express the dsRNA are the roots, leaves and stem. In case of plant pathogenic sucking pests, the dsRNA may be expressed in the phloem under the control of a promoter directing the expressed dsRNA to the phloem. Therefore, in the methods of the present invention, a plant tissue-preferred promoter may be used, such as a root specific promoter, a leaf specific promoter or a stem-specific promoter. Suitable examples of a root specific promoter are PsMTA (Fordam-Skelton, A.P., et al., 1997 Plant Molecular Biology 34: 659-668.) and the Class III Chitinase promoter. Examples of leaf- and stem-specific or photosynthetic tissue-specific promoters that are also photoactivated are promoters of two chlorophyll binding proteins (cab1 and cab2) from sugar beet (Stahl D.J., et al., 2004 BMC Biotechnology 2004 4:31), ribulose-bisphosphate carboxylase (Rubisco), encoded by rbcS (Nomura M. et al., 2000 Plant Mol. Biol. 44: 99-106), A (gapA) and B (gapB) subunits of chloroplast glyceraldehyde-3-phosphate dehydrogenase (Conley T.R. et al. 1994 Mol. Cell Biol. 19: 2525-33; Kwon H.B. et al. 1994 Plant Physiol. 105: 357-67), promoter of the *Solanum tuberosum* gene encoding the leaf and stem specific (ST-LS1) protein (Zaidi M.A. et al., 2005 Transgenic Res. 14:289-98), stem-regulated, defense-inducible genes, such as JAS promoters (patent publication no. 20050034192/US-A1), flower-specific promoters such as chalcone synthase promoter (Faktor O. et al. 1996 Plant Mol. Biol. 32: 849) and fruit-specific promoters such as that of RJ39 from strawberry (WO 98 31812).

In addition, the present invention relates to a recombinant DNA construct wherein said regulatory sequence is selected from the group comprising tissue specific promoters such as any selected from the group comprising root specific promoters of genes encoding PsMTA Class III Chitinase, photosynthetic tissue-specific promoters such as promoters of cab1 and cab2, rbcS, gapA, gapB and ST-LS1 proteins, JAS promoters, chalcone synthase promoter and the promoter of RJ39 from strawberry.

In yet other embodiments of the present invention, other promoters useful for the expression of dsRNA are used and include, but are not limited to, promoters from an RNA Poll, an RNA PolII, an RNA PolIII, T7 RNA polymerase or SP6 RNA polymerase. According to a specific embodiment, the nucleic acid is cloned between two regulatory sequences that are in opposite direction with respect to each other, said regulatory sequences operably linked to said nucleic acid and aid regulatory sequences independently selected from the group comprising RNA Poll, an RNA PolII, an RNA PolII, T7 RNA polymerase or SP6 RNA polymerase. These promoters are typically used for in vitro-production of dsRNA, which dsRNA is then included in an antipesticidal agent, for example in an anti-pesticidal liquid, spray or powder.

Therefore, the present invention also encompasses a method for generating any of the double-stranded RNA or RNA constructs of the invention. This method comprises the steps of:
a. contacting an isolated nucleic acid or a recombinant DNA construct of the invention with cell-free components; or
b. introducing (e.g. by transformation, transfection or injection) an isolated nucleic acid or a recombinant DNA construct of the invention in a cell,
under conditions that allow transcription of said nucleic acid or recombinant DNA construct to produce the dsRNA or RNA construct.

Accordingly, the present invention also encompasses a cell, e.g. a host cell, comprising any of the dsRNA molecules, RNA constructs, nucleotide sequences or recombinant DNA constructs described herein. The invention further encompasses prokaryotic cells (such as, but not limited to, gram-positive and gram-negative bacterial cells) and eukaryotic cells (such as, but not limited to, yeast cells or plant cells). Preferably said cell is a bacterial cell or a plant cell. The present invention also encompasses a transgenic plant, reproductive or propagation material for a transgenic plant comprising such a plant cell.

Optionally, one or more transcription termination sequences may also be incorporated in the expression construct. The term "transcription termination sequence" encompasses a control sequence at the end of a transcriptional unit, which signals 3' processing and polyadenylation of a primary transcript and termination of transcription. Additional regulatory elements, such as transcriptional or translational enhancers, may be incorporated in the expression construct.

The expression constructs of the invention may further include an origin of replication which is required for maintenance and/or replication in a specific cell type. One example is when an expression construct is required to be maintained in a bacterial cell as an episomal genetic element (e.g. plasmid or cosmid molecule) in a cell. Preferred origins of replication include, but are not limited to, f1-ori and colE1 ori.

The expression,construct may optionally comprise a selectable marker gene. As used herein, the term "selectable marker gene" includes any gene, which confers a phenotype on a cell in which it is expressed to facilitate the identification and/or selection of cells, which are transfected or transformed, with an expression construct of the invention. Suitable markers are markers that confer antibiotic or herbicide resistance or visual markers. Examples of selectable markers include neomycin phosphotransferase (nptII), hygromycin phosphotransferase (hpt) or Basta. Further examples of suitable selectable markers include resistance genes against ampicillin (Ampr), tetracycline (Tc^{r}), kanamycin (Kan^{r}), phosphinothricin, and chloramphenicol (CAT). Other suitable marker genes provide a metabolic trait, for example manA. Visual marker genes may also be used and include for example beta-glucuronidase (GUS), luciferase and Green Fluorescent Protein (GFP).

### Transgenic cells and plants

The present invention also relates to a plant comprising at least one dsRNA, at least one RNA construct, at least one nucleic acid or at least one recombinant DNA construct or plant cell described herein. The invention also relates to a a seed, or a plant cell comprising any of the nucleotide sequences or recombinant DNA constructs encoding any of the dsRNA or RNA constructs described herein. Plants that have been stably transformed with a transgene encoding the dsRNA may be supplied as seed, reproductive material, propagation material or cell culture material which does not actively express the dsRNA but has the capability to do so.

The term "plant" as used herein encompasses a plant cell, plant tissue (including callus), plant part, whole plant, ancestors and progeny. A plant part may be any part or organ of the plant and include for example a seed, fruit, stem, leaf, shoot, flower, anther, root or tuber. The term "plant" also encompasses suspension cultures, embryos, meristematic regions, callus tissue, gametophytes, sporophytes, pollen, and microspores. The plant as used herein refers to all plants including algae, ferns and trees. In a preferred embodiment the plant belongs to the superfamily of Viridiplantae, further preferably is a monocot or a dicot. According to one embodiment of the present invention, the plant is susceptible to infestation by a plant pest, for instance a plant pathogenic nematode, fungus or Insect. Particular plants useful in the methods of the present invention are crop plants Including for example monocots such as sugar cane and cereals (including wheat, oats, barley, sorghum, rye, millet, corn, rice, love grass or crabgrass) and dicots such as potato, banana, tomato, vine, apple, pear, soybean, canola, alfalfa, rapeseed and cotton. Particular trees that can be used In the methods of the present Invention are pine, eucalyptus and poplar.

"Administering" a DNA to a cell may be achieved by a variety of means, each well known by the person skilled in the art. Examples of useful techniques are shot-gun, ballistics, electroporation, transfection and transformation. For particular embodiments of the present invention where the cell is a plant cell, general techniques for expression of exogenous double-stranded RNA In plants for the purposes of RNAi are known in the art (see Baulcombe D, 2004, Nature. 431(7006):356-63. RNA silencing in plants.

More particularly, methods for expression of double-stranded RNA in plants for the purposes of down-regulating gene expression in plant pests such as nematodes or insects are also known in the art. Similar methods can be applied in an analogous manner in order to express double-stranded RNA in plants for the purposes of down-reguiating expression of a target gene in a plant pathogenic fungus. In order to achieve this effect it is necessary only for the plant to express (transcribe) the double-stranded RNA in a part of the plant which will come into direct contact with the fungus, such that the double-stranded RNA can be taken up by the fungus. Depending on the nature of the fungus and its relationship with the host plant, expression of the dsRNA could occur within a cell or tissue of a plant within which the fungus is also present during its life cycle, or the RNA may be secreted Into a space between cells, such as the apoplast, that is occupied by the fungus during its life cycle. Furthermore, the dsRNA may be located In the plant cell, for example in the cytosol, or in the plant cell organelles such as a chloroplast, mitochondrion, vacuole or endoplastic reticulum.

Alternatively, the dsRNA may be secreted by the plant cell and by the plant to the exterior of the plant. As such, the dsRNA may form a protective layer on the surface of the plant.

The present invention thus relates to a method for the production of a transgenic cell or organism, comprising the step of administering a recombinant DNA construct described herein to said cell or organism. Preferably, said cell is a plant cell or said organism is a plant. The invention further relates to any transgenic cell or transgenic organism obtainable by the above described method, preferably said transgenic cell or organism is plant cell or plant organism.

The methods of the present invention for the production of transgenic organism may further comprise the steps of cultivating the transgenic cell under conditions promoting growth and development. Where the transgenic organism is a plant, these methods may further comprise the steps of regenerating a plant from plant tissue, allowing growth to reach maturity and to reproduce. Alternatively, the transgenic plant tissue may take other forms or may form part of another plant, examples of which are chimera plants and grafts (for example a transformed rootstock grafted to an untransformed scion).

### Compositions

According to one embodiment, the invention relates to a composition comprising at least one dsRNA or an RNA construct described herein and a physiological or agronomical acceptable carrier, excipient or diluent. The invention also encompasses the use of said composition as a pesticide for a plant or for propagation or reproductive material of a plant.

According to yet another embodiment, the invention relates to a composition comprising at least one nucleic acid or recombinant DNA construct described herein, and a physiological or agronomical acceptable carrier, excipient or diluent.

The composition may contain further components which serve to stabilise the dsRNA and/or prevent degradation of the dsRNA during prolonged storage of the composition.

The composition may still further contain components which enhance or promote uptake of the dsRNA by the pest organism. These may include, for example, chemical agents which generally promote the uptake of RNA into cells e.g. lipofectamin etc., and enzymes or chemical agents capable of digesting the fungal cell wall, e.g. a chitinase.

The composition may be in any suitable physical form for application to the pest, to substrates, to cells (e.g. plant cells), or to organism infected by or susceptible to infection by a pest species.

It is contemplated that the "composition" of the invention may be supplied as a "kit-of-parts" comprising the double-stranded RNA in one container and a suitable diluent or carrier for the RNA in a separate container. The invention also relates to supply of the double-stranded RNA alone without any further components. In these embodiments the dsRNA may be supplied in a concentrated form, such as a concentrated aqueous solution. It may even be supplied in frozen form or in freeze-dried or lyophilised form. The latter may be more stable for long term storage and may be de-frosted and/or reconstituted with a suitable diluent immediately prior to use.

The present invention further relates to the medical use of any of the double-stranded RNAs, double-stranded RNA constructs, nucleotide sequences, recombinant DNA constructs or compositions described herein.

In particular, the present invention relates to pesticidal compositions developed to be used in agriculture or horticulture. These pesticidal compositions may be prepared in a manner known per se. For example, the active compounds can be converted into the customary formulations, such as solutions, emulsions, wettable powders, water dispersible granules, suspensions, powders, dusting agents, foaming agents, pastes, soluble powders, granules, suspo-emulsion concentrates, microcapsules, fumigants, natural and synthetic materials impregnated with active compound and very fine capsules and polymeric substances.

Furthermore, the pesticidal compositions according to the present invention may comprise a synergist. The dsRNA or dsRNA constructs according to the invention, as such or in their formulations, can also be used in a mixture with known fungicides, bactericides, acaricides, nematicides or insecticides, to widen, for example, the activity spectrum or to prevent the development of resistance. In many cases, this results in synergistic effects, i.e. the activity of the mixture exceeds the activity of the individual components.

Additionally the active compounds according to the invention, as such or in their formulations or above-mentioned mixtures, can also be used in a mixture with other known active compounds, such as herbicides, fertilizers and/or growth regulators.

The present invention also relates to fibrous pesticide composition and its use as pesticide, wherein the fibrous composition comprises a non-woven fiber and an effective amount of at least one of the dsRNAs or dsRNA constructs described herein, covalently attached or stably adsorbed to the fiber. In an embodiment, the fibrous composition comprises at least two dsRNAs or dsRNA constructs as described herein.

In a further particular embodiment, the fiber is biodegradable and the adsorbed dsRNA or dsRNA construct as described herein, can be slowly released into a localized area of the environment to control pests in that area over a period of time.

The present invention also encompasses solid formulations of slow-release pesticidal compound as described herein, and their use as pesticide. The formulations release the compound as described herein (a) into the environment (soil, aqueous medium, plants) in a controlled and slow fashion (complete release within several days up to a few months). To prepare the slow release formulations, all components can either be molten together directly in the form of a physical mixture or mixed with the pre-formed polymer melt and then extruded.

The present invention also relates to surfactant-diatomaceous earth compositions for pesticidal use in the form of dry spreadable granules comprising at least one dsRNA or dsRNA construct compound, or at least two dsRNAs or dsRNA constructs compounds as described herein. The granules comprises in addition to the diatomaceous earth, a surfactant composition designed to provide binding, rewetting and disintegration properties to the granules. By diatomaceous earth is meant a silica material characterized by a large surface area per unit volume. Diatomaceous earth is a naturally occurring material and consists mainly of accumulated shells or frustules of intricately structured amorphous hydrous silica secreted by diatoms.

The dry spreadable granules can be prepared by standard pan granulation process, or by homogeneous extrusion process. Of note, granules that are prepared in the absence of a pesticide by extrusion process can subsequently be sprayed with dsRNA(s) or dsRNA construct(s) to adhere same to the granules.

The present invention also provides solid, water-insoluble lipospheres and their use as pesticide, wherein said lipospheres are formed of a solid hydrophobic core having a layer of a phospholipid embedded on the surface of the core, containing at least one dsRNA or dsRNA construct as described herein in the core, in the phospholipid, adhered to the phospholipid, or a combination thereof. In an embodiment, said liposphere comprises at least two dsRNAs or dsRNA constructs as described herein.

The pesticidal compound containing lipospheres have several advantages including stability, low cost of reagents, ease of manufacture, high dispersibility in an aqueous medium, a release rate for the entrapped compound that is controlled by the phospholipid coating and the carrier.

The invention further relates to pesticidal formulations in the form of microcapsules having a capsule wall made from a urea/dialdehyde precondensate and comprising at least one compound as described herein.

In one specific embodiment, the composition may be a coating that can be applied to a substrate in order to protect the substrate from infestation by a pest species and/or to prevent, arrest or reduce pest growth on the substrate and thereby prevent damage caused by the pest species. In this embodiment, the composition can be used to protect any substrate or material that is susceptible to infestation by or damage caused by a pest species, for example foodstuffs and other perishable materials, and substrates such as wood. One example of such pest species are fungi. Preferred target fungal species for this embodiment include, but are not limited to, the following: *Stachybotrys* spp., *Aspergillus* spp., *Alternaria* spp. or *Cladosporium* spp.

The nature of the excipients and the physical form of the composition may vary depending upon the nature of the substrate that is desired to treat. For example, the composition may be a liquid that is brushed or sprayed onto or imprinted into the material or substrate to be treated, or a coating that is applied to the material or substrate to be treated.

### Methods

The present invention further encompasses a method for treating and/or preventing fungal infestation on a substrate comprising applying an effective amount of any of the compositions described herein to said substrate.

The present invention also relates to methods for treating and/or preventing pest growth and/or pest infestation of a plant or propagative or reproductive material of a plant comprising applying an effective amount of a double-stranded RNA, a, RNA construct, or a composition as described herein to a plant or to propagation or reproductive material of a plant.

The present invention also relates to methods for treating and/or preventing pest infestation on a substrate comprising applying an effective amount of a double-stranded RNA, a, RNA construct, or a composition as described herein to said substrate.

In another embodiment, the invention relates to a method for controlling pest growth on a cell or an organism or for preventing pest infestation of a cell or an organism susceptible to infection to said pest species, comprising contacting said pest species with any of the double-stranded RNAs or dsRNA constructs described herein, whereby the double-stranded RNA or RNA construct is taken up by said pest species and thereby controls growth or prevents infestation.

In yet another embodiment, the invention relates to a method for down-regulating expression of at least one target gene in a pest species, comprising contacting said pest species with any of the double-stranded RNAs or dsRNA constructs described herein, whereby the double-stranded RNA or RNA construct is taken up by the pest species and thereby down-regulates expression of the pest target gene(s).

As illustrated in the examples, bacteria can be engineered to produce any of the dsRNA or dsRNA constructs of the invention. These bacteria can be eaten by the pest species.

When taken up, the dsRNA can initiate an RNAi response, leading to the degradation of the target mRNA and weakening or killing of the feeding pest.

Therefore, in a more specific embodiment, said double-stranded RNA or RNA construct is expressed by a prokaryotic, such as a bacterial, or eukaryotic, such as a yeast, host tell or host organism.

Some bacteria have a very close interaction with the host plant, such as symbiotic Rhizobium with the Legminosea (for example Soy). Such recombinant bacteria could be mixed with the seeds (ie coating) and used as soil improvers. Alternatively, dsRNA producing bacteria can be sprayed directly onto the crops, for instance Bacillus thuringiensis species. Possible applications include intensive greenhouse cultures, for instance crops that are less interesting from a GMO point of view, as well as broader field crops such as soy.

This approach has several advantages, eg: since the problem of possible dicing by a plant host is not present, it allows the delivery of large dsRNA fragments into the gut lumen of the feeding pest; the use of bacteria as insecticides does not involve the generation of transgenic crops, especially for certain crops where transgenic variants are difficult to obtain; there is a broad and flexible application in that different crops can be simultaneously treated on the same field and/or different pests can be simultaneously targeted, for instance by combining different bacteria producing distinct dsRNAs.

According to another specific embodiment, the invention encompasses the GMO approaches and thus relates to a method as described above wherein said double-stranded RNA is expressed by said cell or organism infested with or susceptible to infestation by said pest species, for Instance said cell Is a plant cell or said organism is a plant.

The invention further relates to any of the methods described above, wherein said double-stranded RNA or RNA construct is expressed from at least one recombinant DNA construct as described. In further embodiments of the invention, the dsRNA or dsRNA construct is expressed from two (or more) DNA constructs and the annealed transcripts form the double stranded RNA or RNA construct.

The invention further relates to a method for producing a plant resistant against a plant pathogenic pest, comprising:
a) transforming a plant cell with a recombinant DNA construct of any of claims 11 to 14,
b) regenerating a plant from the transformed plant cell; and
c) growing the transformed plant under conditions suitable for the expression of the recombinant DNA construct, said grown transformed plant resistant to said pest compared to an untransformed plant

In another embodiment the present invention encompasses plants comprising more than one dsRNA, dsRNA construct or recombinant DNA construct, each comprising or encoding a single dsRNA fragment; said plants can be obtained by cross-breeding at least two transgenic plants. Said recombinant DNA constructs may comprise distinct regulatory sequences. Said, recombinant DNA constructs may have a distinct origin (ie originating from distinct plasmids or vectors or expression vectors).

The present invention also encompasses methods for producing transgenic plants wherein the recombinant DNA construct comprises, between the left and right border of for instance the plant expression sequences, more than one dsRNA or dsRNA construct comprising multiple dsRNA fragments, which dsRNA fragments may be the same or different; or wherein each of the dsRNA or dsRNA constructs within the one recombinant DNA construct, comprises the same dsRNA fragment.

The invention further relates to a method for increasing plant yield comprising introducing in a plant any of the nucleotide sequences or recombinant DNA constructs of the invention in an expressible format.

The invention also relates to the use of a double stranded RNA, a double stranded RNA construct, a nucleotide sequence, a recombinant DNA construct, a cell, or a composition described herein, for treating pest infection of plants.

According to still a further embodiment, the invention relates to a kit comprising any of the double stranded RNAs, double stranded RNA constructs, nucleotide sequences, recombinant DNA constructs, cells or compositions described herein, for treating pest infection of plants. The kit may be supplied with suitable instructions for use. The instructions may be printed on suitable packaging in which the other components are supplied or may be provided as a separate entity, which may be in the form of a sheet or leaflet for example. The instructions may be rolled or folded for example when in a stored state and may then be unrolled and unfolded to direct use of the remaining components of the kit.

In one specific embodiment, the method of the invention may also be used as a tool for experimental research, particularly in the field of functional genomics. Targeted down-regulation of pest genes by RNAi can be used in *in vitro* or *in vivo* assays in order to study gene function, in an analogous approach to that which has been described in the art for the nematode worm *C.elegans* and also *Drosophilia melanogaster.* Assays based on targeted down-regulation of specific pest genes, leading to a measurable phenotype may also form the basis of compound screens for novel anti-pest agents.

### DESCRIPTION OF FIGURES

The present Invention will now be described with reference to the following figures in which:
Figure 1 shows examples of concatemer constructs with optimal target gene selection, target sequence selection, and dsRNA fragment combination into the concatemer construct as described herein.
Figure 2 shows the different lock types as described herein,
Figure 3 shows the different dsRNA core types of the present invention, which form part of the concatemer and/or stabilized dsRNA constructs as described herein.
Figure 4 shows a preferred construct according to the present invention.
Figure 5 in dsRNA core type 1 and 2, the so-called "cloverleaf" dsRNA cores, each stem may comprise a combination of the dsRNA core types A, B or C of Figure 3. Multiple stems can be built in, with or without the linker and/or a lock at position Y. The stems may be branched or unbranched. These branched and unbranched stems can be combined within one construct according to the present invention. The linker and/or lock at position Y contain a short loop at its extremity. At position X, the core dsRNA 1 or 2 may contain a stem, a linker and/or a lock. When located at position X, a GC-rich clamp or a mismatch lock also forms a dsRNA stem, optionally coupled to other additional locks. A dsRNA stem at position X is build up by a 5' fragment which finds its complementary sequence at the 3' end of the RNA strand. This core type of dsRNA molecules can form 'closed' star-like or sphere-like 3D structures that provide an extra level of RNA processing protection. In dsRNA core type 3, the lock at position Y is preferably a short loop and the linker at position X is preferably an intron. The dsRNA construct preferably starts and ends with a linker/lock combination at position Z, which is at the edges of the construct.
Figure 6 shows a schematic presentation of the general building blocks used in the stabilized dsRNA constructs described herein. In each of the constructs A, B, C or D, different dsRNA core (e.g. concatemer) combinations are possible, different linker sequence combinations are possible, different lock combinations are possible and the number of different building blocks may vary. Additionally in construct D, different combinations of internal linker and/or lock blocks are possible.
Figure 7 shows a "dumbbell" construct comprising sense and antisense fragments of the C. elegant F39H11.5 target gene and two short loops to protect the construct against RNA processing.
Figure 8 shows examples of hairpins In which linkers according to the present invention are combined with locks that are protein binding RNA structures.
Figure 9 shows the Meloidogyne beta-tubulln sequence (SEQ ID NO 43) with annotation of the primers used to produce three dsRNA fragments of different lengths, namely of 105, 258 or 508 base pairs.
Figure 10 shows the number of moving J2 Meloidogyne incognita larvae (counted 2, 3, 4, 6 and 22 hours after plating on agar) after overnight feeding with double-stranded beta-tubulin RNA of different lengths: 1) No dsRNA; 2) 105 bp dsRNA; 3) 258 bp dsRNA; 4) 508 bp dsRNA.
Figures 11 and 12 show the results of protection against RNAse III dicing by IRES sequences as described in Example 2.1.

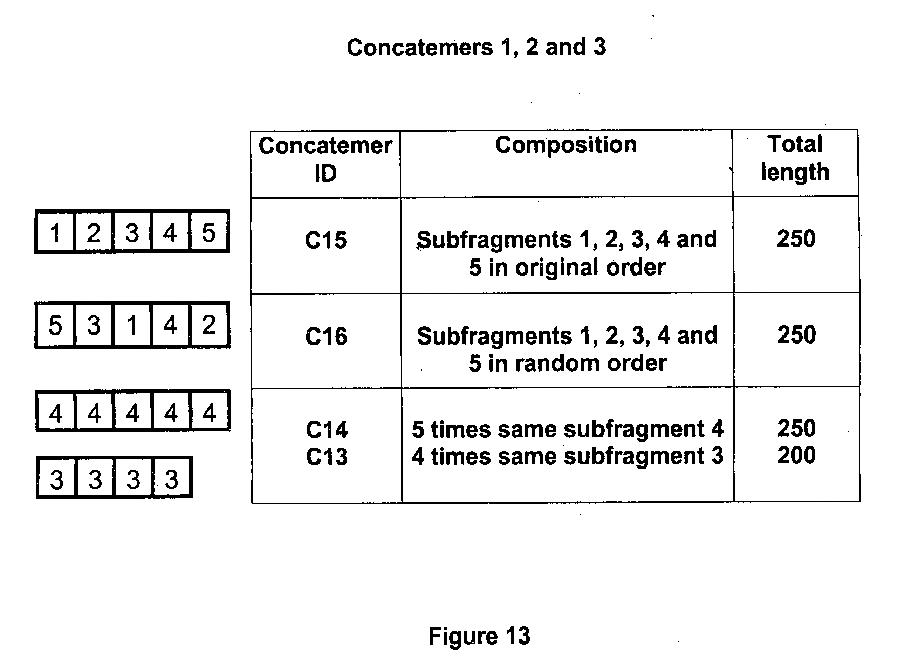

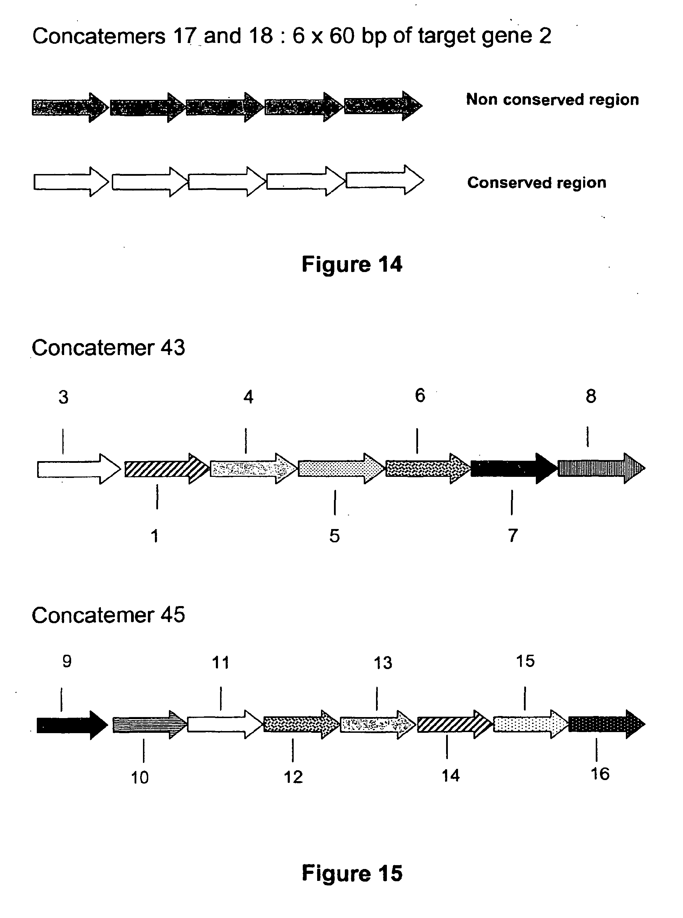

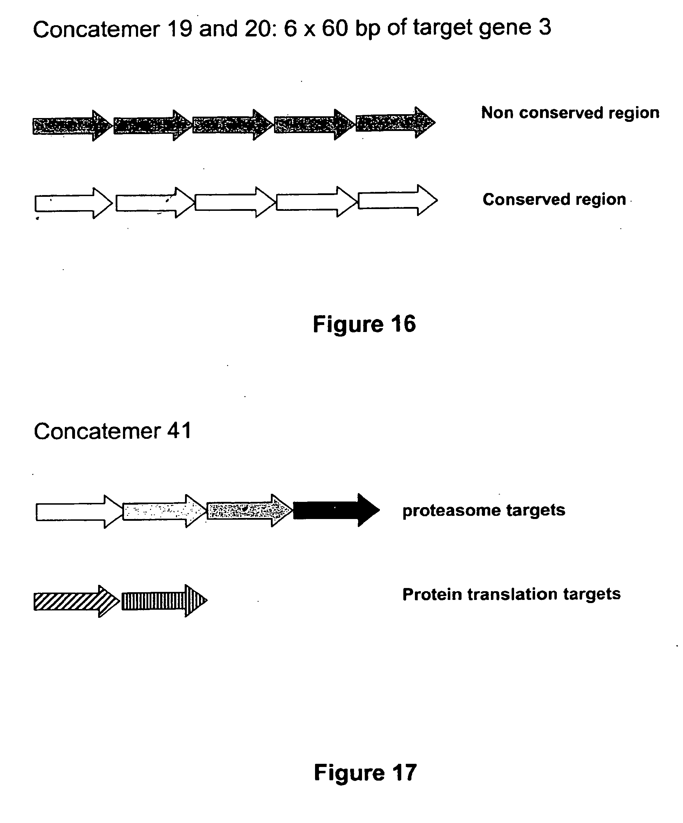

Figures 13 to 20 represent concatemer constructs as described in Example 3 and in Table 3.
Figure 21 shows the construction of concatemers comprising 1 to 6 repeat units of *rps-4* 80bp dsRNA fragments (as described in Example 3.1).
Figure 22 shows RNAi efficacy of the 1 to 6 repeat units of *rps-4* 80bp dsRNA fragments of Figure 21.
Figure 23 shows larvae development stage for the 3 and 6 repeat of Figures 21 and 22.
Figure 24 shows the construction of concatemers comprising 6+0, 5+1, 4+2, 3+3, 2+4 rps-4 and unc-22 80bp dsRNA fragment repeat units (as described in Example 3.2).
Figure 25 shows RNAi efficacy of the repeat units of Figure 24.
Figures 28 and 27 show lethality by inactivating sub-lethal genes *sym-1* and *sym-5* (as described in Example 4).

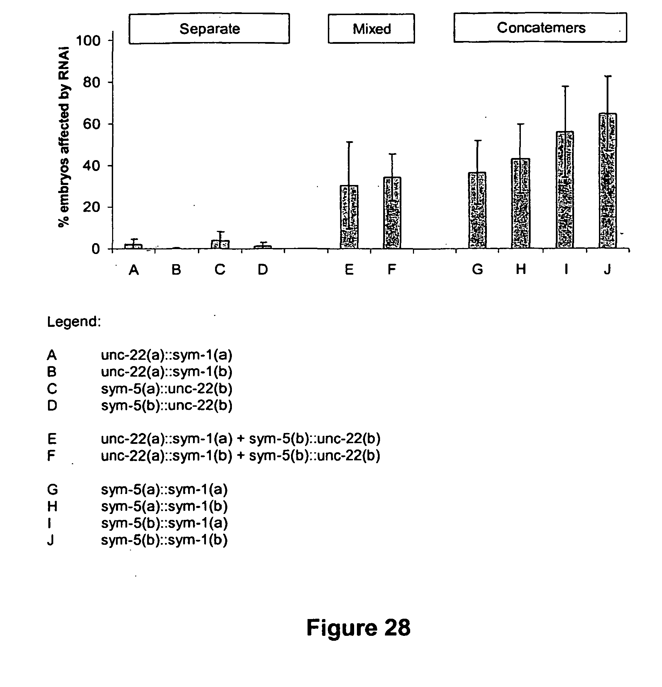

Figure 28 shows the effect of co-inactivating sub-lethal genes *sym-1* and *sym-5* using dsRNA fragments separately, mixed or in single constructs (as described in Example 5).

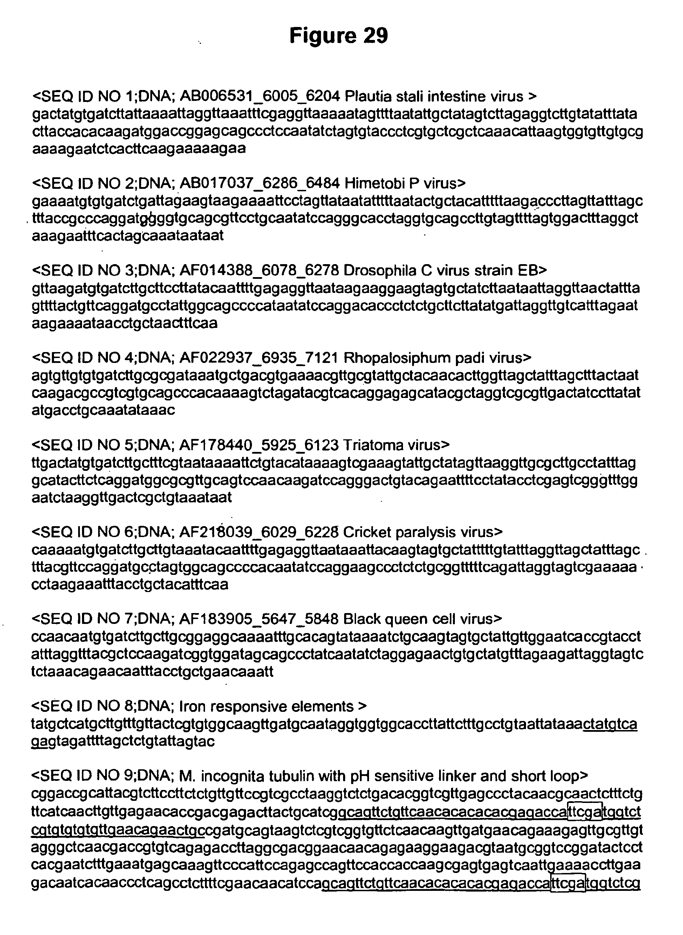

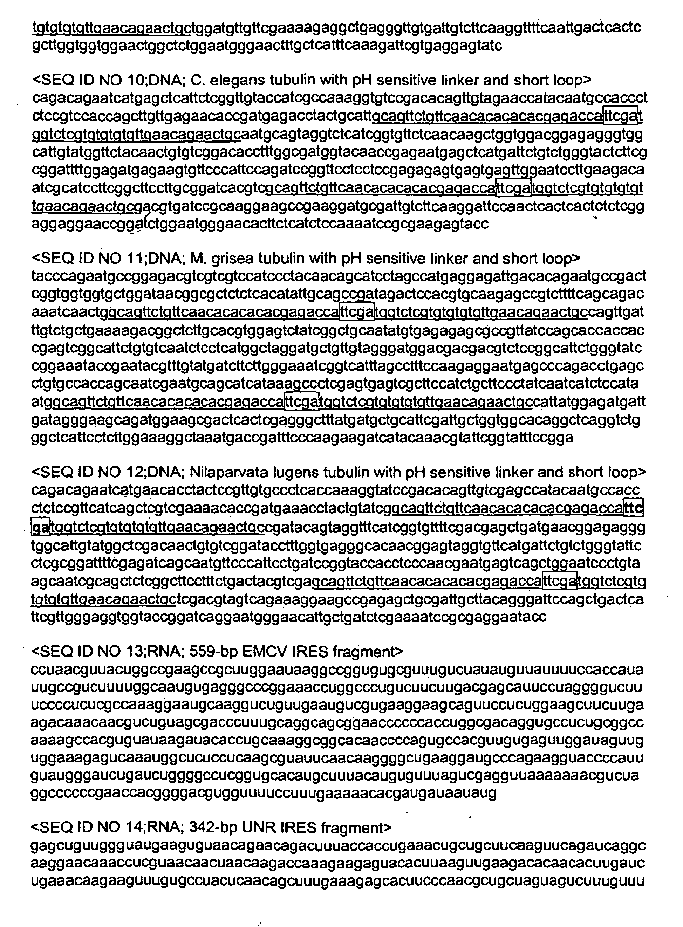

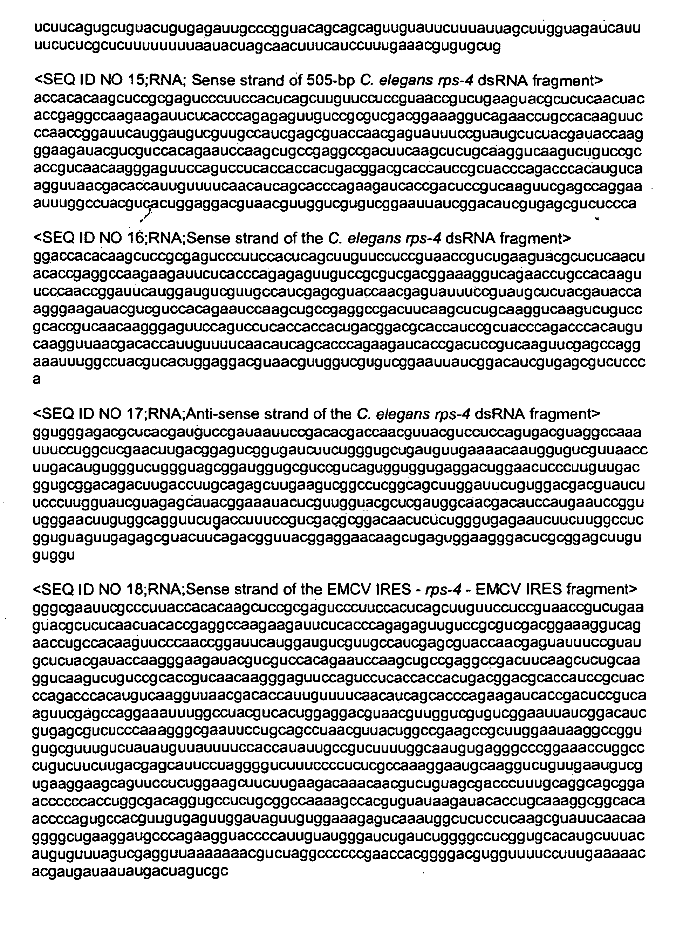

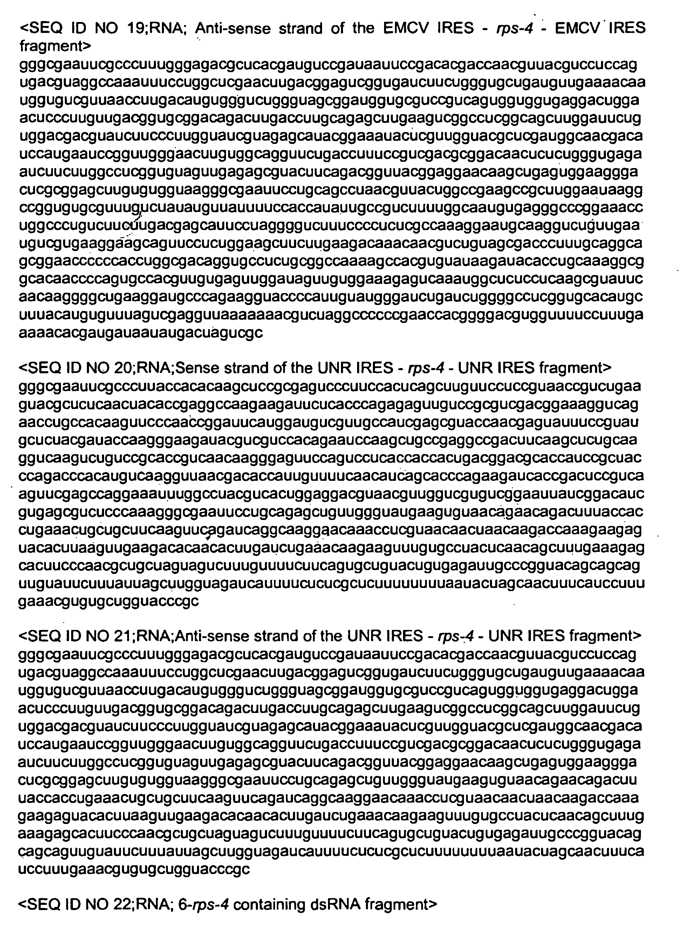

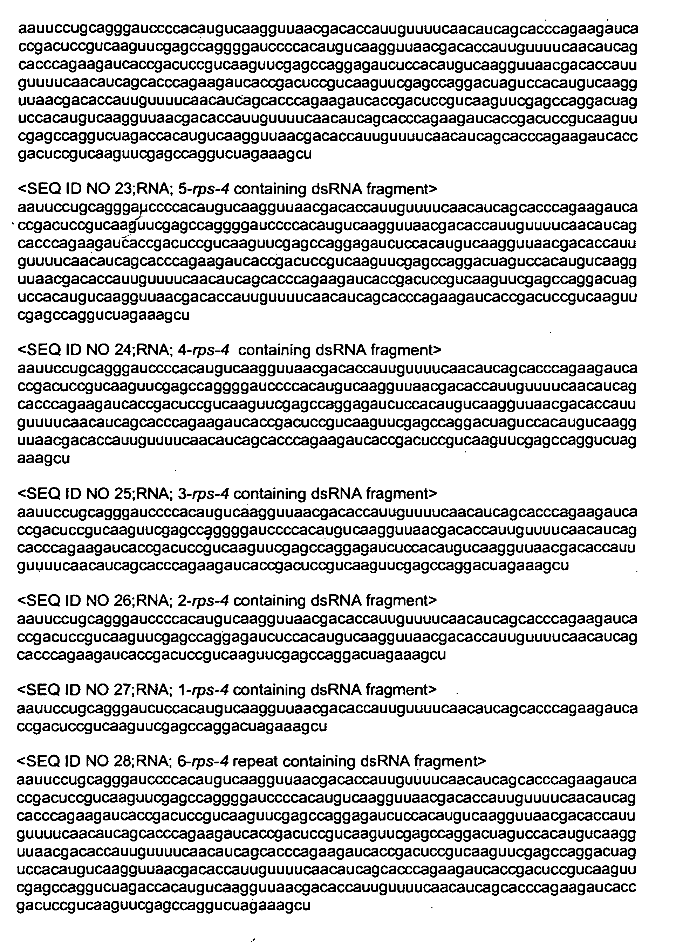

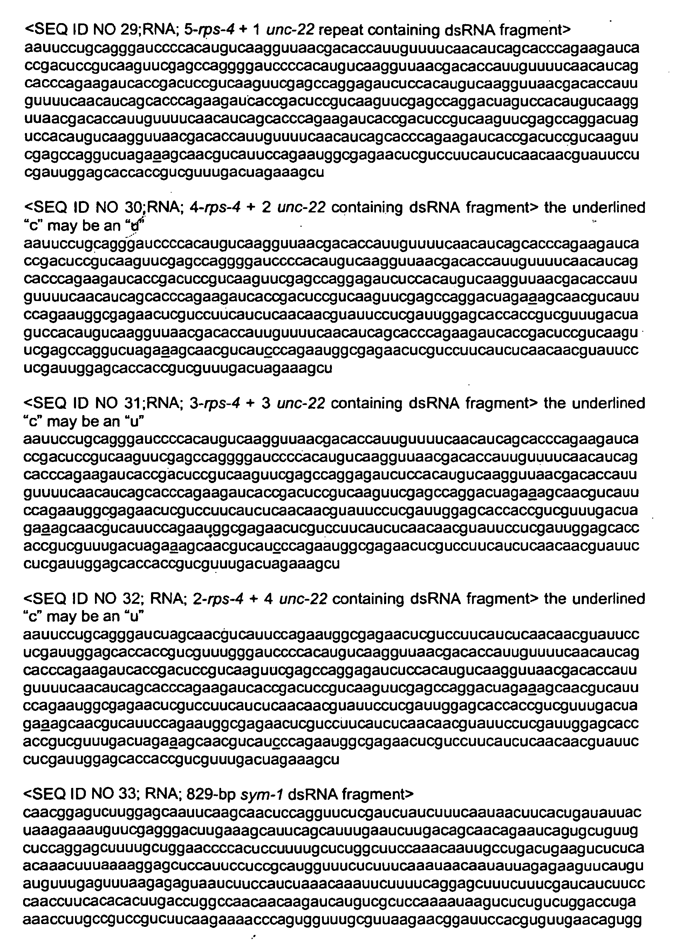

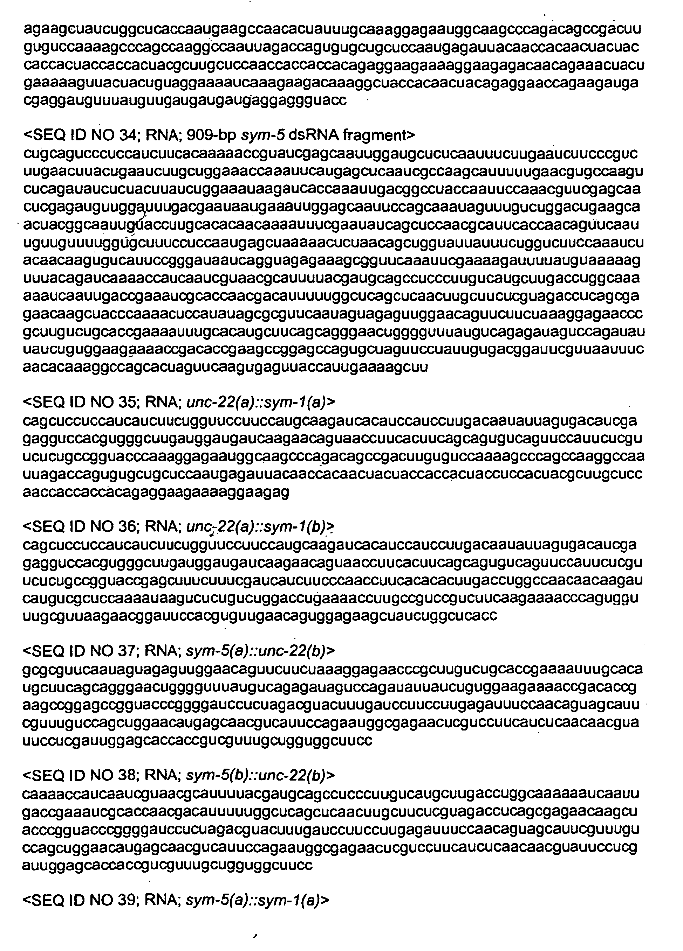

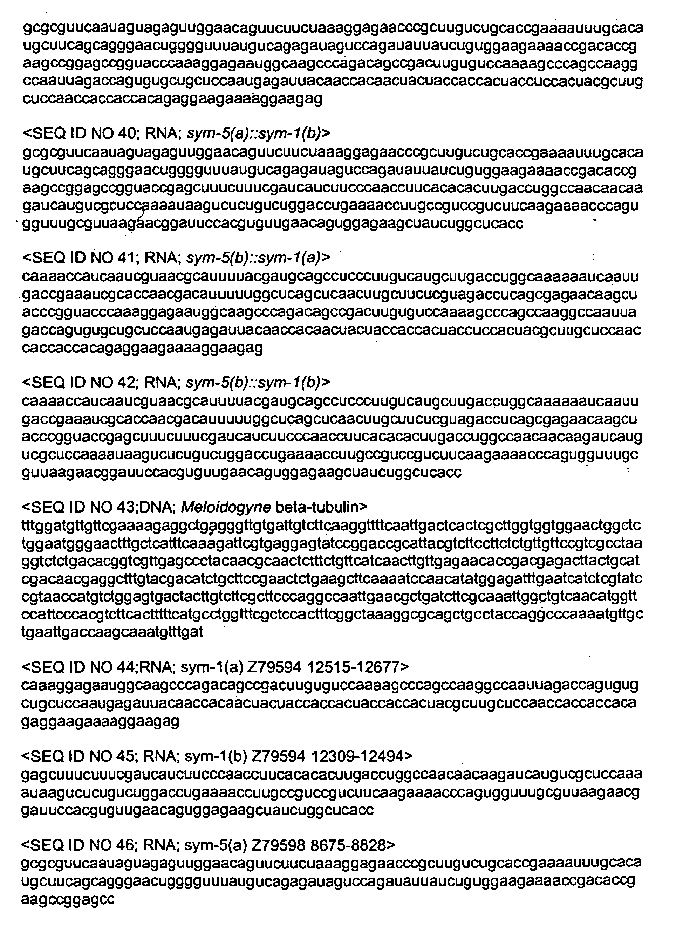

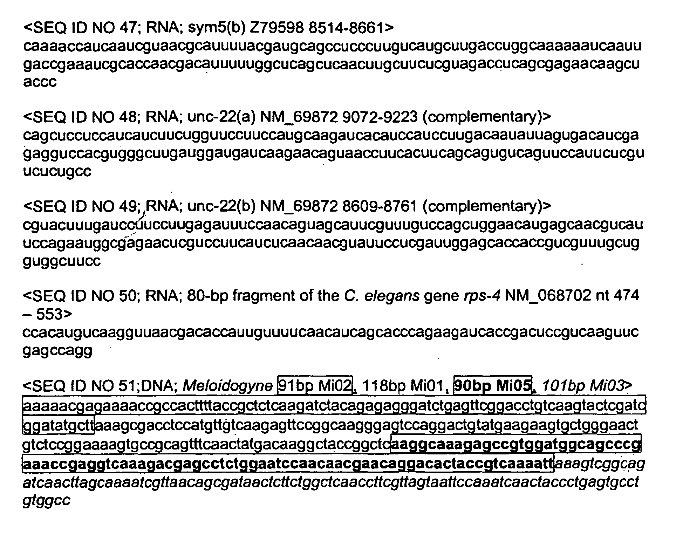

Figure 29 represents a list of exemplary sequences of the Invention.

### EXAMPLES

The invention will be further understood with reference to the following non-limiting examples.

### Example 1: Efficacy of dsRNA in nematodes is length dependent

Short interfering RNAs (siRNAs) mediate cleavage of specific single-stranded target RNAs. These siRNAs are commonly around 21 nt in length, suggesting that siRNA expression in the host causes efficient and specific down-regulation of gene expression, resulting in functional inactivation of the targeted genes. However, there are indications that in invertebrates (e.g. free living nematode *C. elegans* and plant parasitic nematode *Meloidogyne incognita)* the minimum length of dsRNA fed to the invertebrate needs to be at least 80-100 nt to be effective, possibly due to a more efficient uptake of these long dsRNA fragments by the invertebrate.

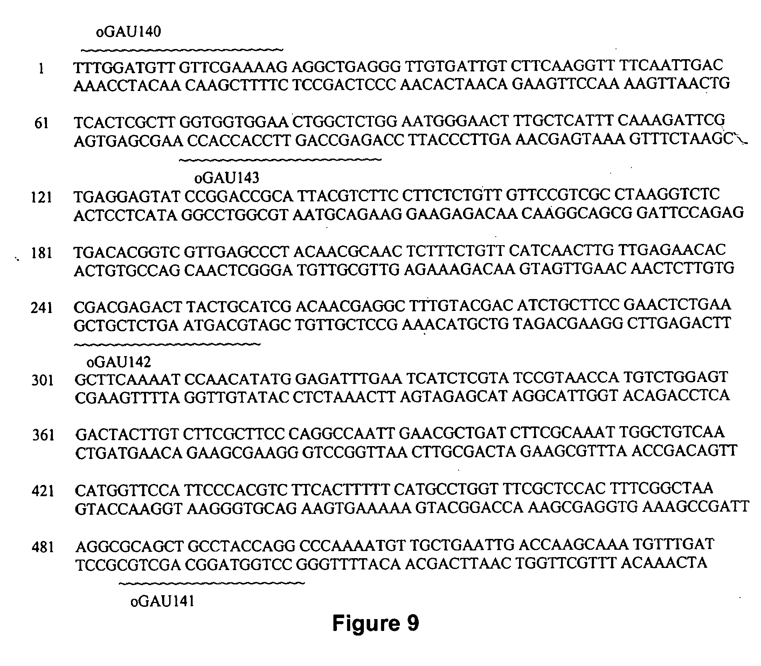

Similar results were now observed for the plant parasitic nematode *Meloidogyne incognita* (SEQ ID NO: 43). dsRNA fragments of the *M*. *incognita* beta-tubulin genes with different lengths (105 bp, 258 bp and 508 bp) were produced *in vitro* (T7 Ribomax Express RNAi System, Promega) using the specific primers as shown in Figure 9 and Table1.

**Table 1: Overview of different M. incognita beta-tubulin fragments and the primers used to isolate them**

| **Primer FW** | **Primer RV** | **Fragment length** |
|---|---|---|
| GAU140 | GAU143 | 105 bp |
| GAU140 | GAU142 | 258 bp |
| GAU140 | GAU141 | 508 bp |

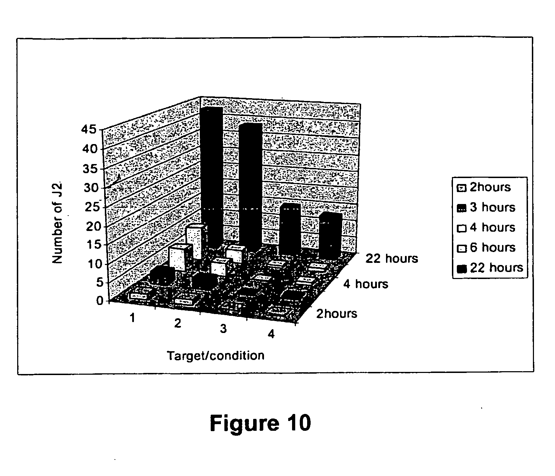

An *in vitro* drinking assay was used to test the efficacy of these beta-tubulin dsRNAs in J2 *Meloidogyne incognita.* J2 is the infective larval second-stage juvenile of the nematode. J2s were stimulated to feed from a liquid medium containing M9 buffer, PEG and 5 mg/ml dsRNA of the different beta-tubulin constructs or free FITC (0.1 mg/ml). J2s were incubated at 26°C. Ingestion of the dsRNA was checked by visualization of FITC uptake via fluorescence microscopy. The downregulation of the endogenous target genes was checked by quantitative PCR or by monitoring phenotypical effects (lethality/motility) of the J2 larvae. The downregulation of the endogenous beta-tubulin gene led to the phenotypical effect of reduced motility of J2 larvae (Figure 10). This reduced motility was observed for J2 larvae that ingested the 258 and 508 bp dsRNA. This effect could not be seen for J2 larvae that ingested the 105 bp dsRNA.

### Example 2: Protection of dsRNA

### 2.1. Protection against RNAse III dicing by IRES sequences

In this example, a dsRNA fragment was flanked on both sites by a lock sequence exhibiting extensive secondary structures. The secondary structures at the termini delayed processing of the dsRNA by two RNase III enzymes, human Dicer and *E.coli* RNase III.

As protecting lock sequences, the internal ribosome entry sites (IRESes) from the encephalomyocarditis virus (EMCV) and Upstream of N-*ras* (UNR) were used. IRESes form complex secondary structures with multiple stem-loop regions, to which proteins can bind such as ribosomes and the polypyrimidine tract binding protein (PTB). In a plant cell the EMCV IRES may protect linked dsRNA from dicing by its secondary structure as well as by binding cellular factors, thereby sterically preventing access of Dicer to the dsRNA.

The IRES sequences used in this example were a 559-nt fragment upstream of the EMCV viral polyprotein coding sequence (Genbank accession number NC_001479, nucleotides 279 - 836) with an extra A nucleotide at position 776 (SEQ ID NO 13), and a 342-nt fragment upstream of the human UNR protein coding sequence (Genbank accession number NM_001007553, nucleotides 69 - 410; SEQ ID NO 14). The dsRNA fragment used in this example is a 505 bp fragment of the *C. elegans rps-4* cDNA (Genbank accession number NM_068702. nucleotides 122 - 626; SEQ ID NO 15).

The IRES sequences were amplified with PCR primers bearing the proper restriction sites at the ends and cloned into a vector containing two T7 promoter sites flanking a multiple cloning site. The *rps-4* fragment was amplified by PCR and cloned into the TOPO-TA® vector (Invitrogen). From here, it was cloned in both orientations in the IRES-containing plasmids using the Eco RI sites from the TOPO-TA® vector. Plasmids were isolated using the QIAprep® Spin Miniprep Kit (Qiagen). To prepare template from the IRES-containing plasmids, plasmids were linearized after the IRES, and PCR was performed with a T7 forward primer and an IRES-specific reverse primer. RNA was prepared by *in vitro* transcription using the T7 RiboMAX^{™} Express RNAi System (Promega). The sequence of the resulting sense and antisense strands of the dsRNA constructs is given in SEQ ID Nos: 16 to 21.Upon annealing, the double stranded *rps-4* RNA is flanked by an IRES sequence at each 3' end. *rps-4* control dsRNA was prepared from a PCR-derived template in which case one of the PCR primers was extended with a T7 promoter site.

To show that the IRES sequences protect the dsRNA from dicing, unlinked and IRES-linked dsRNA were incubated with two commercially available RNase III enzymes according to the manufacturer's protocol.

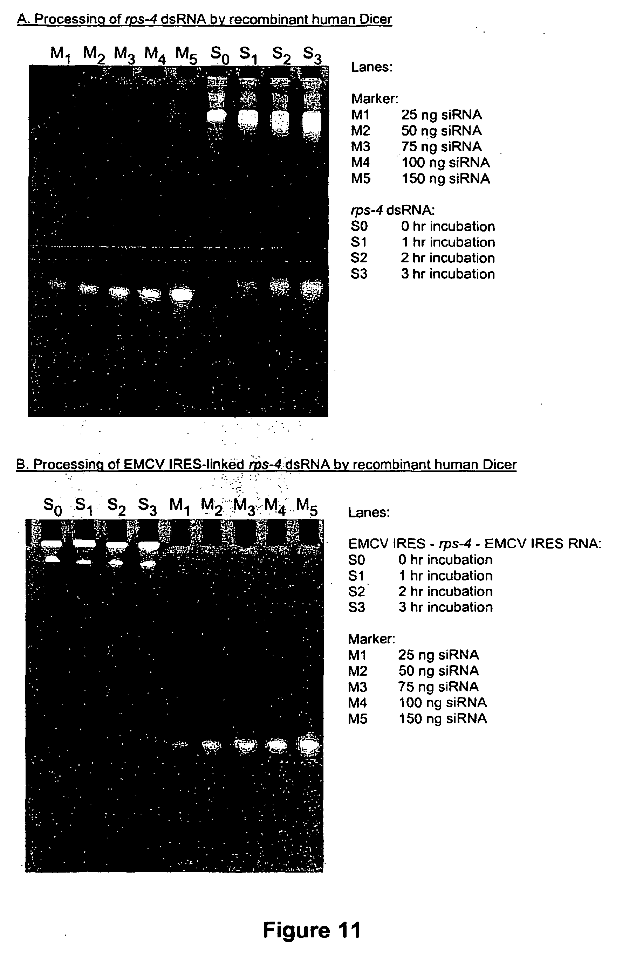

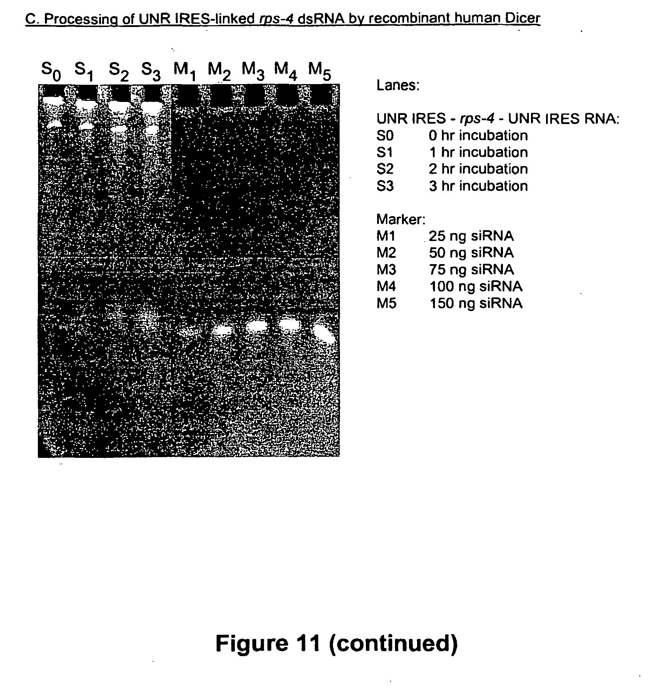

In the first experiment, 400 ng of unlinked or IRES-linked dsRNA was incubated at 37°C with 1 Unit of recombinant human Dicer enzyme (Stratagene). The reaction was stopped after 0, 1, 2 or 3 hours, run on a 20% polyacrylamide gel and stained with ethidium bromide. For comparison, 25, 50, 75, 100 and 150 ng of an unrelated double-stranded 21-mer (siRNA) was loaded on the same gel. The diced product migrated just above the marker siRNA. At each of the 1, 2 and 3 hour incubation time points, less diced product was formed in the reactions with the IRES-linked dsRNA as compared to the unlinked dsRNA (see Figure 11). The bands that migrate high up in the gel represent unprocessed dsRNA or processing intermediates (a lot of the IRES-linked dsRNA did not enter the wells and stuck in the slot; a significant fraction of this will have been washed away while staining the gel). At each time point, more processing intermediates were found with unlinked dsRNA compared to IRES-linked dsRNA, as judged from the smearing of the high molecular weight band that migrated into the gel.

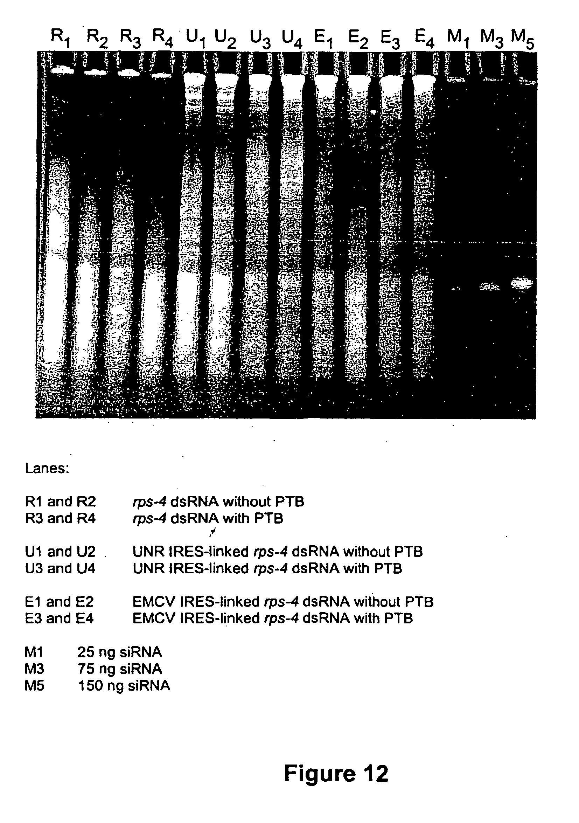

The EMCV IRES and UNR IRES also protected against processing by another RNase III enzyme isolated from *E. coli.* 3 pmol of unlinked or IRES-linked dsRNA was incubated at 37°C with 0.4 Units of recombinant *E*. *coli* ShortCut^{™} RNase III enzyme (New England Biolabs Inc.). Manganese-containing reaction buffer was used to promote processing of dsRNA into a heterogenous mix of 18 - 25 bp siRNAs. The reaction was stopped after 20 min by instantaneous freezing in liquid nitrogen, run on a 20% polyacrylamide gel and stained with ethidium bromide. For comparison, 25, 75, and 150 ng of an unrelated 21-mer siRNA was loaded on the same gel. In parallel, the same set of reactions was performed in the presence of 60 pmol of recombinant human PTB-GST fusion protein that was expressed in bacteria and purified over a GST column (PTB sequence as Genbank sequence NP_114368.1. fusion at sixth amino acid). All conditions were tested in two independent reactions. As was the case with human Dicer, IRES-linked dsRNA was less processed by bacterial RNase III than unlinked dsRNA (see Figure 12, compare lanes R1 and R2 with U1, U2, E1 and E2). In the reactions with unlinked dsRNA, nearly all long dsRNA is processed into end product or low molecular weight processing intermediates. In the reactions with IRES-linked dsRNA, much of the starting material remained unprocessed (bands in the slot and in the top of the gel) and also high molecular weight processing intermediates were present. Moreover, the presence of PTB protects IRES-linked dsRNA even more from RNA processing, as judged from the lower levels of end product and higher levels of processing intermediates. In the case of UNR IRES-linked dsRNA also higher molecular weight partially processed bands indicates increased resistance to RNA processing (see Figure 12, compare lanes U1 and U2 with U3 and U4, and lanes E1 and E2 with E3 and E4).

### 2.2. Construction of dsRNA with linker and lock sequence(s) protecting dsRNA against RNA processing.

Beta-tubulin target genes from different target species are isolated via RT PCR cloning with degenerative primers that are developed based on the sequence of known beta-tubulin genes. For example, suitable fragments of the beta-tubulin gene of *Meloidogyne incognita* to be used in the constructs of the present invention are represented in Figure 9 and by SEQ ID NO: 43. Additonally, "one-cell arrest" (OCS) target genes are isolated, such as the *C.elegans* OCS target gene F39H11.5. The sequence of F39H11.5 is found in genbank (accession number Z81079, version 1, gi number 1627924, region 841-1770 on the complementary strand). Also the *C.elegans* gene sup-35 (mRNA genbank accession number NM_067031) is used as a target gene and the fragment for the dsRNA silencing constructs ranges from nucleotide 396 to nucleotide 999.

The length of the tested dsRNA constructs is about 300 base pairs. Single stem core dsRNA constructs, targeting one single target sequence are tested as well as concatemers, targeting multiple target sequences. In case of concatemers, the length of each dsRNA fragments is about 80 base pairs or about 25 base pairs. In another particular construct, the total length of the concatemer dsRNA is about 80 bp and each dsRNA fragment is about 20 or about 25 bp. In yet another construct the total length of the concatemer dsRNA is about 250 bp or about 300 bp and each dsRNA fragment is about 20 or about 25 bp, or about 50 or about 60 or about 70 bp. Locks are present on both edges of the dsRNA stem. The locks are 5 base pairs non-complementary loops.

**Table 2. Overview of the stabilized constructs. Different constructs of the present invention are tested in four different species, amongst which plant pest species: Meloidogyne incognita, Caenorhabditis elegans, Hopper for example Nilaparvata lugens and Magnaporthe grisea.**

| | ***M. incognita*** | ***C. elegans*** | **Hopper** | ***M. grisea*** |
|---|---|---|---|---|
| **Target gene** | beta-tubulin | beta-tubulin, sup35 and/or OCS | beta-tubulin | beta-tubulin |
| **DsRNA core** | cA, cB | cA, cB | cA, cB | cA, cB |
| **Lock** | 5bp short loop | 5bp short loop | 5bp short loop | 5bp short loop |

| **Linker type** | pH sensitive | pH sensitive | pH sensitive | pH sensitive |
|---|---|---|---|---|
| **In vitro uptake** | Drinking assay | Drinking assay | Spray on leaf | Soaking |
| **In planta** | Hairy roots, whole plant | X | Whole plant, callus | Hairy roots, callus, whole plant |

The specific constructs used in these examples are also represented herein in SEQ ID Nos: 9 to 12.

### Example 3: Design and cloning of dsRNA concatemer constructs efficient for pest control

Concatemer constructs were designed to comprise different combinations of dsRNA fragments which target different target genes; or which target a different target sequence from such target genes, which target sequences have the same or different lengths; or which repeat the same sequence multiple times.

The dsRNA concatemer constructs of the invention have a total length of less than 700 bp, and preferably range from about 250 bp to about 500 bp. Preferably the length of the dsRNA concatemer construct is as such that the corresponding ssRNA is capable of forming efficiently a hairpin dsRNA.

The format of the concatemer construct of the invention may be a dsRNA per se or may be a hairpin dsRNA. A dsRNA per se or a hairpin may be made by in vitro transcription or by recombinant expression systems.

**Table 3: The following concatemer constructs were cloned. Schematic presentations are given in the Figures. "Freefrag" as used herein means a dsRNA fragment with no substantial nucleotide sequence homology to non-target organisms.**

| **Name** | **Target gene**** | **description** | **Figure and/or SEQ ID NO** |
|---|---|---|---|
| C1 | A | 1 x 80 bp, selected on GC content* | Figure 21, 22, SEQ ID No: 27 |
| C2 | A | 2 x 80 bp, selected on GC content* | Figure 21,22, SEQ ID No: 26 |
| C3 | A | 3 x 80 bp, selected on GC content* | Figure 21, 22, 23, SEQ ID No: 25 |
| C4 | A | 4 x 80 bp, selected on GC content* | Figure 21, 22, SEQ ID No: 24 |
| C5 | A | 5 x 80 bp, selected on GC content* | Figure 21,22, SEQ ID No: 23 |
| C6 | A | 6 x 80 bp, selected on GC content* | Figure 21, 22, 23, SEQ ID Nos: 22 and 28 |
| C7 | B | 1 x 80 bp, selected on GC content* | |
| C8 | B | 2 x 80 bp, selected on GC content* | |
| C9 | B | 3 x 80 bp, selected on GC content* | |
| C10 | B | 4 x 80 bp, selected on GC content* | |
| C11 | B | 5 x 80 bp, selected on GC content* | |
| C12 | B | 6 x 80 bp, selected on GC content* | |
| C13 | 1 | 4 x 40 bp of conserved region | Figure 13 |
| C14 | 1 | 5 x 50bp of conserved region | Figure 13 |
| C15 | 1 | Freefrag in biological order | Figure 13 |
| C16 | 1 | Freefrag scrambled | Figure 13 |
| C17 | 2 | 6 x 60bp of conserved region | Figure 14 |
| C18 | 2 | 6 x 60bp of non-conserved region | Figure 14 |
| C19 | 3 | 6 x 60bp of conserved region | Figure 16 |
| C20 | 3 | 6 x 60bp of non-conserved region | Figure 16 |
| C21 | A-C | 1 x 80 bp of A, 5 x 80 bp of C, selected on GC content* | Figure 24, 25, SEQ ID NO: 29 |
| C22 | A-C | 2 x 80 bp of A, 4 x 80 bp of C, selected on GC content* | Figure 24, 25, SEQ ID No: 30 |
| C23 | A-C | 3 x 80 bp of A, 3 x 80 bp of C, selected on GC content* | Figure 24, 25, SEQ ID No: 31 |
| C24 | A-C | 4 x 80 bp of A, 2 x 80 bp of C, selected on GC content* | Figure 24, 25, SEQ ID No: 32 |
| C25 | A-C | 5x80 bp of A,1 x 80 bp of C, selected on GC content* | - |
| C26 | B-C | 1 x 80 bp of B, 5 x 80 bp of C, selected on GC content* | |
| C27 | B-C | 2 x 80 bp of B, 4 x 80 bp of C, selected on GC content* | |
| C28 | B-C | 3 x 80 bp of B, 3 x 80 bp of C, selected on GC content* | |
| C29 | B-C | 4 x 80 bp of B, 2 x 80 bp of C, selected on GC content* | |
| C30 | B-C | 5 x 80 bp of B, 1 x 80 bp of C, selected on GC content* | |
| C31 | D | 909 bp of D, selected on GC content* | Figure 26, 27 |
| C32 | E | 829 bp of E, selected on GC content* | Figure 26, 27 |
| C33 | D-C | 50 bp of D, 50 bp of C, selected on GC content* | |
| C34 | D-C | About 150 bp of D, 152 bp of C, selected on GC content* | Figure 28, SEQ ID Nos: 35 and 36 |
| C35 | D-E | 50 bp of D, 50 bp of E, selected on GC content* | |
| C36 | D-E | About 150 bp of D, about 150 bp of E, selected on GC content* | Figure 28, SEQ ID Nos: 39 to 42 |
| C37 | D-E | 2 x 50 bp of D, 2 x 50 bp of E, selected on GC content* | |
| C38 | D-E | 3 x 50 bp of D, 3 x 50 bp of E, selected on GC content* | |
| C39 | E-C | 50 bp of E, 50 bp of C, selected on GC content* | |
| C40 | E-C | About 150 bp of E, 153 bp of C, selected on GC content* | Figure 28, SEQ ID Nos: 37 and 38 |
| C41 | 17-18 | Target genes in same pathway: protein translation pathway, fragments selected on GC content* + freefrag | Figure 17 |
| C41 | 19-20-21-22 | Target genes in same pathway, for instance, the proteasome pathway, fragments are selected on GC content* + freefrag | Figure 17 |
| C42 | 17-22-23-24-25 | Combination of target genes from different pathways, for instance protein translation, proteasome, transcription, nucleic acid binding and protein binding pathways, fragments are selected on GC content* + freefrag | Figure 18 |
| C43 | 3-1-4-5-6-7-8 | Essential genes: 70bp each, selected on CG content* | Figure 15 |
| C44 | 3-1-4-5-6-7-8 | Essential genes: selection on GC content* + freefrag | Figure 19 |
| C45 | 9-10-11-12-13-14-15-16 | Insect specific genes: 70bp each selected on GC content* | Figure 15 |
| C46 | 9-10-11-12-13-14-15-16 | Pest specific genes: selection on GC content* + freefrag | Figure 19 |

| | | | |
|---|---|---|---|
| * Fragments are selected on GC content between 40% and 60% ** Genes 1 to 25: target genes; Gen A = *C. elegans* rps-4; Gen B = *C. elegans* rps-14; Gen C = *C. elegans* unc-22; Gen D = *C. elegans* sym-1; Gen E = *C. elegans* sym-5 | | | |

### 3.1. Efficacy of dsRNA in nematodes improves with increasing the number of repeat units of a small fragment

This example describes that an 80-bp dsRNA fragment is sufficient to induce RNAi, and that the efficacy increases when this fragment is repeated multiple times in the same construct.

### a) dsRNA fragments

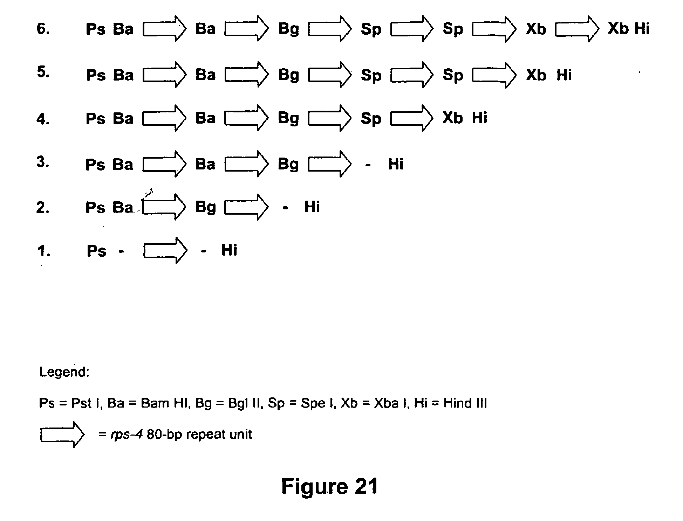

The dsRNA fragments used in this example contain one to six repeat units of an 80-bp fragment (SEQ ID NO: 50) of the *C. elegans* gene *rps-4* (Genbank accession number NM_068702, nucleotides 474 - 553). A schematic representation of these constructs is given in Figure 21, the sequences of the dsRNA fragments (sense strands) used are represented by SEQ ID Nos: 22 to 27.

### b) Methods

Cloning: A DNA fragment was made synthetically containing 6 *rps-4* repeat units separated by restriction sites (see Figure 21). This fragment was first cloned in a vector such that it was flanked by two T7 promoter sites. Plasmids containing 5, 4, 3, 2 or 1 repeat units respectively were derived from this plasmid by digestion with the proper restriction enzyme(s) and religation of the linearized plasmids.

RNA preparation: Plasmids were isolated using the EndoFree® Plasmid Maxi Kit (Qiagen) and in two separate reactions digested with Eco RI and Hind III respectively. RNA was prepared by *in vitro* transcription using the T7 RiboMAX^{™} Express RNAi System (Promega). The sequences of the resulting dsRNA fragments (sense strands) used are represented by SEQ ID Nos: 22 to 27.

*C. elegans* RNAi: *C. elegans* L1 larvae were allowed to ingest dsRNA-containing M9 buffer for 24 hours at 20°C and then transferred to regular NGM plates. The animals were examined after 3 days of growth at 20°C and for all animals the developmental stage was determined.

### c) Results

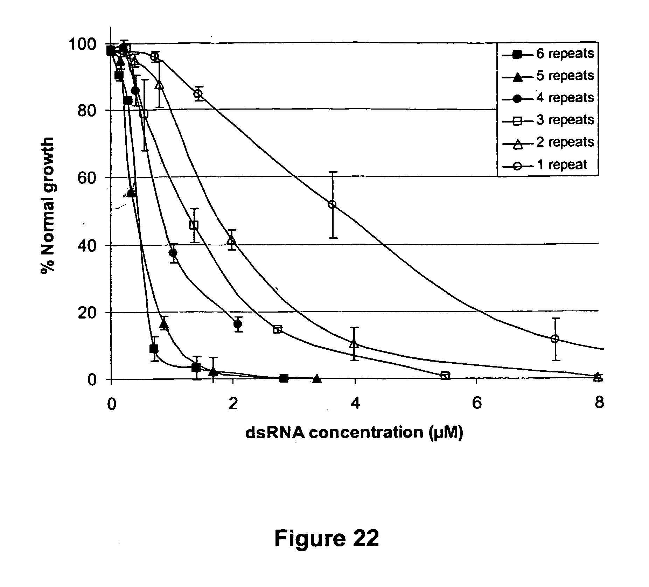

Exposure to *rps-4* dsRNA induced growth delay and arrested development at early larval stages for all constructs. The RNAi efficacy increased with increasing numbers of *rps-4* repeat units present in the dsRNA fragment (see Figure 22). Efficacy was measured as the ability of the dsRNA to prevent animals from becoming adults in 3 days. Moreover, the more *rps-4* repeat units were present in the dsRNA fragment, the lower the concentration needed to induce the same degree of growth inhibition.

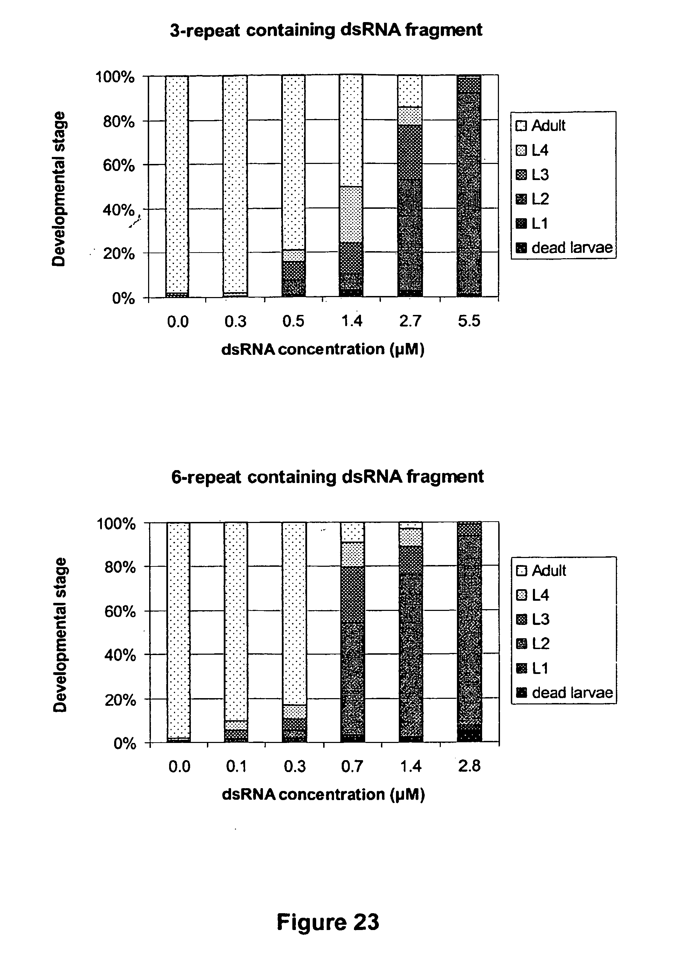

An increased efficacy was not only manifested in a higher number of animals that show growth delay or arrest development, but also in a quicker response (i.e., the larvae arrested at earlier developmental stages). Figure 23 shows that at the highest concentrations nearly no larvae had grown beyond the second larval stage (L2). At intermediate concentrations, some larvae had managed to grow until the third (L3) or fourth (L4) larval stage. The transition from "all adult" to "all L2" occurred faster in the construct with 6 *rps-4* repeat units relative to the construct with 3 *rps-4* repeat units.

### 3.2. Efficacy of dsRNA in nematodes improves with increasing the number of repeat units of a small fragment

This example is a variation of Example 3.1. In this example, however, the total fragment length is kept constant by replacing *rps-4* repeat units with *unc-22* repeat units.

### a) dsRNA fragments

The dsRNA fragments used in this example contain a varying number of the same 80-bp fragment (SEQ ID NO 50) of the *C. elegans* gene *rps-4* described in Example 3.1 together with a varying number of an 80-bp fragment of the *C. elegans* gene *unc-22* (Genbank accession number NM_69872, nucleotides 8621 - 8700). The total number of repeat units in a dsRNA fragment always totals up to six, and therefore all molecules are of the same length. Inactivation of *unc-22* does not influence growth, so all effect on growth inhibition is due to rps-4-specific siRNAs. Due to an extra base in one of the cloning primers the Xba-Spe unc-22 insert in the multiple repeats contains 81 bp. The extra bp is at position 1

### b) Methods

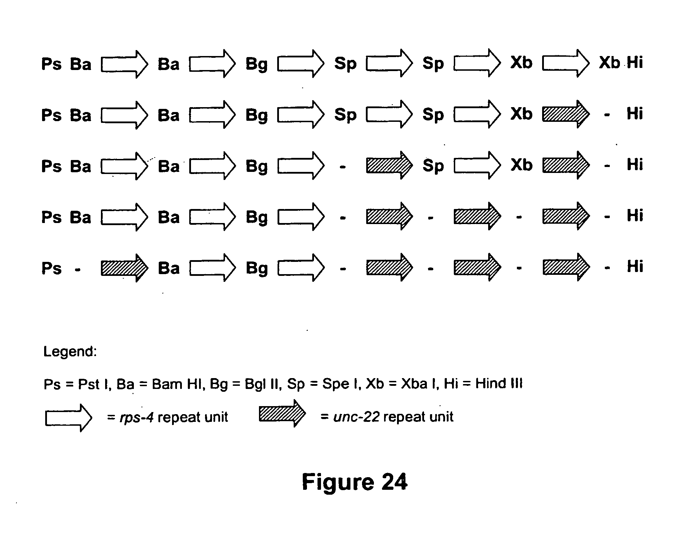

A DNA fragment was made synthetically containing 6 *rps-4* repeat units separated by restriction sites (see Figure 21). This fragment was first cloned in a vector such that it was flanked by two T7 promoter sites. Subsequently one *rps-4* repeat unit at the time was swapped with an *unc-22* repeat fragment that was amplified by PCR using primers with restriction sites flanking the *unc*-22 sequence (see Figure 24).

dsRNA preparation and RNAi experiments were performed as described for Example 3.1. The sequence of the resulting dsRNA fragments (sense strands) is represented by SEQ ID Nos: 28 to 32.

### c) Results

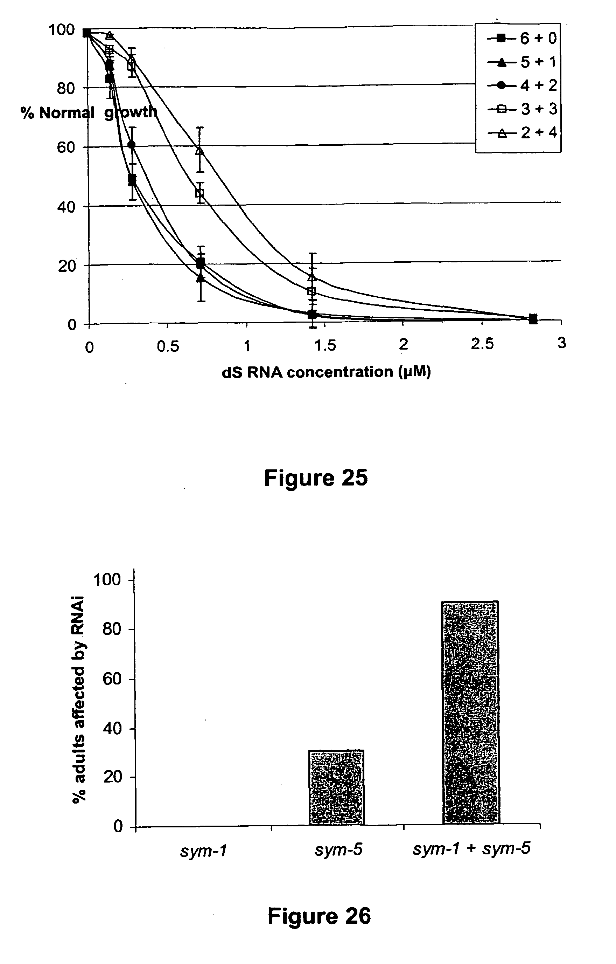

The RNAi efficacy increased with increasing numbers of *rps-4* repeats present in the dsRNA fragment (see Figure 25). dsRNA fragments with 4 or more *rps-4* repeat units were equally active, but were more active than fragments with 2 or 3 repeat units. Since the dsRNA uptake can be considered equal between these constructs, a likely explanation for the increased efficacy of the fragments with 4 or more *rps-4* repeat units is that dicing of these fragments results in more *rps-4*-specific siRNAs.

### Example 4 : Inducing lethality by inactivating multiple sub-lethal targets

This example describes that RNAi co-inactivation of two genes with weak phenotypes on their own, *sym-1* and *sym-5,* results in a greatly enhanced phenotype.

### a) dsRNA fragments

For *sym-1,* a 829-bp fragment was used corresponding to nucleotides 11972 - 2800 of Genbank sequence Z79594. For *sym-5,* a 909-bp fragment was used corresponding to nucleotides 8003 - 8911 of Genbank sequence Z79598. The sequences of the dsRNA fragments (sense strand) used in this example are represented by SEQ ID Nos: 33 and 34.

### b) Method

Feeding: The before-mentioned fragments were amplified with standard PCR primers and cloned in the pGN49A vector (WO01/88121) between two identical T7-promoters and terminators, driving its expression in the sense and antisense direction upon expression of the T7 polymerase, which was induced by IPTG. The resulting plasmids were transformed into the bacterial strain AB301-105 (DE3). Wild-type *C. elegans* L1 larvae were placed on NGM plates with IPTG seeded with transformed AB301-105 (DE3) bacteria, and examined after 3 days of growth at 20°C.

Injection: The before-mentioned fragments were amplified from wild-type genomic DNA using primer combinations in which one primer was extended with the T7 DNA polymerase promoter sequence. PCR products were purified from gel using the QIAquick® Gel Extraction Kit (Qiagen). RNA was prepared by *in vitro* transcription using the T7 RiboMAX^{™} Express RNAi System (Promega). Each dsRNA fragment was injected at 0.7 µg/µl in both gonads of 12 gravid adults. Eggs laid in the period of 2 to 17 hours after injection were separated and their development was examined after 2 days incubation at 20°C.

### c) Results

The effect of *sym-1* and *sym-5* inactivation by RNAi was determined by feeding bacteria expressing dsRNA to wild-type first stage (L1) larvae. L1 larvae growing on *sym-1* dsRNA producing bacteria all became healthy adults within 3 days. L1 larvae growing on *sym-5* dsRNA producing bacteria all became adults, but about 30% of them had a generally sick appearance. However, nearly all L1 larvae growing on a mix of *sym-1* and *sym-5* dsRNA producing bacteria had a generally sick appearance when adult (see Figure 26).

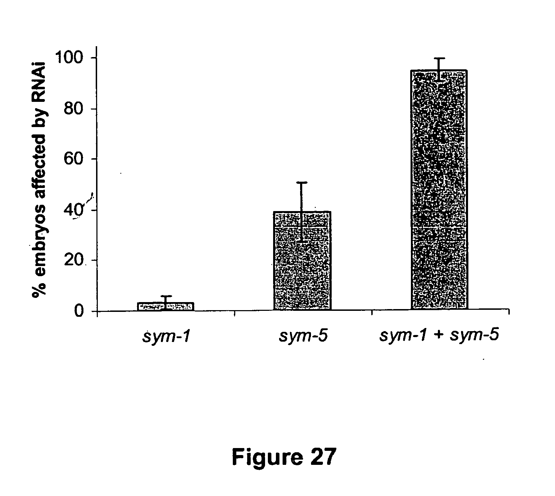

To determine the effect of *sym-1* and *sym-5* on embryonic development, dsRNA was produced *in vitro* and injected into the gonad of healthy, wild-type adults. When *sym-1* dsRNA was injected alone, about 3% of the developing embryos died. When *sym-5* dsRNA was injected alone, about 40% of the developing embryos died. However, when *sym-1* dsRNA and *sym-5* dsRNA were mixed and injected together, nearly all embryos died (see Figure 27).

These results show that co-inactivating multiple genes with a mild phenotype on their own can be beneficial to obtain a much stronger effect.

### Example 5 : Inducing lethality by concatemers of sub-lethal targets

This example describes RNAi co-inactivation of 2 genes by using a single construct containing fragments of each of the genes ("concatemer constructs").

### a) dsRNA fragments

The *sym-1* and *sym-5* fragments used in this example range in size from 146 to 186 bp, and are subfragments of the ones used in Example 4. These smaller fragments are used either separately, or mixed, or in concatemers on the same RNA molecule. Since the concatemers are about twice as long as the single fragments, the single fragments are size-compensated by concatemerization with a 152 or 153-bp fragment of the unrelated gene *unc-22.*

The following sequences were used in this example:

| Gene fragment | Genbank accession nr | Nucleotides | Sequences (see Figure 29) |
|---|---|---|---|
| *sym-1(a)** | Z79594 | 12515 - 12677 | SEQ ID NO 44 |
| *sym-1(b)* | Z79594 | 12309 - 12494 | SEQ ID NO 45 |
| *sym-5(a)* | Z79598 | 8675 - 8828 | SEQ ID NO 46 |
| *sym5(b)* | Z79598 | 8514 - 8661 | SEQ ID NO 47 |
| *unc-22(a)* | NM_69872 | 9072 - 9223 (complementary) | SEQ ID NO 48 |
| *unc-22(b)* | NM_69872 | 8609 - 8761 (complementary) | SEQ ID NO 49 |

| | | | |
|---|---|---|---|
| (* in the sym-1 (a) fragment, an "A" may be present instead of "T" at position 12630) | | | |

### b) Methods

The fragments were PCR amplified using primers with restriction site extensions and sequentially cloned in the Multiple Cloning Site of a plasmid cloning vector dsRNA was prepared and injected as described in Example 3.1 using primers with T7 promoter extensions.

### c) Results

Two fragments of *sym-1* (Figure 28A and B) and two fragments of *sym-5* (Figure 28C and D) ranging from 146 to 186 bp did not induce substantial embryonic lethality when injected separately. Injecting a mixture of the *sym-5(b)* fragments with either of the *sym-1* fragments induced substantial embryonic lethality, showing that the used fragments are active and confirming that co-inactivating multiple genes with a mild phenotype can induce a much stronger effect (Figure 28 E and F).

Concatemer constructs were made between the two *sym-1* and the two *sym-5* fragments (Figure 28 G, H, I and J) and tested the same way. All 4 possible combinations induced embryonic lethality as concatemer, and the penetrance was even stronger as when the two dsRNA molecules were mixed (Figure 28 E and F).

These results show that concatemer dsRNA molecules are effective in co-inactivating multiple genes.

### Example 6: In vitro tests for efficient uptake of the dsRNA by plant parasitic nematode and subsequent gene silencing

The dsRNA constructs according to the present invention (for instance the construct having a sequence represented by SEQ ID NO: 51), were cloned behind the T7 promoter both in sense and antisense direction and were transcribed in vitro using the T7 Ribomax Express RNAi protocol (Promega). dsRNA was produced by mixing sense and antisense RNA. These dsRNA were used in the *in vitro* tests described here below.

With these in vitro assays the performance of the constructs according to the present invention was evaluated for efficient uptake, stability in the pest organism and efficiency in silencing the target gene.

An in vitro drinking assay for *C. elegans* was used following the "soaking" protocol as described in Tabara et al. (Science, 1998, 282 (5388):430-431).

An in vitro drinking assay for *Meloidogyne incognita* was performed as described in Example 1 and is based on forced feeding.

An in vitro assay for dsRNA uptake by fungi was performed as follows. The rice blast fungus *Magnaporthe grisea* was "soaked" in medium containing double-stranded RNA (dsRNA) targeting the fungal target gene. More particularly, conidia (asexual spores) were generated by exposing fungal mycelia to light for 7-10 days. Conidia were harvested and re-suspended in water at a density of 20000 conidia/ml, and inoculated in hydrophilic 96-well plates (50 µl) or on the hydrophilic surface of an artificial membrane (GelBond film, Cambrex) (20 µl). DsRNA transcribed in vitro as described above was added to the spores to final concentrations ranging from 0.01-10 microgram/ml in sterile water After 16-30 h incubation at 28°C the growth of mycelia in the wells was quantitated by optical density reading of the 96-well plates. Growth and phenotype of mycelia on the artificial membrane were also observed with a microscope. Germination of conidia on a hydrophilic surface mimics their germination within the leaf during Invasive growth of the fungus.

Feeding assays for insect, for example for the hopper *Nilaparvata lugens* and the colorado potato beetle *(Leptinotarsa decemlineata),* were based on artificial diet technique. This technique is previously described by Couty A, Down RE, Gatehouse AM, Kaiser L. Pham-Delegue M and Poppy GM in J Insect Physiol. 2001 Dec; 47(12):1357-1366 Effects of artificial diet containing GNA and GNA-expressing potatoes on the development of the aphid parasitoid *Aphidius ervi Haliday* (Hymenoptera: Aphidildae)".

### Example 7: In planta test for stability of the dsRNA and for efficient pest control

The constructs of the present Invention, i.e. comprising SEQ ID NO: 51, were cloned behind the CaMV35S promoter, a root specific promoter or a feeding site specific promoter (like tobRB7), present in a binary vector suitable for plant transformation. The binary vectors were transferred to *Agrobacterium rhizogenes* by three-parental mating (e.g. by *E. coli* HB101 containing pRK2013 helper plasmid). The binary vectors were transferred from *Esherichia coli* Into *Agrobacterium tumefaciens.* Subsequently, crop plants (such as tomato, soybean, cotton, arabidopsis, rice, corn, potato or tobacco) were transformed with the constructs via Agrobacterium-mediated transformation techniques well described in the art, for example as described in "Transgenic plants. Methods and Protocols. Methods in Molecular Biology, Volume 286, by Pena. Leandro"). As a negative control, Agrobacterium without binary vector was also used to transform the plants.

### Stability of the dsRNA constructs of the present invention in plant cells

The stability of the expressed dsRNA constructs according to the present invention was analyzed by quantitative real-time PCR based on Taqman probes or intercalating dyes (SYBR green), as previously described.

The expressed dsRNA constructs were quantified relative towards a standard dilution series of the template. The results were normalized by using the quantitative PCR data of a set of housekeeping genes from the same samples (Vandesompele et al., Genome Biology 2002, 3:research0034.1-0034.11). The quantity of the dsRNA constructs according to the present invention was compared to the quantity of control dsRNA not comprising a lock:

Alternatively, the stability and form of the dsRNA may be analyzed by Northern blot.

### Hairy root transformation of tomato or cotton or potato

The constructs of the present invention were introduced into tomato (e.g. *Lycopersicum esculentum cv. Marmande*), or into tobacco or Into cotton (*Gossypium hirsutum*) cotyledons via transformation with *A. rhizogenes.* The transformed hairy roots were Subsequently tested for nematode resistance. The necessary number of independent transformed lines (e.g. 15) and replicates per line (e.g. 10) were inoculated with *Meloidogyne incognita* J2 larvae. The phenotypic effects on root galling and egg mass formation were measured and scored. Egg masses were put to hatch and the fecundity of the parasite were investigated. The offspring was used to test infectivity/viability of the second generation.

An analogous assay was performed whereby the hairy roots were transformed with the dsRNA construct against a fungal target gene sequence and whereby the hairy roots were Inoculated with a fungus.

### Whole Plant transformation

Plant tissues (such as tomato tissue) were transformed with A. tumefaciens with the constructs of the present invention and regenerated into whole plants. Whole transgenic plants were inoculated with the pest species and the phenotype of the plant and the inoculated pest species was monitored.

## Claims

1. An isolated double-stranded ribonucleic acid (dsRNA) with a length of at least 80 base pairs effective in RNAi gene silencing, wherein the dsRNA comprises at least two dsRNA fragments of at least 17 base pairs, each fragment comprising annealed complementary strands, wherein each dsRNA fragment comprises a strand that is complementary to at least part of the nucleotide sequence of a target sequence wherein:
(i) each dsRNA fragment comprises a strand that is complementary to at least part of the nucleotide sequence of a different target sequence, wherein said different target sequences originate:
(a) from a single target gene, wherein the dsRNA fragments are scrambled and combined deliberately in any rank order in the dsRNA and wherein the dsRNA fragments are not combined in the original order in which the fragments appear in the target gene, or
(b) from different target genes in a single target species or in different target species, or
(c) from different target species, or
(ii) said dsRNA comprises (a) at least two copies of one dsRNA fragment or (b) at least two copies of a series of dsRNA fragments.

2. An isolated dsRNA according to claim 1 wherein said at least two dsRNA fragments are not separated by a non-complementary region.

3. An isolated dsRNA according to any of claims 1 to 2 wherein said dsRNA comprises (i) at least two copies of one dsRNA fragment or (ii) at least two copies of a series of dsRNA fragments.

4. An isolated dsRNA according to any of claims 1 to 3, wherein said at least two dsRNA fragments are (i) repeats of one dsRNA fragment or (ii) repeats of a series of dsRNA fragments.

5. An isolated dsRNA according to any of claims 3 or 4 further comprising at least one dsRNA fragment which is distinct from the repeated fragments.

6. An isolated dsRNA according to claim 1, wherein said different target species belong to the same genus, family or order.

7. An isolated dsRNA according to claim 1, wherein said different target species belong to a different genus, family, order or phylum.

8. An isolated dsRNA according to any of claims 1 to 7, wherein the target sequence or target gene is from a plant pest organism.

9. An isolated dsRNA according to any of claims 1 to 8, wherein the dsRNA portion has a length between about 80 base pairs and about 500 base pairs.

10. An isolated deoxyribonucleic acid encoding a dsRNA of any of claims 1 to 9.

11. A recombinant DNA construct comprising a nucleic acid of claim 10.

12. A recombinant DNA construct according to claim 11 further comprising a regulatory sequence operably linked to said nucleic acid.

13. A recombinant DNA construct according to claim 12 wherein said regulatory sequence is selected from the group comprising constitutive promoters such as any selected from the group comprising the CaMV35S promoter, doubled CaMV35S promoter, ubiquitin promoter, actin promoter, rubisco promoter, GOS2 promoter, Figwort mosaic virus (FMV) 34S; or tissue specific promoters such as any selected from the group comprising root specific promoters of genes encoding PsMTA Class III Chitinase, photosynthetic tissue-specific promoters such as promoters of cab1 and cab2, rbcS, gapA, gapB and ST-LS1 proteins, JAS promoters, chalcone synthase promoter and promoter of RJ39 from strawberry.

14. A recombinant DNA construct according to claim 11 wherein said nucleic acid is cloned between two regulatory sequences that are in opposite direction with respect to each other, said regulatory sequences operably linked to said nucleic acid and said regulatory sequences independently selected from the group comprising RNA Poll, an RNA PolII, an RNA PolIII, T7 RNA polymerase or SP6 RNA polymerase.

15. A host cell comprising a dsRNA of any of claims 1 to 9, a nucleic acid of claim 10 or a recombinant DNA construct of any of claims 11 to 14.

16. A host cell according to claim 15, which is chosen from a bacterial cell, a yeast cell and a plant cell.

17. A transgenic plant, reproductive or propagation material for a transgenic plant comprising a plant cell of claim 16.

18. A plant comprising at least one dsRNA of any of claims 1 to 9, at least one nucleic acid of claim 10 or at least one recombinant DNA construct of any of claims 11 to 14.

19. A seed comprising at least one nucleic acid of claim 10 or at least one recombinant DNA construct of any of claims 11 to 14.

20. A method for the production of a transgenic plant cell or plant, comprising the step of administering a recombinant DNA construct of any of claims 11 to 14 to said plant cell or plant.

21. A composition comprising at least one dsRNA of any of claims 1 to 9 and a physiologically or agronomically acceptable excipient.

22. A composition comprising at least one nucleic acid of claim 10 or a recombinant DNA construct of any of claims 11 to 14, and a physiologically or agronomically acceptable excipient.

23. Use of a composition of claim 21 as a pesticide for a plant or for propagation or reproductive material of a plant.

24. A method for treating and/or preventing pest growth and/or pest infestation of a plant or propagative or reproductive material of a plant comprising applying an effective amount of a double-stranded RNA of any of claims 1 to 9, or a composition according to claims 21 or 22 to a plant or to propagation or reproductive material of a plant.

25. A method for treating and/or preventing pest infestation on a substrate comprising applying an effective amount of a double-stranded RNA of any of claims 1 to 9, or a composition according to claims 21 or 22 to said substrate.

26. A double-stranded RNA of any of claims 1 to 9 for use in controlling pest growth on a plant cell or plant or for preventing pest infestation of a plant cell or plant susceptible to infection by said pest species, wherein said use comprises contacting said pest species with said double-stranded RNA whereby the double-stranded RNA is taken up by said pest species and thereby controls pest growth or prevents pest infestation.

27. A double-stranded RNA for use according to claim 26, wherein said double-stranded RNA is expressed by a prokaryotic or eukaryotic, non-human host cell or non-human host organism.

28. A double-stranded RNA for use according to claim 26, wherein said double-stranded RNA is expressed by a plant cell or plant infested with or susceptible to infestation by said pest species.

29. A double-stranded RNA of any of claims 1 to 9 for use in down-regulating expression of at least one target gene in a pest species, wherein said use comprises contacting said pest species with said double-stranded RNA, whereby the double-stranded RNA is taken up by the pest species and thereby down-regulates expression of the pest target gene(s).

30. A double-stranded RNA for use according to claim 29, wherein said double-stranded RNA is expressed by a prokaryotic or eukaryotic, non-human host cell or non-human host organism.

31. A double-stranded RNA for use according to claim 29, wherein said double-stranded RNA is expressed by a plant cell or plant infested with or susceptible to infestation by said pest species.

32. A method according to claim 24 or claim 25, wherein said double-stranded RNA is expressed from at least one recombinant DNA construct of any of claims 11 to 14.

33. A method according to claim 24 or claim 25, wherein said double-stranded RNA is expressed from two recombinant DNA constructs of any of claims 11 to 14.

34. A method for producing a plant resistant against a plant pathogenic pest, comprising:
a) transforming a plant cell with a recombinant DNA construct of any of claims 11 to 14,
b) regenerating a plant from the transformed plant cell; and
c) growing the transformed plant under conditions suitable for the expression of the recombinant DNA construct, said grown transformed plant thus being resistant to said pest compared to an untransformed plant

35. A method for increasing plant yield comprising introducing in a plant at least one nucleic acid of claim 10 or a recombinant DNA construct of any of claims 11 to 14, in an expressible format.

36. Use of a double stranded RNA of any of claims 1 to 9 or a nucleic acid of claim 10, or a recombinant DNA construct of any of claims 11 to 14, or a cell according to claim 15 or 16, or a composition according to claim 21 or 22 for treating pest infection or infestation of plants.

37. A kit comprising a double stranded RNA of any of claims 1 to 9, or a nucleic acid of claim 10, or a recombinant DNA construct of any of claims 11 to 14, or a cell according to claims 15 or 16, or a composition according to claim 21 or 22 and instructions for use of the said double stranded RNA, nucleic acid, recombinant DNA, cell or composition for treating pest infection of plants.

38. An isolated double stranded RNA of any of claims 1 to 9 for use in silencing a target gene in a target organism wherein the target sequence is chosen from the genome of a target organism, which target organism is different from the organism in which the dsRNA is expressed and wherein the organism in which the dsRNA is expressed is a plant or plant cell or bacterial cell or fungal cell.

39. An isolated double stranded RNA of any of claims 1 to 9 for use in silencing a target gene in a target organism, wherein the target gene is chosen from the genome of a nematode, a fungus or an insect and the organism in which the dsRNA is expressed is a plant or a plant cell.

## Patentansprüche

1. Isolierte doppelsträngige Ribonukleinsäure (dsRNA) mit einer Länge von mindestens 80 Basenpaaren, die effektiv im RNAi-Gen-Silencing sind, worin die dsRNA mindestens zwei dsRNA-Fragmente mit mindestens 17 Basenpaaren umfasst, worin jedes Fragment annealte komplementäre Stränge umfasst, worin jedes dsRNA-Fragment einen Strang umfasst, der komplementär zu mindestens einem Teil der Nukleotidsequenz einer Targetsequenz ist, worin:
(i) jedes dsRNA-Fragment einen Strang umfasst, der komplementär zu mindestens einem Teil der Nukleotidsequenz einer verschiedenen Targetsequenz ist, worin die verschiedene Targetsequenz abstammt von:
(a) einem einzelnen Targetgen, worin die dsRNA-Fragmente bewusst zerteilt und in einer beliebigen Rangfolge in der dsRNA kombiniert sind, und worin die dsRNa-Fragmente nicht in der ursprünglichen Reihenfolge kombiniert sind, in welcher die Fragmente im Targetgen auftreten, oder
(b) von verschiedenen Targetgenen in einer einzelnen Targetspezies oder einer verschiedenen Targetspezies, oder
(c) von verschiedenen Targetspezies, oder
(ii) die dsRNA (a) mindestens zwei Kopien eines dsRNA-Fragments oder (b) mindestens zwei Kopien einer Reihe von dsRNA-Fragmenten umfasst.

2. Isolierte dsRNA nach Anspruch 1, worin die mindestens zwei dsRNA-Fragmente nicht durch eine nichtkomplementäre Region getrennt sind.

3. Isolierte dsRNA nach einem der Ansprüche 1 bis 2, worin die dsRNA (i) mindestens zwei Kopien eines dsRNA-Fragments oder (ii) mindestens zwei Kopien einer Reihe von dsRNA-Fragmenten umfasst.

4. Isolierte dsRNA nach einem der Ansprüche 1 bis 3, worin die mindesten zwei dsRNA-Fragmente (i) Wiederholungen eines dsRNA-Fragments oder (ii) Wiederholungen einer Reihe von dsRNA-Fragmenten sind.

5. Isolierte dsRNA nach einem der Ansprüche 3 bis 4, weiterhin umfassend mindestens ein dsRNA-Fragment, das verschieden von den wiederholten Fragmenten ist.

6. Isolierte dsRNA nach Anspruch 1, worin die verschiedenen Targetspezies zur gleichen Gattung, Familie oder Ordnung gehören.

7. Isolierte dsRNA nach Anspruch 1, worin die verschiedenen Targetspezies zu einer verschiedenen Gattung, Familie, Ordnung oder einem verschiedenen Stamm gehören.

8. Isolierte dsRNA nach einem der Ansprüche 1 bis 7, worin die Targetsequenz oder das Targetgen von einem Pflanzenschädlingsorganismus ist.

9. Isolierte dsRNA nach einem der Ansprüche 1 bis 8, worin der dsRNA-Teil eine Länge zwischen etwa 80 Basenpaaren und etwa 500 Basenpaaren hat.

10. Isolierte Deoxyribonukleinsäure, die für eine dsRNA nach einem der Ansprüche 1 bis 9 codiert.

11. Rekombinantes DNA-Konstrukt, umfassend eine Nukleinsäure nach Anspruch 10.

12. Rekombinantes DNA-Konstrukt nach Anspruch 11, weiterhin umfassend eine regulatorische Sequenz, die operativ mit der Nukleinsäure verbunden ist.

13. Rekombinantes DNA-Konstrukt nach Anspruch 12, worin die regulatorische Sequenz ausgewählt ist aus der Gruppe, umfassend konstitutive Promotoren, wie etwa diejenigen, ausgewählt aus der Gruppe, umfassend den CaMV35S-Promotor, doppelten CaMV35S-Promotor, Ubiquitin-Promotor, Aktin-Promotor, Rubisco-Promotor, GOS2-Promotor, Figwort-Maosaikvirus (FMV) 34S; oder gewebespezifische Promotoren, wie etwa jeden beliebigen, ausgewählt aus der Gruppe von stammspezifischen Promotoren von Genen, die für PsMTA Klasse III-Chitinase codieren, photosynthetische gewebespezifische Promotoren, wie etwa Promotoren von cab1 und cab2, rbcS, gapA, gapB und ST-LS1-Proteinen, JAS-Promotoren, Chalcon-Synthase-Promotor und Promotor von RJ39 von Erdbeere.

14. Rekombinantes DNA-Konstrukt nach Anspruch 11, worin die Nukleinsäure kloniert ist zwischen zwei regulatorischen Sequenzen, die in entgegengesetzter Richtung zueinander sind, worin die regulatorischen Sequenzen operativ mit der Nukleinsäure verbunden sind und die regulatorischen Sequenzen unabhängig ausgewählt sind aus der Gruppe, umfassend RNA Poll, eine RNA PolII, eine RNA PolIII, T7 RNA-Polymerase oder SP6 RNA-Polymerase,

15. Wirtszelle, umfassend eine dsRNA nach einem der Ansprüche 1 bis 9, eine Nukleinsäure nach Anspruch 10 oder eine rekombinantes DNA-Konstrukt nach einem der Ansprüche 11 bis 14.

16. Wirtszelle nach Anspruch 15, ausgewählt aus einer Bakterienzelle, einer Hefezelle und einer Pflanzenzelle.

17. Transgene Pflanze, Reproduktions- oder Vermehrungsmaterial für eine transgene Pflanze, umfassend eine Pflanzenzelle nach Anspruch 16.

18. Pflanze, umfassend mindestens eine dsRNA nach einem der Ansprüche 1 bis 9, mindestens eine Nukleinsäure nach Anspruch 10 oder mindestens ein rekombinantes DNA-Konstrukt nach einem der Ansprüche 11 bis 14.

19. Samen, umfassend mindestens eine Nukleinsäure nach Anspruch 10 oder mindestens ein rekombinantes DNA-Konstrukt nach einem der Ansprüche 11 bis 14.

20. Verfahren zu Herstellung einer transgenen Pflanzenzelle oder Pflanze, umfassend den Schritt des Verabreichens eines rekombinanten DNA-Konstrukts nach einem der Ansprüche 11 bis 14 an die Pflanzenzelle oder Pflanze.

21. Zusammensetzung, umfassend mindestens eine dsRNA nach einem der Ansprüche 1 bis 9 und einen physiologisch oder agronomisch verträglichen Exzipienten.

22. Zusammensetzung, umfassend mindestens eine Nukleinsäure nach Anspruch 10 oder ein rekombinantes DNA-Konstrukt nach einem der Ansprüche 11 bis 14 und einen physiologisch oder agronomisch verträglichen Exzipienten.

23. Verwendung einer Zusammensetzung nach Anspruch 21 als ein Pestizid für eine Pflanze oder für Vermehrungs- oder Reproduktionsmaterial einer Pflanze.

24. Verfahren zur Behandlung und/oder Verhinderung von Schädlingswachstum und/oder Schädlingsbefall einer Pflanze oder Vermehrungs- oder Reproduktionsmaterial einer Pflanze, umfassend das Anwenden einer wirkungsvollen Menge einer doppelsträngigen RNA nach einem der Ansprüche 1 bis 9 oder einer Zusammensetzung nach den Ansprüchen 21 oder 22 auf eine Pflanze oder ein Vermehrungs- oder Reproduktionsmaterial einer Pflanze.

25. Verfahren zur Behandlung und/oder Verhinderung von Schädlingsbefall auf einem Substrat, umfassend das Anwenden einer wirkungsvollen Menge einer doppelstängigen RNA nach einem der Ansprüche 1 bis 9 oder einer Zusammensetzung nach Anspruch 21 oder 22 auf das Substrat.

26. Doppelsträngige RNA nach einem der Ansprüche 1 bis 9 zur Verwendung zur Bekämpfung von Schädlingswachstum auf einer Pflanzenzelle oder Pflanze oder zur Verhinderung von Schädlingsbefall einer Pflanzenzelle oder Pflanze, die anfällig für Infektion durch die Schädlingsspezies ist, worin die Anwendung das In-Kontakt-bringen der Schädlingsspezies mit der doppelstängigen RNA umfasst, wobei die doppelsträngige RNA durch die Schädlingsspezies aufgenommen wird und dadurch das Schädlingswachstum kontrolliert oder Schädlingsbefall verhindert.

27. Doppelsträngige RNA zur Verwendung nach Anspruch 26, worin die doppelsträngige RNA durch eine prokaryotische oder eukaryotische, nichthumane Wirtszelle oder einen nichthumanen Wirtsorganismus exprimiert wird.

28. Doppelsträngige RNA zur Verwendung nach Anspruch 26, worin die doppelsträngige RNA durch eine Pflanzenzelle oder Pflanze exprimiert wird, die befallen ist oder anfällig ist für einen Befall durch die Schädlingsspezies.

29. Doppelsträngige RNA nach einem der Ansprüche 1 bis 9 zur Verwendung beim hinunterregulieren der Expression mindestens eines Targetgens in einer Schädlingsspezies, worin die Anwendung das In-Kontakt-bringen der Schädlingsspezies mit der doppelsträngigen RNA umfasst, wobei die doppelsträngige RNA durch die Schädlingsspezies aufgenommen wird und dadurch die Expression des (der) Schädlings-Targetgens (Schädlings-Targetgene) hinunterreguliert.

30. Doppelsträngige RNA zur Verwendung nach Anspruch 29, worin die doppelsträngige RNA durch eine prokaryotische oder eukaryotische, nichthumane Wirtszelle oder einen nichthumanen Wirtsorganismus exprimiert wird.

31. Verfahren nach Anspruch 29, worin die doppelsträngige RNA durch eine Pflanzenzelle oder Pflanze exprimiert wird, die befallen ist oder anfällig ist für einen Befall durch die Schädlingsspezies.

32. Verfahren nach Anspruch 24 oder Anspruch 25, worin die doppelsträngige RNA von mindestens einem rekombinanten DNA-Konstrukt nach einem der Ansprüche 11 bis 14 exprimiert wird.

33. Verfahren nach Anspruch 24 oder Anspruch 25, worin die doppelsträngige RNA von zwei rekombinanten DNA-Konstrukten nach einem der Ansprüche 11 bis 14 exprimiert wird.

34. Verfahren zum Herstellen einer Pflanze, die resistent gegen einen für Pflanzen pathogenen Schädling ist, umfassend:
a) Transformieren einer Pflanzenzelle mit einem rekombinanten DNA-Konstrukt nach einem der Ansprüche 11 bis 14,
b) Regenerieren einer Pflanze aus der transformierten Pflanzenzelle; und
c) Züchten der transformierten Pflanze unter Bedingungen, die geeignet sind zur Expression des rekombinanten DNA-Konstrukts, wobei die gezüchtete transformierte Pflanze damit im Vergleich mit einer nichttransformierten Pflanze resistent gegenüber Schädlingen ist.

35. Verfahren zur Erhöhung des Pflanzenertrags, umfassend das Einbringen mindestens einer Nukleinsäure nach Anspruch 10 oder eines rekombinanten DNA-Konstrukts nach einem der Ansprüche 11 bis 14 in einem exprimierbaren Format.

36. Verwendung einer doppelsträngigen RNA nach einem der Ansprüche 1 bis 9 oder einer Nukleinsäure nach Anspruch 10 oder eines rekombinanten DNA-Konstrukts nach einem der Ansprüche 11 bis 14 oder einer Zelle nach Anspruch 15 oder 16 oder einer Zusammensetzung nach Anspruch 21 oder 22 zur Behandlung von Schädlingsinfektion oder Schädlingsbefall von Pflanzen.

37. Kit, umfassend eine doppelsträngige RNA einem der Ansprüche 1 bis 9 oder eine Nukleinsäure nach Anspruch 10 oder ein rekombinantes DNA-Konstrukt nach einem der Ansprüche 11 bis 14 oder eine Zelle nach den Ansprüchen 15 oder 16 oder eine Zusammensetzung nach Anspruch 21 oder 22 und Anleitungen zur Verwendung der doppelsträngigen RNA, Nukleinsäure, rekombinanten DNA, Zelle oder Zusammensetzung zum Behandeln von Schädlingsbefall von Pflanzen.

38. Isolierte doppelsträngige RNA nach einem der Ansprüche 1 bis 9 zur Verwendung beim Silencing eines Targetgens in einem Targetorganismus, worin die Targetgensequenz ausgewählt ist aus dem Genom eines Targetorganismus, worin der Targetorganismus verschieden ist von dem Organismus, in welchem die dsRNA exprimiert wird und worin der Organismus, in welchem die dsRNA exprimiert wird, eine Pflanze oder Pflanzenzelle oder eine Bakterienzelle oder eine Pilzzelle ist.

39. Isolierte doppelsträngige RNA nach einem der Ansprüche 1 bis 9 zur Verwendung beim Silencing eines Targetgens in einem Targetorganismus, wobei das Targetgen ausgewählt ist aus dem Genom eines Nematoden, eines Pilzes oder eines Insekts und der Organismus, in welchem die dsRNA exprimiert wird, eine Pflanze oder Pflanzenzelle ist.

## Revendications

1. Acide ribonucléique bicaténaire (ARNbc) isolé ayant une longueur d'au moins 80 paires de bases, efficace pour rendre silencieux un gène ARNi, ledit ARNbc comprenant au moins deux fragments d'ARNbc d'au moins 17 paires de bases, chaque fragment comprenant des brins complémentaires circularisés, où chaque fragment d'ARNbc comprend un brin qui est complémentaire d'au moins une partie de la séquence de nucléotides, d'une quantité cible, où :
(i) chaque fragment comprend un brin qui est complémentaire d'au moins une partie de la séquence de nucléotides, d'une séquence cible différente, où ladite séquence cible différente provient :
(a) d'un gène cible unique, où les fragments d'ARNbc sont brouillés et combinés délibérément dans n'importe quel ordre des rangs dans l'ARNbc et où les fragments d'ARNbc ne sont pas combinés dans l'ordre initial dans lequel les fragments apparaissent dans le gène cible, ou
(b) de gènes cibles différents chez une seule espèce cible ou chez des espèces cibles différentes, ou
(c) d'espèces cibles différentes, ou
(ii) ledit ARNbc comprend (a) au moins deux copies d'un fragment d'ARNbc ou (b) au moins deux copies d'une série de fragments d'ARNbc.

2. ARNbc isolé suivant la revendication 1, dans lequel lesdits au moins deux fragments d'ARNbc ne sont pas séparés par une région non complémentaire.

3. ARNbc isolé suivant l'une quelconque des revendications 1 et 2, ledit ARNbc comprenant (i) au moins deux copies d'un fragment d'ARNbc ou (ii) au moins deux copies d'une série de fragments d'ARNbc.

4. ARNbc isolé suivant l'une quelconque des revendications 1 à 3, dans lequel lesdits au moins deux fragments d'ARNbc sont (i) des répétitions d'un fragment d'ARNbc ou (ii) des répétitions d'une série de fragments d'ARNbc.

5. ARNbc isolé suivant l'une quelconque des revendications 3 et 4, comprenant en outre au moins un fragment d'ARNbc qui est distinct des fragments répétés.

6. ARNbc isolé suivant la revendication 1, lesdites espèces cibles différentes appartenant au même genre, à la même famille ou au même ordre.

7. ARNbc isolé suivant la revendication 1, lesdites espèces cibles différentes appartenant à un genre différent, une famille différente, un ordre différent ou un phylum différent.

8. ARNbc isolé suivant l'une quelconque des revendications 1 à 7, dans lequel la séquence cible ou le gène cible provient d'un organisme parasite de végétaux.

9. ARNbc isolé suivant l'une quelconque des revendications 1 à 8, dans lequel la portion d'ARNbc a une longueur comprise entre environ 80 paires de bases et environ 500 paires de bases.

10. Acide désoxyribonucléique isolé codant pour un ARNbc de l'une quelconque des revendications 1 à 9.

11. Produit d'assemblage d'ADN recombinant comprenant un acide nucléique de la revendication 10.

12. Produit d'assemblage d'ADN recombinant suivant la revendication 11, comprenant en outre une séquence régulatrice liée de manière fonctionnelle audit acide nucléique.

13. Produit d'assemblage d'ADN recombinant suivant la revendication 12, dans lequel ladite séquence régulatrice est choisie dans le groupe comprenant des promoteurs constitutifs tels que n'importe quel promoteur constitutif choisi dans le groupe comprenant le promoteur CaMV35S, le promoteur CaMV35S doublé, le promoteur ubiquitine, le promoteur actine, le promoteur rubisco, le promoteur GOS2, le promoteur 34S du virus de la mosaïque du ficaire (FMV) ; ou des promoteurs spécifiques de tissus tels que n'importe quels promoteurs choisis dans le groupe comprenant des promoteurs spécifiques des racines, de gènes codant pour la chitinase PsMTA de classe III, des promoteurs spécifiques de tissus photosynthétiques tels que les promoteurs des protéines cab1 et cab2, rbcS, gapA, gapB et ST-LS1, les promoteurs JAS, le promoteur de chalcone-synthase et le promoteur de RJ39 de la fraise.

14. Produit d'assemblage d'ADN recombinant suivant la revendication 11, dans lequel ledit acide nucléique est cloné entre deux séquences régulatrices qui sont dans le sens opposé l'une par rapport à l'autre, lesdites séquences régulatrices étant liées de manière fonctionnelle audit acide nucléique et ladite séquence régulatrice étant choisie indépendamment dans le groupe comprenant une ARN PolI, une ARN PolII, une ARN PolIII, l'ARN-polymérase T7 et l'ARN-polymérase SP6.

15. Cellule hôte comprenant un ARNbc de l'une quelconque des revendications 1 à 9, un acide nucléique de la revendication 10 ou un produit d'assemblage d'ADN recombinant de l'une quelconque des revendications 11 à 14.

16. Cellule hôte suivant la revendication 15, qui est choisie entre une cellule bactérienne, une cellule de levure et une cellule végétale.

17. Plante transgénique, matière reproductrice ou multiplicatrice pour une plante transgénique, comprenant une cellule végétale de la revendication 16.

18. Plante comprenant au moins un ARNbc de l'une quelconque des revendications 1 à 9, au moins un acide nucléique de la revendication 10 ou au moins un produit d'assemblage d'ADN recombinant de l'une quelconque des revendications 11 à 14.

19. Semence comprenant au moins un acide nucléique de la revendication 10 ou au moins un produit d'assemblage d'ADN recombinant de l'une quelconque des revendications 11 à 14.

20. Procédé pour la production d'une cellule végétale ou plante transgénique, comprenant l'étape d'administration d'un produit d'assemblage d'ADN recombinant de l'une quelconque des revendications 11 à 14 à ladite cellule végétale ou plante.

21. Composition comprenant au moins un ARNbc de l'une quelconque des revendications 1 à 9 et un excipient physiologiquement ou agronomiquement acceptable.

22. Composition comprenant au moins un acide nucléique de la revendication 10 ou un produit d'assemblage d'ADN recombinant de l'une quelconque des revendications 11 à 14 et un excipient physiologiquement ou agronomiquement acceptable.

23. Utilisation d'une composition de la revendication 21 comme pesticide pour une plante ou pour une matière multiplicatrice ou reproductrice d'une plante.

24. Procédé pour le traitement et/ou la prévention de la croissance de parasites et/ou de l'infestation par des parasites d'une plante ou d'une matière multiplicatrice ou reproductrice d'une plante, comprenant l'application d'une quantité efficace d'un ARN bicaténaire de l'une quelconque des revendications 1 à 9, ou d'une composition suivant la revendication 21 ou 22 à une plante ou à la matière multiplicatrice ou reproductrice d'une plante.

25. Procédé pour le traitement et/ou la prévention de l'infestation par des parasites d'un substrat, comprenant l'application d'une quantité efficace d'un ARN bicaténaire de l'une quelconque des revendications 1 à 9, ou d'une composition suivant la revendication 21 ou 22 audit substrat.

26. ARN bicaténaire de l'une quelconque des revendications 1 à 9, pour une utilisation pour lutter contre la croissance de parasites sur une cellule végétale ou plante ou pour la prévention de l'infestation par des parasites d'une cellule végétale ou plante sensible à l'infection par ladite espèce de parasite, ladite utilisation comprenant la mise en contact de ladite espèce de parasite avec ledit ARN bicaténaire, ce qui fait que l'ARN bicaténaire est absorbé par ladite espèce de parasite et lutte ainsi contre la croissance du parasite ou prévient l'infestation par la parasite.

27. ARN bicaténaire pour une utilisation suivant la revendication 26, ledit ARN bicaténaire étant exprimé par une cellule hôte procaryotique ou eucaryotique, non humaine, ou un organisme hôte non humain.

28. ARN bicaténaire pour une utilisation suivant la revendication 26, ledit ARN bicaténaire étant exprimé par une cellule végétale ou plante infestée par, ou sensible à, l'infestation par ladite espèce de parasite.

29. ARN bicaténaire de l'une quelconque des revendications 1 à 9 pour une utilisation dans la régulation négative de l'expression d'au moins un gène cible chez une espèce de parasite, ladite utilisation comprenant la mise en contact de ladite espèce de parasite avec ledit ARN bicaténaire, ce qui fait que l'ARN bicaténaire est absorbé par l'espèce de parasite et provoque ainsi la régulation négative de l'expression d'un ou plusieurs gènes cibles du parasite.

30. ARN bicaténaire pour une utilisation suivant la revendication 29, ledit ARN bicaténaire étant exprimé par une cellule hôte procaryotique ou eucaryotique non humaine ou un organisme hôte non humain.

31. ARN bicaténaire pour une utilisation suivant la revendication 29, ledit ARN bicaténaire étant exprimé par une cellule végétale ou une plante infestée ou sensible à l'infestation par ladite espèce de parasite.

32. Procédé suivant la revendication 24 ou la revendication 25, dans lequel ledit ARN bicaténaire est exprimé à partir d'au moins un produit d'assemblage d'ADN recombinant de l'une quelconque des revendications 11 à 14.

33. Procédé suivant la revendication 24 ou la revendication 25, dans lequel ledit ARN bicaténaire est exprimé à partir de deux produits d'assemblage d'ADN recombinant de l'une quelconque des revendications 11 à 14.

34. Procédé pour la production d'une plante résistante à un parasite pathogène de végétaux, comprenant .
a) la transformation d'une cellule végétale avec un produit d'assemblage d'ADN recombinant de l'une quelconque des revendications 11 à 14 ;
b) la régénération d'une plante à partir de la cellule végétale transformée ; et
c) la croissance de la plante transformée dans des conditions convenables pour l'expression du produit d'assemblage d'ADN recombinant, ladite plante transformée qui s'est développée étant ainsi résistante audit parasite, comparativement à une plante non transformée.

35. Procédé pour augmenter le rendement d'une plante, comprenant l'introduction dans une plante d'au moins un acide nucléique de la revendication 10 ou d'un produit d'assemblage d'ADN recombinant de l'une quelconque des revendications 11 à 14, dans un format apte à l'expression.

36. Utilisation d'un ARN bicaténaire de l'une quelconque des revendications 1 à 9 ou d'un acide nucléique de la revendication 10, ou d'un produit d'assemblage d'ADN recombinant de l'une quelconque des revendications 11 à 14, ou d'une cellule suivant la revendication 15 ou 16, ou bien d'une composition suivant la revendication 21 ou 22 pour le traitement de l'infection ou de l'infestation par un parasite de plantes.

37. Kit comprenant un ARN bicaténaire de l'une quelconque des revendications 1 à 9, un acide nucléique de la revendication 10, un produit d'assemblage d'ADN recombinant de l'une quelconque des revendications 11 à 14, d'une cellule suivant la revendication 15 ou 16 ou d'une composition suivant la revendication 21 ou 22 et des instructions pour l'utilisation dudit ARN bicaténaire, dudit acide nucléique, dudit ADN recombinant, de ladite cellule ou de ladite composition pour le traitement d'une infection de plante par un parasite.

38. ARN bicaténaire isolé de l'une quelconque des revendications 1 à 9 pour une utilisation pour rendre silencieux un gène cible dans un organisme cible, ladite séquence cible étant choisie dans le génome d'un organisme cible, organisme cible qui est différent de l'organisme dans lequel l'ARNbc est exprimé, et l'organisme dans lequel l'ARNbc est exprimé étant une plante ou une cellule végétale ou une cellule bactérienne ou bien une cellule fongique.

39. ARN bicaténaire isolé de l'une quelconque des revendications 1 à 9 pour une utilisation pour rendre silencieux un gène cible dans un organisme cible, ledit gène cible étant choisi dans le génome d'un nématode, d'un champignon ou d'un insecte et l'organisme dans lequel l'ARNbc est exprimé étant une plante ou une cellule végétale.
